# EUROPÄISCHE PATENTSCHRIFT

(11) **EP 3 232 549 B1**
(45) Veröffentlichungstag und Bekanntmachung des Hinweises auf die Patenterteilung: **16.12.2020**
(21) Anmeldenummer: 17161634.5
(22) Anmeldetag: 17.03.2017
(51) Int. Cl.: B01F 13/08, H02K 21/44, H02K 7/09, F16C 32/04, A61M 1/10

(54) **ELEKTROMAGNETISCHER DREHANTRIEB UND ROTATIONSVORRICHTUNG**
ELECTROMAGNETIC ROTARY DRIVE AND ROTARY DEVICE
ENTRAINEMENT ROTATIF ELECTROMAGNETIQUE ET DISPOSITIF DE ROTATION

(30) Priorität: 14.04.2016 EP 16165393
(43) Veröffentlichungstag der Anmeldung: 18.10.2017
(73) Patentinhaber: Levitronix GmbH, 8005 Zürich (CH)
(72) Erfinder: Holenstein, Thomas, 5222 Umiken (CH); Schöb, Reto Dr., 8964 Rudolfstetten (CH)
(74) Vertreter: Intellectual Property Services GmbH

(56) Entgegenhaltungen:
- EP-A1- 1 063 753
- WO-A1-96/31934
- WO-A1-2012/159966

## Beschreibung

Die Erfindung betrifft einen Stator für einen elektromagnetischen Drehantrieb sowie eine Rotationsvorrichtung gemäss dem Oberbegriff des unabhängigen Patentanspruchs der jeweiligen Kategorie.

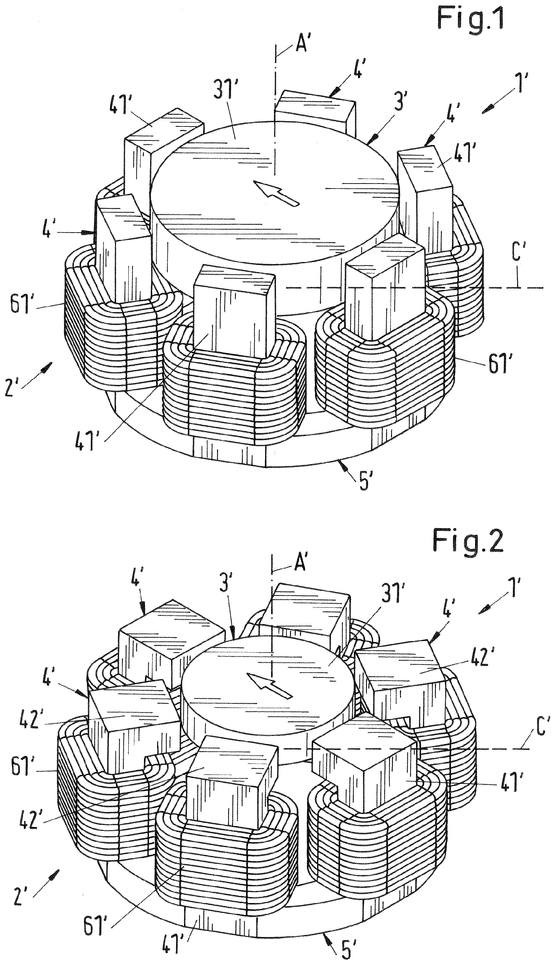

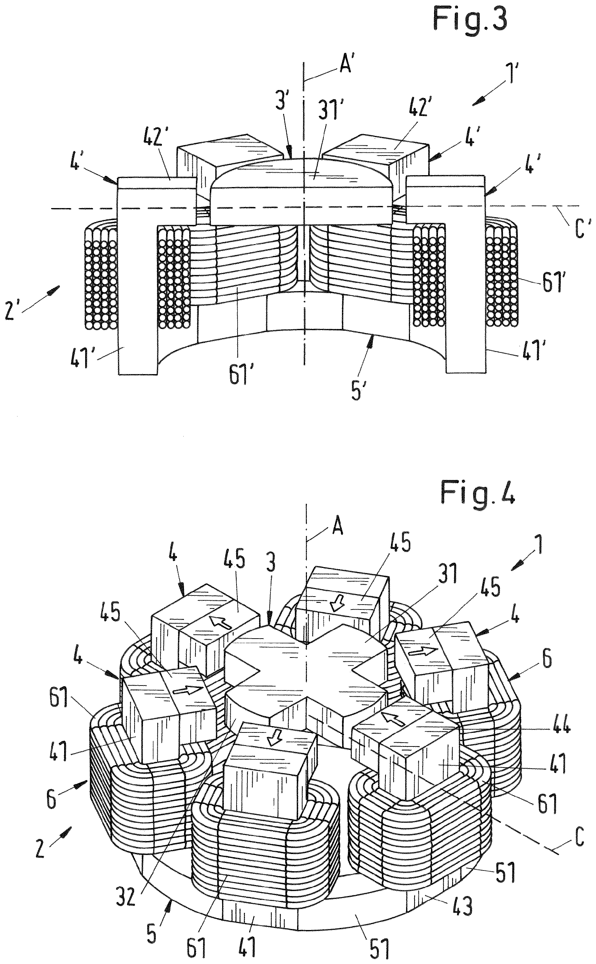

Es sind elektromagnetische Drehantriebe bekannt, die als sogenannter Tempelmotor ausgestaltet sind. Auf diese Ausgestaltung bezieht sich auch die vorliegende Erfindung. In den Fig. 1 und 2 sind jeweils in einer perspektivischen Darstellung zwei Ausgestaltungen eines Tempelmotors zu sehen, die vom Stand der Technik bekannt sind. Zum besseren Verständnis zeigt Fig. 3 noch einen Schnitt durch den Tempelmotor aus Fig. 2 in axialer Richtung. Um anzuzeigen, dass es sich bei den Darstellungen in den Fig. 1-3 um Vorrichtungen aus dem Stand der Technik handelt, sind hier die Bezugszeichen jeweils mit einem Hochkomma bzw. mit einem Strich versehen. Der Tempelmotor ist gesamthaft mit dem Bezugszeichen 1' bezeichnet.

Das Charakteristische eines Tempelmotors ist es, dass der Stator 2' eine Mehrzahl von Spulenkernen 4' aufweist, von denen jeder einen stabförmigen Längsschenkel 41' umfasst, der sich parallel zur axialen Richtung A' erstreckt. Mit der axialen Richtung A' ist dabei diejenige Richtung gemeint, welche durch die Solldrehachse des Rotors 3' definiert ist, also die Drehachse, um welche der Rotor 3' im Betriebszustand rotiert, wenn er in der radialen Ebene, die senkrecht zur axialen Richtung liegt, bezüglich des Stators 2' in einer zentrierten und unverkippten Position ist. In den Fig. 1-3 ist von dem Rotor 3' jeweils nur der magnetisch wirksame Kern 31' des Rotors 3' dargestellt, welcher jeweils als scheibenförmiger Permanentmagnet ausgestaltet ist. Die Magnetisierung des Permanentmagneten ist jeweils durch den Pfeil ohne Bezugszeichen dargestellt.

Ferner sind elektromagnetische Drehantriebe bekannt, die nach dem Prinzip des lagerlosen Motors ausgestaltet sind und betrieben werden. Mit dem Begriff lagerloser Motor ist dabei ein elektromagnetischer Drehantrieb gemeint, bei welchem der Rotor vollkommen magnetisch bezüglich des Stators gelagert ist, wobei keine separaten magnetischen Lager vorgesehen sind. Der Stator ist dazu als Lager- und Antriebsstator ausgestaltet, der sowohl Stator des elektrischen Antriebs als auch Stator der magnetischen Lagerung ist. Mit den elektrischen Wicklungen des Stators lässt sich ein magnetisches Drehfeld erzeugen, welches zum einen ein Drehmoment auf den Rotor ausübt, das dessen Rotation bewirkt, und welches zum anderen eine beliebig einstellbare Querkraft auf den Rotor ausübt, sodass dessen radiale Position aktiv steuerbar bzw. regelbar ist. Das Nichtvorhandensein eines separaten magnetischen Lagers bei vollständiger magnetischer Lagerung des Rotors ist die Eigenschaft, welcher der lagerlose Motor seinen Namen verdankt.

Der lagerlose Motor ist dem Fachmann mittlerweile hinlänglich bekannt, und wird für zahlreiche verschiedene Anwendungen eingesetzt. Grundlegende Beschreibungen finden sich beispielsweise in der EP-A-0 860 046 und in der EP-A-0 819 330.

Aus der WO-A-2012/159966 ist eine Rotationsmaschine bekannt, die als lagerloser Motor ausgestaltet ist, wobei der Rotor innerhalb des Stators angeordnet ist. Im Stator ist ein Permanentmagnet zur Erzeugung eines homopolaren magnetischen Flusses vorgesehen. Der Rotor ist um ein Polstück des Stators drehbar angeordnet und frei von Permanentmagneten.

Aufgrund der Abwesenheit von mechanischen Lagern eignet sich der lagerlose Motor insbesondere für Pump-, Misch- oder Rührvorrichtungen, mit denen sehr empfindliche Substanzen gefördert werden, beispielsweise Blutpumpen, oder bei denen sehr hohe Anforderungen an die Reinheit gestellt werden, beispielsweise in der pharmazeutischen Industrie oder in der biotechnologischen Industrie, oder mit denen abrasive Substanzen gefördert werden, welche mechanische Lager sehr schnell zerstören würden, beispielsweise Pumpen oder Mischer für Slurry in der Halbleiterindustrie. Auch werden lagerlose Motoren in der Halbleiterfertigung zum Tragen und Rotieren von Wafern verwendet, beispielsweise, wenn diese mit Photolack oder anderen Substanzen beschichtet bzw. behandelt werden.

Ein weiterer Vorteil des Prinzips des lagerlosen Motors bei Pump-, Rühr- oder Mischanwendungen ergibt sich bei der Ausgestaltung des Rotors als Integralrotor, der sowohl der Rotor des elektromagnetischen Antriebs ist, als auch der Rotor der Pumpe, des Rührers oder des Mischers. Neben der berührungslosen magnetischen Lagerung resultiert hier der Vorteil einer sehr kompakten und platzsparenden Ausgestaltung.

Zudem erlaubt das Prinzip des lagerlosen Motors auch Ausgestaltungen, bei denen der Rotor sehr leicht vom Stator trennbar ist. Dies ist ein sehr grosser Vorteil, weil damit beispielsweise der Rotor als Einmalteil für den Einmalgebrauch ausgestaltet werden kann. Solche Einmalanwendungen ersetzen heute häufig Prozesse, bei denen früher aufgrund der sehr hohen Reinheitsanforderungen alle diejenigen Komponenten, welche im Prozess mit den zu behandelnden Substanzen in Kontakt kommen, aufwändig gereinigt und sterilisiert werden müssen, beispielsweise mittels Dampfsterilisierung. Bei der Ausgestaltung für den Einmalgebrauch werden diejenigen Komponenten, welche mit den zu behandelnden Substanzen in Kontakt kommen, nur genau einmal verwendet und dann bei der nächsten Anwendung durch neue, das heisst ungebrauchte, Einmalteile ersetzt.

Als Beispiele seien hier die Pharmaindustrie und die biotechnologische Industrie genannt. Hier werden häufig Lösungen und Suspensionen hergestellt, die eine sorgfältige Durchmischung oder Förderung von Substanzen verlangen.

In der Pharmaindustrie müssen beispielsweise bei der Herstellung von pharmazeutisch wirksamen Substanzen höchste Ansprüche an die Reinheit gestellt werden, oft müssen die mit den Substanzen in Kontakt kommenden Komponenten sogar steril sein. Ähnliche Anforderungen ergeben sich auch in der Biotechnologie, beispielsweise bei der Herstellung, Behandlung oder Züchtung von biologischen Substanzen, Zellen oder Mikroorganismen, wo ein extrem hohes Mass an Reinheit gewährleistet sein muss, um die Brauchbarkeit des hergestellten Produkts nicht zu gefährden. Als ein weiteres Beispiel seien hier Bioreaktoren genannt, in denen beispielsweise biologische Substitute für Gewebe oder spezielle Zellen oder andere sehr empfindliche Substanzen gezüchtet werden. Auch hier benötigt man Pump-, Rühr- oder Mischvorrichtungen, um beispielsweise eine kontinuierliche Durchmischung der Nährflüssigkeit beziehungsweise deren kontinuierliche Zirkulation im Mischbehälter zu gewährleisten. Dabei muss eine sehr hohe Reinheit gewährleistet sein, um die Substanzen oder die erzeugten Produkte vor Kontaminationen zu schützen.

Bei solchen Anwendungen setzt sich dann die Pump-, Rühr- oder Mischvorrichtung aus einer Einmalvorrichtung und einer wiederverwendbaren Vorrichtung zusammen. Dabei umfasst die Einmalvorrichtung diejenigen Komponenten, die mit den Substanzen in Kontakt kommen und die als Einmal (single-use) -teile für den Einmalgebrauch ausgestaltet sind. Dies ist beispielsweise der Pump- oder Mischbehälter mit dem darin vorgesehenen Rotor, der dann beispielsweise ein Flügelrad zum Fördern der Substanzen umfasst. Die wiederverwendbare Vorrichtung umfasst diejenigen Komponenten, die dauerhaft also mehrfach verwendet werden, beispielsweise den Stator. Eine solche Vorrichtung ist zum Beispiel in der EP-B-2 065 085 offenbart.

Bei der Ausgestaltung als Einmalteil ist der Pump- oder Mischbehälter häufig als flexibler Kunststoffbeutel bzw. als Plastiksack mit darin enthaltenem Rotor ausgestaltet. Diese Beutel werden oft schon bei der Herstellung oder aber nach dem Verpacken und dem Lagern sterilisiert und dem Kunden in steriler Form in der Verpackung zugestellt.

Bei der Herstellung bzw. der Konzipierung von Einmalteilen für den Einmalgebrauch ist es ein wichtiges Kriterium, dass sie in möglichst einfacher Weise mit der wiederverwendbaren Vorrichtung bzw. deren Komponenten zusammengesetzt werden können. Es ist wünschenswert, dass dieses Zusammensetzen mit möglichst geringem Aufwand, mit wenigen Handgriffen, rasch und vorzugsweise ohne Werkzeug erfolgen kann.

Ein anderer Aspekt ist es, dass diese Einmalteile möglichst wirtschaftlich und kostengünstig gefertigt werden können. Hierbei wird insbesondere auch Wert auf preisgünstige, einfache Ausgangsmaterialien, wie beispielsweise handelsübliche Kunststoffe, gelegt. Auch ein umweltbewusster Umgang sowie eine verantwortungsvolle Nutzung der zur Verfügung stehenden Ressourcen sind wesentliche Aspekte bei der Konzeption von Einwegteilen.

Auch sind solche Ausgestaltungen bekannt, bei denen die gesamte Pump-, Rühr- oder Mischvorrichtung für den Einmalgebrauch ausgestaltet ist.

Eine besonders vorteilhafte und an sich bekannte Ausgestaltung ist es, den eingangs beschriebenen Tempelmotor als lagerlosen Motor auszubilden - egal ob er Komponenten für den Einmalgebrauch umfasst oder nicht.

Bei den in den Fig. 1-3 dargestellten Ausgestaltungen des Tempelmotors 1' sind die Spulenkerne 4' - hier beispielsweise sechs Spulenkerne 4' - mit den stabförmigen Längsschenkeln 41'kreisförmig und äquidistant um den Rotor 3' herum angeordnet (Innenläufer). Bei einer Ausgestaltung als Aussenläufer ist der Rotor z. B. ringförmig ausgestaltet und die Spulenkerne sind innenliegend bezüglich des Rotors angeordnet. Die Mehrzahl der stabförmigen Längsschenkel 41', die sich in axialer Richtung A' erstrecken und an die Säulen eines Tempels erinnern, hat dem Tempelmotor seinen Namen gegeben.

Jeder stabförmige Längsschenkel 41' erstreckt sich von einem ersten, darstellungsgemäss unteren, Ende in axialer Richtung A' bis zu einem zweiten, darstellungsgemäss oberen, Ende. Die ersten Enden sind in radialer Richtung durch einen Rückschluss 5' verbunden, welcher mehrere Segmente umfasst, die jeweils zwischen zwei benachbarten Spulenkernen 4' angeordnet sind. Der permanentmagnetische Rotor 3' ist zwischen den zweiten Enden der Längsschenkel 41' angeordnet und rotiert im Betriebszustand um die axiale Richtung A', wobei der Rotor 3' berührungslos magnetisch angetrieben und berührungslos magnetisch bezüglich des Stators 2' gelagert ist, und wobei die radiale Position des Rotors 3', so geregelt wird, dass er sich in einer zentrierten Position zwischen den zweiten Enden der Längsschenkel 41' befindet.

Um die für den magnetischen Antrieb und die magnetische Lagerung des Rotors 3' notwendigen elektromagnetischen Drehfelder zu erzeugen, tragen die Längsschenkel 41' Wicklungen. Bei den in den Fig. 1-3 gezeigten Ausführungsformen, sind die Wicklungen beispielsweise so ausgestaltet, dass um jeden Längsschenkel 41' herum eine diskrete Spule 61' gewickelt ist, das heisst, die Spulenachse jeder Spule 61' erstreckt sich jeweils in axialer Richtung A'. Dabei ist es typisch für den Tempelmotor, dass die Spulenachsen der Spulen 61' parallel zur Solldrehachse verlaufen und, dass die Spulen 61' bzw. die Wicklungen nicht in der magnetischen Rotorebene C' angeordnet sind. Die magnetische Rotorebene C' ist die magnetische Mittelebene des magnetisch wirksamen Kerns 31' des Rotors 3'. Dies ist diejenige Ebene senkrecht zur axialen Richtung A', in welcher der Rotor 3' bzw. der magnetisch wirksame Kern 31' des Rotors 3' im Betriebszustand gelagert wird. In der Regel und insbesondere bei der in den Fig. 1-3 dargestellten Ausgestaltung des magnetisch wirksamen Kerns 31' des Rotors 3 als Scheibe ist die magnetische Rotorebene C' die geometrische Mittelebene des magnetisch wirksamen Kerns 31' des Rotors 3', die senkrecht zur axialen Richtung A' liegt. Wie dies die Fig. 1-3 zeigen, sind die Spulen 61' unterhalb der magnetischen Rotorebene C' angeordnet und vorzugsweise unterhalb des magnetisch wirksamen Kerns 31' des Rotors 3'.

Die Fig. 2 und 3 zeigen eine häufig realisierte Ausgestaltung des Tempelmotors. Bei dieser Ausgestaltung umfasst jeder Spulenkern 4' zusätzlich zu dem Längsschenkel 41' einen Querschenkel 42', welcher jeweils an dem zweiten Ende des Längsschenkels 41' vorgesehen ist, und welcher sich in radialer Richtung erstreckt, also im Wesentlichen rechtwinklig zum Längsschenkel 41'. Bei dieser Ausgestaltung haben die Spulenkerne 4' jeweils die Form eines L, wobei die Querschenkel 42' die kurzen Schenkel des L bilden. Der Rotor 3' ist dann zwischen den Querschenkeln 42' angeordnet. Einer der Vorteile der Ausgestaltung als Tempelmotor ist es, dass in der magnetischen Rotorebene C' keine Wicklungen oder Wickelköpfe des Stators vorhanden sind. Dies ermöglicht es beispielsweise bei einer Anwendung des Tempelmotors in einer Zentrifugalpumpe, dass der Auslass der Zentrifugalpumpe in der Ebene vorgesehen werden kann, in welcher das Flügelrad des Pumpenrotors rotiert, der Auslass also auf gleicher Höhe bezüglich der axialen Richtung A' liegt wie die Flügel des Pumpenrotors, ohne dass dabei die Wicklungen des Stators stören. Diese zentrale, d.h. mittige Anordnung des Pumpenauslasses ist unter hydrodynamischen Aspekten und im Hinblick speziell auf die passive Lagerung und Stabilisierung des Rotors gegen Verkippungen besonders günstig.

Ausgehend von diesem Stand der Technik ist es eine Aufgabe der Erfindung, einen Stator für einen anderen elektromagnetischen Drehantrieb bereitzustellen, der als Tempelmotor ausgestaltet ist und der sich für eine Vielzahl von Anwendungen einsetzen lässt. Zudem ist es eine Aufgabe der Erfindung, einen elektromagnetischen Drehantrieb mit einem solchen Stator vorzuschlagen, sowie eine Rotationsvorrichtung zum Fördern, Pumpen, Mischen oder Rühren, die einen solchen Drehantrieb umfasst. Ferner soll der Drehantrieb auch für Anwendungen mit Komponenten für den Einmalgebrauch ausgestaltbar sein.

Die diese Aufgabe lösenden Gegenstände der Erfindung sind durch die Merkmale des unabhängigen Patentanspruchs 1 gekennzeichnet.

Erfindungsgemäss wird also ein Stator für einen elektromagnetischen Drehantrieb vorgeschlagen, der als Tempelmotor ausgestaltet ist, wobei mit dem Stator ein Rotor im Betriebszustand berührungslos magnetisch um eine Solldrehachse antreibbar ist, wobei der Stator eine Mehrzahl von Spulenkernen aufweist, von denen jeder einen stabförmigen Längsschenkel umfasst, welcher sich von einem ersten Ende in einer Richtung parallel zur Solldrehachse bis zu einem zweiten Ende erstreckt, wobei alle ersten Enden durch einen Rückschluss verbunden sind, und wobei eine Mehrzahl von Wicklungen zur Erzeugung eines elektromagnetischen Drehfelds vorgesehen ist, von denen jede einen der Längsschenkel umgibt, wobei die Spulenkerne eine Mehrzahl von Permanentmagneten umfassen, mit welchen ein permanentmagnetischer Vormagnetisierungsfluss generierbar ist, wobei jeder Spulenkern einen permanentmagnetischen Teil umfasst, der sich von dem ersten Ende bis zu dem zweiten Ende des Längsschenkels erstreckt, sowie zwei permanentmagnetfreie Teile, die sich jeweils von dem ersten Ende bis zu dem zweiten Ende erstrecken, wobei der permanentmagnetische Teil zwischen den beiden permanentmagnetfreien Teilen angeordnet ist.

Durch die spezielle Ausgestaltung des Stators für einen Tempelmotor, die Permanentmagnete im Stator umfasst, ist es möglich, einen grossen Teil oder sogar den gesamten magnetischen Fluss im Stator zu generieren. Dabei ist es insbesondere vorteilhaft - wie bereits vorangehend erläutert - dass die Permanentmagnete im Stator bereits einen konstanten Vormagnetisierungsfluss erzeugen, sodass zusammen mit dem elektromagnetisch generierten Fluss auch bei einer sehr kompakten Bauweise des Stators eine ausreichend hohe Durchflutung erzeugbar ist, um den Rotor berührungslos magnetisch anzutreiben oder berührungslos magnetisch zu lagern. Die spezielle Ausgestaltung des Stators mit den Spulenkernen, welche jeweils einen permanentmagnetischen Teil aufweisen, welcher zwischen zwei permanentmagnetfreien Teilen angeordnet ist, ermöglicht es, dass die elektromagnetischen Flusspfade im Stator so geführt werden, dass diese nicht durch die Permanentmagnete hindurchführen. Die permanentmagnetisch erregten Flüsse und die elektromagnetisch erregten Flüsse können somit derart geführt werden, dass diese sich im magnetischen Luftspalt zwischen dem Stator und dem Rotor überlagern, im Bereich der Permanentmagnete des Stators aber getrennt geführt werden. Die elektromagnetisch erregten Flüsse sollen vorzugsweise ausser im Bereich der Luftspalte zwischen Rotor und Stator, möglichst durch weichmagnetisches Material wie Eisen oder Silizium-Eisen geführt werden.

In einer besonders bevorzugten Ausgestaltung ist der Stator als Lager- und Antriebsstator ausgestaltet, mit welchem der Rotor im Betriebszustand berührungslos magnetisch bezüglich des Stators lagerbar ist.

Der erfindungsgemässe Stator eignet sich sowohl für Tempelmotoren, bei welchen der Rotor spulenfrei und frei von Permanentmagneten ausgestaltet ist, als auch für Tempelmotoren, bei welchen der Rotor Permanentmagnete und/oder Spulen umfasst.

Durch die Erfindung wird ferner ein elektromagnetischer Drehantrieb vorgeschlagen, der als Tempelmotor ausgestaltet ist, mit einem Rotor, welcher berührungslos magnetisch antreibbar ist, welcher spulenfrei und frei von Permanentmagneten ausgestaltet ist, und welcher einen magnetisch wirksamen Kern umfasst, sowie mit einem Stator, mit welchem der Rotor im Betriebszustand berührungslos magnetisch um eine Solldrehachse antreibbar ist, wobei der Stator erfindungsgemäss ausgestaltet ist.

Durch die spezielle Ausgestaltung des Stators des Tempelmotors, die Permanentmagnete im Stator umfasst, ist es möglich, den gesamten magnetischen Fluss im Stator zu generieren. Hierdurch wird es insbesondere möglich, dass der Rotor nicht zur Generierung des magnetischen Flusses beitragen, sondern diesen nur leiten bzw. führen muss. Somit kann im Rotor auf Permanentmagnete bzw. sehr hartmagnetische Stoffe zur Flussgenerierung verzichtet werden.

Durch den vollständigen Verzicht auf Permanentmagnete im Rotor, die zur Generierung des Antriebs- oder Steuerflusses beitragen, lässt sich dieser besonders einfach, wirtschaftlich und kostengünstig produzieren, was insbesondere auch für eine Ausgestaltung als Einmal-Rotor einen enormen Vorteil darstellt. Im Bereich zwischen dem Stator und dem magnetisch wirksamen Kern des Rotors werden je nach Ausgestaltung häufig verschiedene Ummantelungen, Spalte und Wandungen untergebracht, insbesondere eine Ummantelung des magnetisch wirksamen Kerns des Rotors, der Fluidspalt, oder ein Spalttopf, welcher den Stator umgibt. Um alle diese Elemente unterzubringen, ist ein Abstand von mindestens 3 Millimetern, besser von 4-6 Millimetern zwischen dem Stator und dem magnetisch wirksamen Kern des Rotors bevorzugt. Da der Rotor des erfindungsgemässen Drehantriebs keine Permanentmagnete aufweist und somit nicht zur magnetischen Durchflutung beitragen kann, muss die gesamte magnetische Durchflutung im Stator erzeugt werden. Für einen Abstand von beispielsweise 3 Millimetern zwischen Stator und dem magnetisch wirksamen Kern des Rotors ist vorzugsweise eine Durchflutung von rund 5000 Ampere erforderlich, um den Rotor zuverlässig magnetisch lagern und antreiben zu können. Wird der Stator, wie üblich, allein mit Wicklungen, also elektromagnetisch erregt, ist bei vernünftigen Abmessungen eine solch hohe Durchflutung unmöglich im meist engen Bauraum des Stators realisierbar. Erfindungsgemäss werden deshalb im Stator mehrere Permanentmagnete angebracht, welche einen konstanten Vormagnetisierungsfluss erzeugen. Da sich mit einem konstanten Magnetfluss jedoch weder ein Drehfeld zur Erzeugung eines Drehmoments noch ein regelbarer Magnetfluss zur aktiven magnetischen Lagerung des Rotors erzeugen lassen, werden im Stator zusätzlich Wicklungen angebracht, durch welche elektromagnetische und damit veränderbare und regelbare Magnetflüsse erzeugt werden können.

Es ist gemäss dem heutigen Stand der Technik üblich, als Permanentmagnete im Rotor insbesondere Metalle der Seltenen Erden bzw. Verbindungen oder Legierungen dieser Metalle zu verwenden, weil sich mit diesen aufgrund ihrer magnetischen Eigenschaften sehr starke permanentmagnetische Felder erzeugen lassen. Bekannte und häufig verwendete Beispiele dieser Seltenen Erden sind Neodym und Samarium. Solche Metalle stellen jedoch aufgrund ihres vergleichsweise geringen Vorkommens sowie ihrer aufwendigen Gewinnung und Verarbeitung einen erheblichen Kostenfaktor dar. Zudem ist die Entsorgung solcher Permanentmagnete, beispielsweise nach dem Einmalgebrauch auch unter umwelttechnischen Aspekten häufig mit Problemen oder hohem Aufwand verbunden, wodurch zusätzliche Kosten entstehen. Es ist daher unter wirtschaftlichen, Kosten- und Umweltaspekten insbesondere auch bei Einmalanwendungen vorteilhaft, dass es die Erfindung ermöglicht, bei den Rotoren insbesondere auf solche permanentmagnetischen Materialien, die aus Seltenen Erden bestehen oder diese enthalten, verzichten zu können.

Besonders bevorzugt ist der Stator als Lager- und Antriebsstator ausgestaltet, mit welchem der Rotor im Betriebszustand berührungslos magnetisch bezüglich des Stators lagerbar ist. Diese Ausgestaltung nach dem Prinzip des lagerlosen Motors ermöglicht eine besonders kompakte Ausgestaltung, weil nur ein einziger Stator vorgesehen ist, mit welchem sowohl die Antriebsfunktion als auch die Lagerfunktion für den Rotor realisierbar ist.

In einer bevorzugten Ausgestaltung umfasst jeder Spulenkern einen Querschenkel, welcher an dem zweiten Ende des Längsschenkels angeordnet ist, und welcher sich in einer radialen Richtung erstreckt, die senkrecht zu einer durch die Solldrehachse definierten axialen Richtung ist. Durch diese Querschenkel lassen sich an den Spulenkernen jeweils Stirnflächen ausbilden, welche dem magnetisch wirksamen Kern des Rotors gegenüberliegen. Durch diese Stirnflächen der Querschenkel ist eine besonders günstige Führung des magnetischen Flusses aus dem Stator in den Rotor bzw. umgekehrt möglich.

Erfindungsgemäss umfasst jeder Spulenkern einen permanentmagnetischen Teil, der sich von dem ersten Ende bis zu dem zweiten Ende des Längsschenkels erstreckt, sowie zwei permanentmagnetfreie Teile, die sich jeweils von dem ersten Ende bis zu dem zweiten Ende erstrecken, wobei der permanentmagnetische Teil zwischen den beiden permanentmagnetfreien Teilen angeordnet ist. Durch diese Ausgestaltung ist es möglich, dass die elektromagnetischen Flusspfade so geführt werden, dass diese nicht durch die Permanentmagnete hindurchführen. Die meisten Permanentmagnete, insbesondere Selten-Erden-Magnete aber auch Ferritmagnete haben eine relative Permeabilität, welche nur unwesentlich über eins liegt. Würden die elektromagnetischen Flusspfade also durch den oder die Permanentmagnet/e hindurch geführt, würde sich der elektromagnetisch wirksame Luftspalt somit um die Erstreckung der sich im Flusspfad befindlichen Permanentmagnete erhöhen und den Durchflutungsbedarf zusätzlich erhöhen. Es ist daher ein Vorteil, wenn die permanentmagnetisch erregten Flüsse und die elektromagnetisch erregten Flüsse so geführt werden können, dass diese sich im magnetischen Luftspalt zwischen dem Stator und dem Rotor überlagern, im Bereich der Permanentmagnete aber getrennt geführt werden. Die elektromagnetisch erregten Flüsse sollen vorzugsweise ausser im Bereich der Luftspalte zwischen Rotor und Stator, möglichst durch weichmagnetisches Material wie Eisen oder Silizium-Eisen geführt werden. Durch die Überlagerung der permanentmagnetisch erregten Flüsse und der elektromagnetisch erregten Flüsse im Bereich der Luftspalte zwischen Rotor und Stator können die Luftspaltflüsse so moduliert werden, dass sowohl eine Regelung der radialen Rotorposition als auch die Bildung von tangentialen Kraftkomponenten, welche ein Drehmoment bewirken, ermöglicht wird.

Ferner ist es vorteilhaft, wenn sich der permanentmagnetische Teil und die beiden permanentmagnetfreien Teile des Spulenkerns jeweils durch den Querschenkel erstrecken, und wobei im Querschenkel der permanentmagnetische Teil zwischen den beiden permanentmagnetfreien Teilen angeordnet ist. Hierdurch ist es nämlich besonders gut möglich, den permanentmagnetisch erregten Fluss und den elektromagnetisch erregten Fluss im Stator in dem Sinne getrennt voneinander zu führen, dass der elektromagnetisch erregte Fluss im Stator nicht durch Permanentmagnete geführt werden muss..

Gemäss einer bevorzugten Ausgestaltung weist der permanentmagnetische Teil des Spulenkerns in einem Schnitt in axialer Richtung eine im Wesentlichen gleiche Querschnittsfläche auf, wie einer der permanentmagnetfreien Teile in einem Schnitt in axialer Richtung. Diese Massnahme ermöglicht eine einfache und kostengünstige Gestaltung des Stators, bei welcher der permanentmagnetische Fluss im Stator in einfacher Weise im Wesentlichen getrennt vom elektromagnetisch erregten Fluss geführt werden kann.

Eine weitere vorteilhafte Massnahme besteht darin, dass die permanentmagnetischen Teile jeweils senkrecht zur radialen Richtung und senkrecht zur axialen Richtung polarisiert sind, wobei die Permanentmagnete benachbarter Spulenkerne jeweils in entgegengesetzter Richtung polarisiert sind. Diese Massnahme ermöglicht eine besonders gute und einfache Regelung des Antriebs und der Lagerung des Rotors.

Um insbesondere Wirbelstromverluste im Stator zu minimieren, ist es vorteilhaft, wenn die permanentmagnetfreien Teile der Spulenkerne jeweils geblecht aus Elementen hergestellt sind, wobei die Elemente in Umfangsrichtung des Rotors gestapelt sind.

Aus regelungstechnischen Gründen ist es bevorzugt, wenn der Stator eine gerade Anzahl von Spulenkernen aufweist, vorzugsweise sechs oder acht oder zwölf Spulenkerne.

Gemäss einer bevorzugten Ausgestaltung weisen die dem Rotor zugewandten Stirnflächen der Querschenkel der Spulenkerne in axialer Richtung eine Höhe auf, die jeweils grösser ist als die axiale Höhe des magnetisch wirksamen Kerns des Rotors. Hieraus resultiert insbesondere eine stärkere bzw. bessere passiv magnetische Stabilisierung des Rotors gegen Verkippungen gegenüber der Solldrehachse.

In einer bevorzugten Ausführungsform umfassen die Wicklungen Antriebsspulen zum Erzeugen eines elektromagnetischen Antriebsfelds für den Rotor, sowie davon separate Steuerspulen zum Einstellen einer auf den Rotor wirkenden Querkraft in radialer Richtung. Bei dieser Ausführungsform sind somit zwei separate Wicklungssysteme vorgesehen, nämlich die Antriebsspulen, mit welchen ein elektromagnetisches Drehfeld generierbar ist, welches ein Drehmoment auf den Rotor bewirkt und so dessen Rotation bewirkt, sowie die Steuerspulen, mit denen ein zusätzliches Drehfeld generierbar ist, mit welchem die auf den Rotor wirkende Querkraft, also eine Kraft in radialer Richtung, einstellbar ist. Dabei können die Antriebsspulen auf denselben Spulenkernen vorgesehen sein wie die Steuerspulen, oder es gibt Spulenkerne auf denen nur Antriebsspulen oder nur Steuerspulen vorgesehen sind, oder es sind Mischformen dieser beiden Varianten realisiert. Auf einem individuellen Spulenkern sind entweder nur Steuerspulen oder nur Antriebsspulen oder sowohl Steuer- als auch Antriebsspulen vorgesehen. Innerhalb des Stators kann dies natürlich von Spulenkern zu Spulenkern variieren, d.h. der Stator kann sowohl Spulenkerne umfassen, die nur Antriebs- bzw. Steuerspulen tragen, als auch Spulenkerne, die Antriebs- und Steuerspulen tragen. Apparativ gesehen hat die Aufteilung in Antriebs- und davon separate Steuerspulen den Vorteil, dass nicht für jede individuelle Spule jeweils ein separater bipolarer Leistungsverstärker zur Ansteuerung dieser Spule bereitgestellt werden muss.

In einer bevorzugten Ausgestaltung ist der magnetisch wirksame Kern des Rotors scheiben- oder ringförmig ausgestaltet. Vorzugsweise weist der Rotor eine radial äussere Begrenzungsfläche auf, die in einem zentrierten Zustand des Rotors von allen Spulenkernen den gleichen Abstand in radialer Richtung hat. Durch diese Massnahme lässt sich die für die Steuerung und Regelung der Rotorposition benötigte Sensorik vereinfachen, weil der Sollabstand des Rotors von den Spulenkernen über den Umfang des Rotors gesehen eine konstante Grösse ist.

Eine vorteilhafte Massnahme bezüglich der Ausgestaltung des Rotors besteht darin, dass der Rotor mit Flussbarrieren für den magnetischen Fluss ausgestaltet ist. Hierdurch ist es möglich, dass die magnetische Anisotropie des Rotors praktisch beliebig eingestellt werden kann und somit in einfacher Weise für den jeweiligen Anwendungsfall optimiert werden kann.

Durch die Erfindung wird ferner eine Rotationsvorrichtung zum Fördern, Pumpen, Mischen oder Rühren von Fluiden vorgeschlagen, welche einen elektromagnetischen Drehantrieb oder einen Stator umfasst, der erfindungsgemäss ausgestaltet ist.

Die erfindungsgemässe Rotationsvorrichtung kann insbesondere auch so ausgestaltet sein, dass sie Komponenten für den Einmalgebrauch umfasst. In dieser Ausgestaltung hat die Rotationsvorrichtung vorzugsweise eine Einmalvorrichtung, die für den Einmalgebrauch ausgestaltet ist, sowie eine wiederverwendbaren Vorrichtung, die für den Mehrfachgebrauch ausgestaltet ist, wobei die Einmalvorrichtung zumindest den Rotor umfasst, welcher eine Mehrzahl von Flügeln zum Fördern, Pumpen, Mischen oder Rühren des Fluides oder der Fluide aufweist, und wobei die wiederverwendbare Vorrichtung einen Stützbehälter zur Aufnahme des Rotors umfasst, sowie den Stator, mit welchem der Rotor im Betriebszustand berührungslos magnetisch antreibbar und lagerbar ist, wobei der Stator mindestens einen Permanentmagneten zum Erzeugen eines permanentmagnetischen Vormagnetisierungsflusses umfasst, sowie mindestens eine Wicklung zum Erzeugen eines elektromagnetischen Flusses, und wobei der permanentmagnetische Vormagnetisierungsfluss und der elektromagnetische Fluss zusammen den Rotor antreiben und lagern.

Weitere vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung näher erläutert. In der Zeichnung zeigen (teilweise im Schnitt):
- Fig. 1:: eine perspektivische Darstellung eines Tempelmotors gemäss dem Stand der Technik,
- Fig. 2:: eine perspektivische Darstellung eines anderen Tempelmotors gemäss dem Stand der Technik,
- Fig. 3:: einen Schnitt in axialer Richtung durch den Tempelmotor aus Fig. 2,
- Fig. 4:: eine perspektivische Darstellung eines ersten Ausführungsbeispiels eines elektromagnetischen Drehantriebs, das nicht Gegenstand der Erfindung ist,
- Fig. 5:: eine perspektivische Darstellung eines zweiten Ausführungsbeispiels eines erfindungsgemässen elektromagnetischen Drehantriebs,
- Fig. 6:: einen Schnitt in axialer Richtung durch das zweite Ausführungsbeispiel aus Fig. 5,
- Fig. 7:: eine perspektivische Darstellung eines dritten Ausführungsbeispiels eines erfindungsgemässen elektromagnetischen Drehantriebs,
- Fig. 8:: einen Schnitt in axialer Richtung durch das dritte Ausführungsbeispiel aus Fig. 7,
- Fig. 9:: eine Aufsicht auf das dritte Ausführungsbeispiel aus Fig. 7 mit dem Verlauf von magnetischen Flusslinien,
- Fig. 10:: eine Variante des dritten Ausführungsbeispiels in einer zur Fig. 7 analogen Darstellung,
- Fig. 11:: eine weitere Variante des dritten Ausführungsbeispiels in einer zur Fig. 7 analogen Darstellung,
- Fig. 12:: eine perspektivische Darstellung eines vierten Ausführungsbeispiels eines erfindungsgemässen elektromagnetischen Drehantriebs,
- Fig. 13:: einen Schnitt in axialer Richtung durch das vierte Ausführungsbeispiel aus Fig. 12,
- Fig. 14:: eine erste Variante für die Ausgestaltung der Wicklungen in einer zu Fig. 7 analogen Darstellung,
- Fig. 15:: einen Schnitt in axialer Richtung durch die in Fig. 14 dargestellte Variante,
- Fig. 16:: eine zweite Variante für die Ausgestaltung der Wicklungen in einer zu Fig. 11 analogen Darstellung,
- Fig. 17:: eine perspektivische Darstellung eines fünften Ausführungsbeispiels eines erfindungsgemässen elektromagnetischen Drehantriebs,
- Fig. 18:: eine perspektivische Darstellung eines sechsten Ausführungsbeispiels eines erfindungsgemässen elektromagnetischen Drehantriebs,
- Fig. 19:: einen Schnitt in axialer Richtung durch das sechste Ausführungsbeispiel aus Fig. 18,
- Fig. 20-23:: verschiedene Varianten für die Ausgestaltung der Spulenkerne, jeweils in einer perspektivischen Darstellung,
- Fig. 24-28:: verschiedene Varianten für die Ausgestaltung des Rotors, jeweils in einer perspektivischen Darstellung,
- Fig. 29:: einen Schnitt in axialer Richtung durch eine Variante für die Ausgestaltung des Rotors,
- Fig. 30-34:: weitere Varianten für die Ausgestaltung des Rotors, jeweils in einer perspektivischen Darstellung,
- Fig. 35-41:: verschiedene Varianten für die Ausgestaltung des Rotors mit Flussbarrieren, jeweils in einer perspektivischen Darstellung,
- Fig. 42-45:: verschiedene Varianten für die Anordnung von Sensoren, jeweils in einer perspektivischen Darstellung,
- Fig. 46:: einen Schnitt in axialer Richtung durch die in Fig. 45 dargestellte Variante für die Anordnung der Sensoren,
- Fig. 47:: eine weitere Variante für die Anordnung der Sensoren in einer zu Fig. 46 analogen Schnittdarstellung,
- Fig. 48:: eine perspektivische Darstellung eines ersten Ausführungsbeispiels einer erfindungsgemässen Rotationsvorrichtung, das als Mischvorrichtung ausgestaltet ist,
- Fig. 49:: einen Schnitt in axialer Richtung durch das erste Ausführungsbeispiel aus Fig. 48,
- Fig. 50:: eine perspektivische Darstellung eines zweiten Ausführungsbeispiels einer erfindungsgemässen Rotationsvorrichtung, das als Mischvorrichtung ausgestaltet ist,
- Fig. 51:: einen Schnitt in axialer Richtung durch das zweite Ausführungsbeispiel aus Fig. 50,
- Fig. 52:: einen Schnitt in axialer Richtung durch ein drittes Ausführungsbeispiel einer erfindungsgemässen Rotationsvorrichtung, welches eine Einmalvorrichtung sowie eine wiederverwendbare Vorrichtung umfasst,
- Fig. 53:: einen Schnitt in axialer Richtung durch ein viertes Ausführungsbeispiel einer erfindungsgemässen Rotationsvorrichtung, welches eine Einmalvorrichtung sowie eine wiederverwendbare Vorrichtung umfasst,
- Fig. 54:: einen Schnitt in axialer Richtung durch ein fünftes Ausführungsbeispiel einer erfindungsgemässen Rotationsvorrichtung, welches eine Einmalvorrichtung sowie eine wiederverwendbare Vorrichtung umfasst,
- Fig. 55:: eine perspektivische Darstellung eines sechsten Ausführungsbeispiels einer erfindungsgemässen Rotationsvorrichtung, die als Pumpvorrichtung ausgestaltet ist, und
- Fig. 56:: einen Schnitt in axialer Richtung durch das sechste Ausführungsbeispiel aus Fig. 55.

Wie bereits erwähnt, sind in den Fig. 1-3 zwei Tempelmotoren dargestellt, die aus dem Stand der Technik bekannt sind.

Fig. 4 zeigt eine perspektivische Darstellung eines ersten Ausführungsbeispiels eines elektromagnetischen Drehantriebs, das nicht Gegenstand der Erfindung ist. Der elektromagnetische Drehantrieb istgesamthaft mit dem Bezugszeichen 1 bezeichnet. Der Drehantrieb 1 ist als Tempelmotor ausgestaltet und umfasst einen Stator 2 sowie einen in dem Stator 2 berührungslos magnetisch gelagerten Rotor 3, welcher spulenfrei und frei von Permanentmagneten ausgestaltet ist. Der Rotor 3 ist als Reluktanzläufer ausgestaltet und umfasst einen magnetisch wirksamen Kern 31, welcher bei dem ersten Ausführungsbeispiel kreuzförmig mit vier ausgeprägten Rotorzähnen 32 ausgebildet ist. Im Betriebszustand ist der Rotor 3 mittels des Stators 2 berührungslos magnetisch um eine Solldrehachse antreibbar. Die Solldrehachse bezeichnet dabei diejenige Achse, um welche sich der Rotor 3 im Betriebszustand dreht, wenn sich der Rotor 3 bezüglich des Stators 2 in einer zentrierten und unverkippten Lage befindet. Diese Solldrehachse definiert eine axiale Richtung A. Üblicherweise stimmt die die axiale Richtung A festlegende Solldrehachse mit der Mittelachse des Stators 2 überein.

Da es für das Verständnis der Erfindung ausreichend ist, ist bei den im Folgenden beschriebenen Ausführungsbeispielen und Varianten des elektromagnetischen Drehantriebs 1 jeweils nur der magnetisch wirksame Kern 31 des Rotors 3 dargestellt. Es versteht sich, dass der Rotor 3 natürlich auch noch weitere Komponenten umfassen kann wie beispielsweise Ummantelungen oder Kapselungen, die vorzugsweise aus einem Kunststoff hergestellt sind, oder Flügel zum Mischen, Rühren oder Pumpen eines Fluides oder sonstige Komponenten.

Im Folgenden wird mit einer radialen Richtung eine Richtung bezeichnet, welche senkrecht auf der axialen Richtung A steht. Ferner wird mit der magnetischen Rotorebene C die magnetische Mittelebene des magnetisch wirksamen Kerns 31 des Rotors 3 bezeichnet. Dies ist diejenige Ebene senkrecht zur axialen Richtung A, in welcher der Rotor 3 bzw. der magnetisch wirksame Kern 31 des Rotors 3 im Betriebszustand gelagert wird, wenn der Rotor 3 nicht verkippt ist. In der Regel ist die magnetische Rotorebene C die geometrische Mittelebene des magnetisch wirksamen Kerns 31 des Rotors 3, die senkrecht zur axialen Richtung A liegt. Diejenige Ebene, in welcher der Rotor 3 im Betriebszustand gelagert wird, wird auch als radiale Ebene bezeichnet. Die radiale Ebene definiert die x-y -Ebene eines kartesischen Koordinatensystems, dessen z-Achse in axialer Richtung A verläuft. Ist der Rotor 3 also nicht verkippt, so stimmt die radiale Ebene mit der magnetischen Rotorebene C überein.

Das Charakteristische einer Ausgestaltung als Tempelmotor ist es, dass der Stator 2 eine Mehrzahl von separaten Spulenkernen 4 umfasst, von denen jeder einen stabförmigen Längsschenkel 41 umfasst, welcher sich von einem ersten Ende 43 in axialer Richtung A bis zu einem zweiten Ende 44 erstreckt, wobei alle ersten Enden 43 - gemäss der Darstellung in Fig. 4 sind dies die unteren Enden - durch einen Rückschluss 5 miteinander verbunden sind. Dabei umfasst der Rückschluss 5 mehrere Segmente 51, von denen jedes jeweils das erste Ende 43 eines Spulenkerns 4 mit dem ersten Ende 43 des benachbarten Spulenkerns 4 verbindet. Wie dies auch Fig. 4 zeigt, sind die individuellen Spulenkerne 4 vorzugsweise so angeordnet, dass sie bei einer Ausgestaltung als Innenläufer den Rotor 3 kreisförmig umgeben und auf diesem Kreis äquidistant angeordnet sind. Im Betrieb ist der Rotor 3 zwischen den zweiten Enden 44 der Spulenkerne 4 berührungslos magnetisch gelagert.

Die zueinander parallel ausgerichteten Längsschenkel 41 der Spulenkerne 4, die sich alle parallel zur axialen Richtung A erstrecken, und welche den Rotor 3 umgeben (oder bei einer Ausgestaltung als

Aussenläufer von dem Rotor 3 umgeben werden), sind es, welche dem Tempelmotor seinen Namen gegeben haben, weil diese parallelen Längsschenkel 41 an die Säulen eines Tempels erinnern.

Der Stator 2 umfasst ferner eine Mehrzahl von Wicklungen 6 zur Erzeugung eines elektromagnetischen Drehfelds. Die Wicklungen 6 sind hier als individuelle Spulen 61 ausgestaltet, von denen jede einen der Längsschenkel 41 umgibt. Das bedeutet, dass die Achsen der Spulen 61 jeweils parallel zur axialen Richtung A verlaufen. Bei dem in Fig. 4 dargestellten ersten Ausführungsbeispiel trägt jeder Längsschenkel 41 genau eine Spule 61.

Ein weiteres Merkmal des Tempelmotors 1 ist es deshalb, dass die Spulen 61 des Stators 2 ausserhalb der magnetischen Rotorebene C angeordnet sind, darstellungsgemäss unterhalb der magnetischen Rotorebene C. Vorzugsweise sind die Spulen 61 vollständig unterhalb des magnetisch wirksamen Kerns 31 angeordnet. Die Spulen 61 sind also nicht in der Ebene angeordnet, in welcher der Rotor 3 im Betriebszustand angetrieben und gelagert wird. Im Unterschied zu anderen elektromagnetischen Drehantrieben, bei welchen die Spulen des Stators so angeordnet sind, dass die Spulenachsen jeweils in der magnetischen Rotorebene liegen, also in der Ebene, in welcher der Rotor angetrieben und gelagert wird, sind beim Tempelmotor 1 die Spulen 61 des Stators 2 so angeordnet, dass die Achsen der Spulen 61 senkrecht auf der magnetischen Rotorebene C stehen.

Im Rahmen der vorliegenden Anmeldung ist also unter einer Ausgestaltung als Tempelmotor bzw. unter einem Tempelmotor 1 ein solcher elektromagnetischer Drehantrieb 1 zu verstehen, welcher eine Mehrzahl von Spulenkernen 4 aufweist, von denen jeder einen Längsschenkel 41 umfasst, der sich jeweils parallel zur axialen Richtung A erstreckt, wobei die ersten Enden 43 aller Spulenkerne 4 über den Rückschluss 5 miteinander verbunden sind, und wobei die Wicklungen 6, 61 des Stators 2 jeweils um die Längsschenkel 41 herum angeordnet sind, sodass die individuellen Spulen 61 jeweils mit ihrer Spulenachse parallel zur axialen Richtung A ausgerichtet sind. In einer bevorzugten Ausgestaltung ist der als Tempelmotor 1 ausgestaltete elektromagnetische Drehantrieb nach dem Prinzip eines lagerlosen Motors ausgestaltet ist. Der Tempelmotor 1 ist in diesem Falle also also eine spezielle Ausgestaltung eines lagerlosen Motors.

Auch wenn die Ausgestaltung des Tempelmotors 1 nach dem Prinzip des lagerlosen Motors bevorzugt ist, so ist die Erfindung jedoch nicht auf diese Ausgestaltung beschränkt. Es sind durchaus auch Ausgestaltungen möglich, bei denen die Lagerfunktion des Rotors durch andere Massnahmen, beispielsweise durch ein oder mehrere separate magnetische Lagereinheite(n) oder andere Lager, insbesondere mechanische Lager, realisiert ist.

Bei einem lagerlosen Motor 1 ist der Rotor 3 berührungslos magnetisch antreibbar und berührungslos magnetisch bezüglich des Stators 2 lagerbar. Dazu ist der Stator 2 als Lager- und Antriebsstator ausgestaltet, mit welchem der Rotor 3 im Betriebszustand berührungslos magnetisch um die Solldrehachse antreibbar - also in Rotation versetzbar- und bezüglich des Stators 2 berührungslos magnetisch lagerbar ist.

Der lagerlose Motor ist dem Fachmann mittlerweile hinlänglich bekannt, sodass eine detaillierte Beschreibung seiner Funktion nicht mehr notwendig ist. Mit dem Begriff lagerloser Motor ist gemeint, dass der Rotor 3 vollkommen magnetisch gelagert ist, wobei keine separaten Magnetlager vorgesehen sind. Der Stator 2 ist dazu als Lager- und Antriebsstator ausgestaltet, er ist also sowohl Stator des elektrischen Antriebs als auch Stator der magnetischen Lagerung. Dabei umfasst der Stator 2 die Wicklungen 6, mit denen sich ein magnetisches Drehfeld erzeugen lässt, welches zum einen ein Drehmoment auf den Rotor 3 ausübt, das dessen Rotation bewirkt, und welches zum anderen eine beliebig einstellbare Querkraft auf den Rotor 3 ausübt, sodass dessen radiale Position -also seine Position in der radialen Ebene- aktiv steuerbar bzw. regelbar ist. Somit sind zumindest drei Freiheitsgrade des Rotors 3 aktiv regelbar. Bezüglich seiner axialen Auslenkung in axialer Richtung A ist der Rotor 3 zumindest passiv magnetisch, das heisst nicht ansteuerbar, durch Reluktanzkräfte stabilisiert. Auch bezüglich der verbleibenden zwei Freiheitsgrade, nämlich Verkippungen bezüglich der zur Solldrehachse senkrechten radialen Ebene kann der Rotor 3 - je nach Ausführungsform - ebenfalls passiv magnetisch stabilisiert sein.

Aus dem Stand der Technik, beispielsweise aus der US-A-2009/121571 ist eine elektromagnetische Antriebs- und Lagervorrichtung bekannt, bei welcher der Stator des Antriebs und der Stator der magnetischen Lagerung zu einer baulichen Einheit zusammengefügt sind. Hier umfasst der Stator eine Lagereinheit, die aus einer oberen und einer unteren Lagerebene besteht, sowie eine Antriebseinheit, die zwischen diesen Lagerebenen angeordnet sind. Auch diese Vorrichtung zeigt also eine von der Antriebseinheit separierbare Lagereinheit, die ausschliesslich der magnetischen Lagerung dient. Derartige Vorrichtungen sind jedoch nicht als lagerlose Motoren im Sinne der vorliegenden Anmeldung zu verstehen, weil hier tatsächlich separate Lagereinheiten vorhanden sind, welche getrennt von der Antriebsfunktion die Lagerung des Rotors realisieren. Bei einem lagerlosen Motor im Sinne der vorliegenden Anmeldung ist es nicht möglich, den Stator in eine Lagereinheit und in eine Antriebseinheit aufzuteilen. Gerade diese Eigenschaft ist es ja, die dem lagerlosen Motor seinen Namen gibt.

Beim lagerlosen Motor wird im Unterschied zu klassischen Magnetlagern die magnetische Lagerung und der Antrieb des Motors über elektromagnetische Drehfelder realisiert, deren Summe zum einen ein Antriebsmoment auf den Rotor 3 erzeugen, sowie eine beliebig einstellbare Querkraft, mit welcher die radiale Position des Rotors 3 regelbar ist. Diese Drehfelder können entweder separat - also mit unterschiedlichen Spulen -, generiert werden, oder die Drehfelder können durch rechnerische Überlagerung der benötigten Ströme und dann mithilfe eines einzigen Spulensystems generiert werden.

Der Rotor 3 des erfindungsgemässen Drehantriebs 1 ist spulenfrei ausgestaltet, d. h. auf dem Rotor 3 sind keine Wicklungen vorgesehen. Der Rotor 3 umfasst den magnetisch wirksamen Kern 31, der je nach Ausgestaltung von einer Kunststoffummantelung umgeben sein kann. Beispiele für die Ausgestaltung des Rotors werden weiter hinten noch erläutert.

Bei dem erfindungsgemässen Drehantrieb 1 weist der Rotor 3 bzw. der magnetisch wirksame Kern 31 des Rotors 3 keine Permanentmagnete auf, er ist also frei von Permanentmagneten. Diese Massnahme ermöglicht eine besonders kostengünstige Ausgestaltung des Rotors 3 - beispielsweise auch als Einmalteil -, denn insbesondere sind für die Herstellung des Rotors 3 keine Seltenen Erden wie z. B. Neodym oder Samarium, bzw. Verbindungen oder Legierungen dieser notwendig, die häufig für die Herstellung von Permanentmagneten verwendet werden. Der Verzicht auf diese Permanentmagnete im Rotor bedeutet auch unter Umweltaspekten einen grossen Vorteil.

Als Permanentmagnete bezeichnet man üblicherweise solche ferromagnetischen oder ferrimagnetischen Werkstoffe, die hartmagnetisch sind, also eine hohe Koerzitivfeldstärke aufweisen. Die Koerzitivfeldstärke ist diejenige magnetische Feldstärke, die man benötigt, um einen Stoff zu entmagnetisieren. Im Rahmen dieser Anmeldung wird unter einem Permanentmagneten ein Werkstoff verstanden, der eine Koerzitivfeldstärke, genauer gesagt eine Koerzitivfeldstärke der magnetischen Polarisation aufweist, die mehr als 10'000 A/m beträgt.

Wenn der Rotor 3 also frei von Permanentmagneten ist, so bedeutet dies, dass der magnetisch wirksame Kern 31 des Rotors 3 nur Materialien umfasst, deren Koerzitivfeldstärke höchstens 10'000 A/m beträgt.

Mit der Bezeichnung, dass der Rotor 3 "frei von Permanentmagneten" ist, soll im Rahmen dieser Anmeldung verstanden werden, dass der Rotor 3 keine Permanentmagnete umfasst, die einen wesentlichen Beitrag zum Antriebsfeld zum Antreiben der Rotation des Rotors 3 leisten. Es ist natürlich möglich, dass am Rotor 3 andere Magnete bzw. Permanentmagnete vorgesehen sind, welche beispielsweise nur der Erfassung der Winkelstellung des Rotors dienen, oder die sonst einen Zweck erfüllen, der nichts mit dem Generieren des Antriebsflusses für den Rotors zu tun hat. Die Bezeichnung "frei von Permanentmagneten" bezieht sich also nur auf den Antrieb des Rotors 3.

Die Bezeichnung "frei von Permanentmagneten" bezüglich des Rotors ist im Rahmen dieser Anmeldung also so zu verstehen, dass der Rotor 3 frei von Permanentmagneten ist, welche einen Beitrag zum Antrieb des Rotors leisten, bzw. dass der Rotor 3 frei von Permanentmagneten ist, die zum Antriebsfluss für das Antreiben des Rotors 3 beitragen.

Vorzugsweise ist der magnetisch wirksame Kern 31 des Rotors aus einem weichmagnetischen Material gefertigt, beispielsweise aus Eisen, Nickel-Eisen oder Silizium-Eisen. Dabei kann der magnetisch wirksame Kern 31 z.B. durch Giessen, Stanzen, Pressen von weichmagnetischem Pulver mit anschliessendem Sintern, Schmieden, Umformen oder Zusammensetzen von Teilen wie Blechen hergestellt werden.

Bei der in Fig. 4 dargestellten Ausführungsform mit dem scheibenförmigen oder alternativ ringförmigen magnetisch wirksamen Kern 31 des Rotors 3 ist der Rotor 3 bezüglich dreier Freiheitsgrade aktiv magnetisch, d. h. ansteuerbar gelagert. Dies sind die beiden Freiheitsgrade der radialen Position des Rotors 3 in der radialen Ebene sowie der Freiheitsgrad der Rotation. Bezüglich der drei anderen Freiheitsgrade ist der Rotor rein passiv magnetisch, also nicht ansteuerbar, über Reluktanzkräfte stabilisiert. Dies sind die beiden Freiheitsgrade der Verkippungen des Rotors 3 bezüglich der radialen Ebene sowie die axiale Position des Rotors 3, also seine Lage bezüglich der axialen Richtung A. Bei dem in Fig. 4 dargestellten ersten Ausführungsbeispiel des erfindungsgemässen Drehantriebs 1 handelt es sich um eine Konfiguration als Innenläufer, das heisst, der Rotor 3 ist innerhalb des Stators 2 angeordnet. Bei diesem ersten Ausführungsbeispiel umfasst der Stator 2 insgesamt sechs Spulenkerne 4, von denen jeder einen stabförmingen Längsschenkel 41 umfasst, der sich parallel zur axialen Richtung erstreckt. Die sechs Spulenkerne 4 sind kreisförmig um den Rotor 3 herum angeordnet, wobei die Spulenkerne 4 auf dem Umfang dieses Kreises äquidistant verteilt sind. Die darstellungsgemäss unteren ersten Enden 43 der stabförmigen Längsschenkel 41 sind durch den Rückschluss 5 miteinander verbunden, wobei der Rückschluss 5 mehrere Segmente 51 umfasst, von denen jedes jeweils zwei benachbarte erste Enden 43 miteinander verbindet. Die Längsschenkel 41 weisen senkrecht zur axialen Richtung A einen rechteckigen Querschnitt auf. Der Stator 2 umfasst ferner eine Mehrzahl von Wicklungen 6 zur Erzeugung von elektromagnetischen Drehfeldern, mit welchen der Rotor 3 berührungslos magnetisch antreibbar und berührungslos magnetisch bezüglich des Stators 2 lagerbar ist. Bei dem ersten Ausführungsbeispiel sind als Wicklungen 6 insgesamt sechs individuelle Spulen 61 vorgesehen, wobei an jedem der Längsschenkel 41 jeweils eine Spule 61 vorgesehen ist. Jede Spule 61 ist um den jeweiligen Längsschenkel 41 herum angeordnet, sodass die Spulenachse jeweils parallel zur axialen Richtung A und damit senkrecht zur magnetischen Rotorebene C liegt.

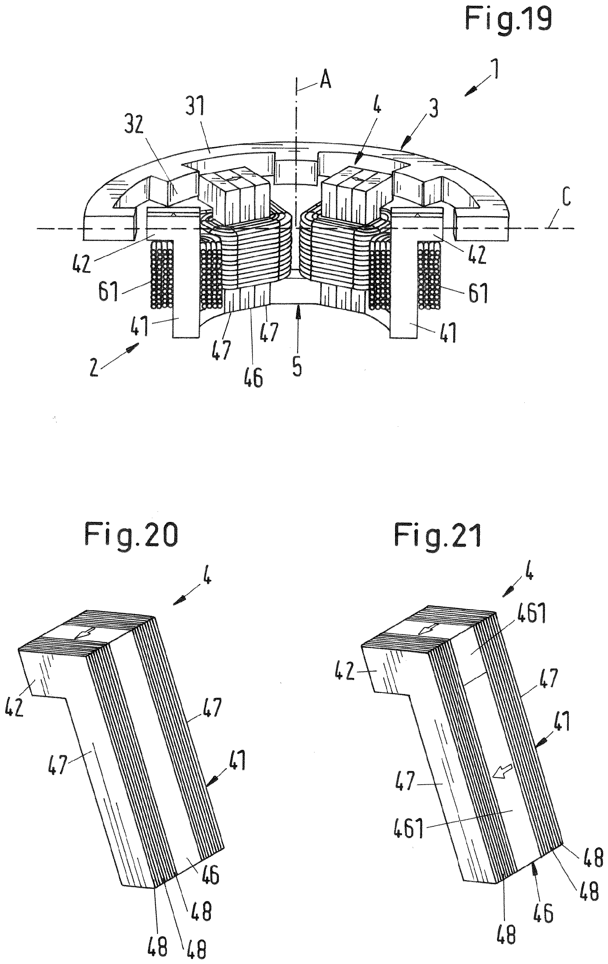

Der Rückschluss 5 bzw. seine Segmente 51 und die Längsschenkel 41 der Spulenkerne 4 sind jeweils aus einem weichmagnetischen Material gefertigt, weil sie als Flussleitelemente zur Führung des magnetischen Flusses dienen. Geeignete weichmagnetische Materialien sind beispielsweise ferromagnetische oder ferrimagnetische Materialien, also insbesondere Eisen, Nickel-Eisen oder Silizium-Eisen. Hierbei ist eine Ausgestaltung als Statorblechpaket bevorzugt, bei welcher die einzelnen Längsschenkel 41 und die Segmente 51 geblecht ausgestaltet sind, das heisst sie bestehen aus mehreren dünnen Elementen, die gestapelt sind. Spezielle Ausgestaltungen der Längsschenkel werden weiter hinten im Zusammenhang mit den Fig. 20-23 erläutert.

Der Rotor 3 ist als Reluktanzläufer ausgestaltet und umfasst den magnetisch wirksamen Kern 31, der hier als scheibenförmiges Kreuz mit vier ausgeprägten Rotorzähnen 32 ausgestaltet ist, und im Betriebszustand zwischen den zweiten Enden 44 - also den darstellungsgemäss oberen Enden - der Längsschenkel 41 der Spulenkerne 4 gelagert ist.

Erfindungsgemäss umfassen die Spulenkerne 4 eine Mehrzahl von Permanentmagneten 45 zum Generieren einer permanentmagnetischen Vormagnetisierung, die zum Erzeugen des magnetischen Antriebsflusses beitragen, mit welchem die Rotation des Rotors 3 um die Solldrehachse angetrieben wird.

Bei dem ersten Ausführungsbeispiel sind dazu insgesamt sechs Permanentmagnete 45 vorgesehen, wobei jeweils am zweiten Ende jedes Längsschenkels 41 ein Permanentmagnet 45 vorgesehen ist, welcher jeweils radial innenliegend an dem Längsschenkel 41 des Spulenkerns 4 angeordnet ist, also auf der dem Rotor 3 zugewandten Seite der Längsschenkel 41. Insgesamt hat somit jeder Spulenkern 4 in einem Schnitt in axialer Richtung A eine L-förmige Querschnittsfläche, wobei jeweils die Längsschenkel 41 den langen Schenkel des L bilden und die Permanentmagnete 45 den kurzen Schenkel des L, welcher auf den Rotor 3 hin ausgerichtet ist. Die Permanentmagnete 45 sind hier jeweils im Wesentlichen quaderförmig ausgestaltet, wobei ihre radial aussenliegende Begrenzungsfläche jeweils deckungsgleich mit den zweiten Enden der Längsschenkel 41 ist.

Die Permanentmagnete 45 sind jeweils in radialer Richtung magnetisiert, wie dies die Pfeile ohne Bezugszeichen in Fig. 4 andeuten. Dabei sind in Umfangsrichtung benachbarte Permanentmagnete 45 jeweils in entgegengesetzter Richtung polarisiert, d.h. ein Permanentmagnet 45, dessen Magnetisierung in radialer Richtung nach innen gerichtet ist, hat als Nachbarn in Umfangsrichtung jeweils zwei Permanentmagnete 45, deren Magnetisierung jeweils in radialer Richtung nach aussen gerichtet ist.

Im Betriebszustand werden in der vom lagerlosen Motor bekannten Weise mittels der Spulen 61 elektromagnetische Drehfelder erzeugt, mit welchen zum einen eine Tangentialkraft auf den Rotor 3 generiert wird, welche ein Drehmoment bewirkt, das die Rotation des Rotors 3 antreibt, und mit welchen zum anderen eine beliebig einstellbare Querkraft in radialer Richtung auf den Rotor 3 ausübbar ist, mit welcher die Position des Rotors 3 in der radialen Ebene aktiv magnetisch regelbar ist. Die Permanentmagnete 45 generieren dabei einen permanentmagnetischen Vormagnetisierungsfluss, wobei der permanentmagnetische Fluss und der elektromagnetische Fluss zusammen den Rotor 3 antreiben.

Der grosse Vorteil des permanentmagnetischen Vormagnetisierungsflusses ist es - wie bereits voranstehend erläutert - dass nicht der gesamte magnetische Fluss zum Antreiben und Lagern des Rotors 3 auf elektromagnetischem Wege erzeugt werden muss.

Beim Tempelmotor 1 dienen dabei die Längsschenkel 41 als in axialer Richtung A ausgerichtete Flussleitelemente, welche den magnetischen Fluss, und insbesondere auch den von den Spulen 61 generierten elektromagnetischen Fluss in die magnetische Rotorebene C führen, in welcher der Rotor 3 angetrieben und gelagert wird.

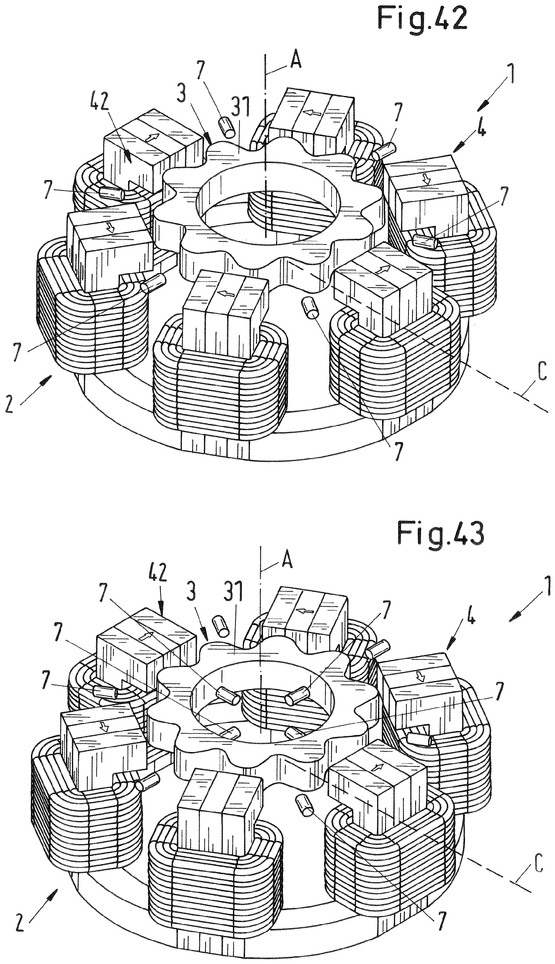

Die für die Ansteuerung der Spulen 61 benötigte Leistungselektronik sowie die entsprechenden Kontroll- und Regeleinrichtungen sind dem Fachmann hinlänglich bekannt und brauchen daher hier nicht näher erläutert zu werden. Auf die Ausgestaltung bzw. die Anordnung der Positionssensorik, mit welcher die radiale Position und die Winkelstellung des Rotors 3 detektiert wird, wird weiter hinten im Zusammenhang mit den Fig. 42-47 noch eingegangen.

Es versteht sich, dass das erste Ausführungsbeispiel in einer Variante auch als Aussenläufer ausgestaltet sein kann, bei welchem der dann vorzugsweise ringförmige magnetische Kern 31 des Rotors 3 die zweiten Enden 44 der Längsschenkel 41 umgibt. Die Permanentmagnete 45 sind dann natürlich in sinngemäss gleicher Weise an den radial aussenliegenden Flächen der zweiten Enden 44 der Längsschenkel 41 angeordnet, sodass sie wiederrum dem Rotor 3 zugewandt sind.

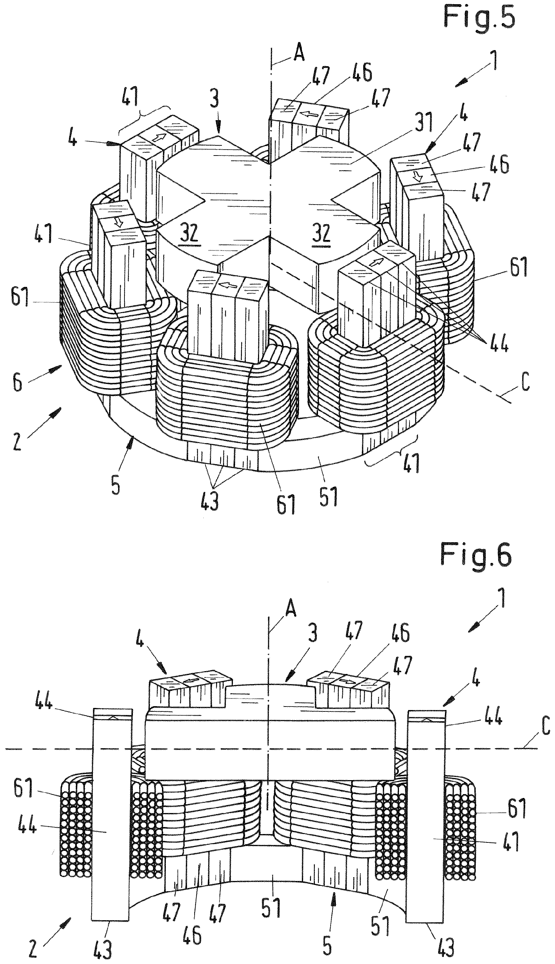

Fig. 5 zeigt in einer perspektivischen Darstellung ein zweites Ausführungsbeispiel eines erfindungsgemässen Drehantriebs 1. Fig. 6 zeigt zusätzlich einen Schnitt in axialer Richtung A durch dieses zweite Ausführungsbeispiel. Im Folgenden wird nur auf die Unterschiede zu dem vorangehend beschriebenen ersten Ausführungsbeispiel eingegangen. Insbesondere haben die Bezugszeichen die gleiche Bedeutung wie sie bereits im Zusammenhang mit dem ersten Ausführungsbeispiel erläutert sind. Es versteht sich, dass alle vorangehenden Erläuterungen in gleicher Weise oder in sinngemäss gleicher Weise auch für das zweite Ausführungsbeispiel gelten.

Das zweite Ausführungsbeispiel unterscheidet sich von dem ersten hauptsächlich durch die Ausgestaltung und die Anordnung der Permanentmagnete 45 im Stator 2. Bei dem zweiten Ausführungsbeispiel umfasst jeder Spulenkern 4 als Permanentmagnet einen permanentmagnetischen Teil 46, der sich jeweils vom ersten Ende 43 des Längsschenkels 41 des jeweiligen Spulenkerns 4 in axialer Richtung A bis zum zweiten Ende 44 des Längsschenkels 41 erstreckt. Zudem umfasst jeder Längsschenkel 41 zwei permanentmagnetfreie Teile 47, die sich jeweils vom ersten Ende 43 bis zum zweiten Ende 44 des jeweiligen Längsschenkels 41 erstrecken. Der permanentmagnetische Teil 46 ist dabei jeweils zwischen den beiden permanentmagnetfreien Teilen 47 des Längsschenkels 41 angeordnet. In Umfangsrichtung des Stators 2 gesehen, umfasst jeder Längsschenkel 41 jedes Spulenkerns 4 also den stabförmig ausgestalteten permanentmagnetischen Teil 46, der zwischen den beiden ebenfalls stabförmig ausgestalteten permanentmagnetfreien Teilen 47 angeordnet ist. Dabei ist die Länge des permanentmagnetischen Teils 46 in axialer Richtung A gleich gross wie die Länge der beiden an ihn angrenzenden permanentmagnetfreien Teile 47 in axialer Richtung A. Auch bezüglich der radialen Richtung hat der permanentmagnetische Teil 46 die gleiche Erstreckung wie die beiden an ihn angrenzenden permanentmagnetfreien Teile 47, sodass die permanentmagnetfreien Teile 47 durch den dazwischenliegenden permanentmagnetischen Teil 46 vollkommen voneinander getrennt sind. Die Querschnittsflächen der beiden permanentmagnetfreien Teile 47 und des permanentmagnetischen Teils 46 in einem axialen Schnitt senkrecht zur Umfangsrichtung des Stators 2 sind identisch, sodass jeder Spulenkern 4 in einem Schnitt senkrecht zur axialen Richtung A und parallel zur radialen Ebene, bzw. parallel zur magnetischen Mittelebene C des Rotors 3, eine rechteckige Querschnittsfläche aufweist, welche von den beiden permanentmagnetfreien Teilen 47 und dem dazwischen angeordneten permanentmagnetischen Teil 46 gebildet wird. Jeder Spulenkern 4 umfasst somit drei stabförmige, parallel zueinander angeordnete und sich jeweils in axialer Richtung A erstreckende Komponenten, nämlich die beiden permanentmagnetfreien Teile 47 und den zwischen ihnen angeordneten permanentmagnetischen Teil 46.

Diese Anordnung hat den Vorteil, wie weiter hinten noch erläutert wird, dass sich innerhalb des Stators 2 insbesondere der elektromagnetisch erzeugte Fluss nur durch die als Flussleitelemente dienenden permanentmagnetfreien Teile 47 und den Rückschluss 5, also nur durch weichmagnetisches Material, führen lässt. Somit kann es vermieden werden, dass der elektromagnetisch generierte Fluss im Stator 2 durch die permanentmagnetischen Teile 46 geführt werden muss, die einen sehr hohen Widerstand für den elektromagnetisch generierten Fluss darstellen.

Wie dies in den Fig. 5 und 6 die Pfeile ohne Bezugszeichen in den permanentmagnetischen Teilen 46 zeigen, sind die permanentmagnetischen Teile 46 jeweils in Umfangsrichtung des Stators 2 polarisiert bzw. magnetisiert, d.h. jeder permanentmagnetische Teil 46 weist eine Magnetisierung auf, die senkrecht zur radialen Richtung und senkrecht zur axialen Richtung A ausgerichtet ist. Die permanentmagnetischen Teile 46 benachbarter Spulenkerne 4 sind dabei jeweils in umgekehrter Richtung magnetisiert, das heisst jeder permanentmagnetische Teil 46 eines Spulenkerns 4 ist in Umfangsrichtung gesehen von zwei permanentmagnetischen Teilen 46 benachbarter Spulenkerne 4 umgeben, die jeweils in entgegengesetzter Richtung magnetisiert sind wie er selbst.

Auch bei dem zweiten Ausführungsbeispiel sind insgesamt sechs Spulenkerne 4 vorgesehen, welche den Rotor 3 kreisförmig und äquidistant umgeben. Die Anzahl von sechs Spulenkernen 4 ist dabei beispielhaft zu verstehen. Es kann natürlich auch eine andere Anzahl von Spulenkernen 4 vorgesehen sein, beispielsweise acht oder zwölf oder vier Spulenkerne 4, wobei aus regelungstechnischen Gründen eine gerade Anzahl von Spulenkernen 4 bevorzugt ist. Für viele Anwendungen hat sich eine Anzahl von sechs oder acht oder zwölf Spulenkernen 4 als vorteilhaft erwiesen.

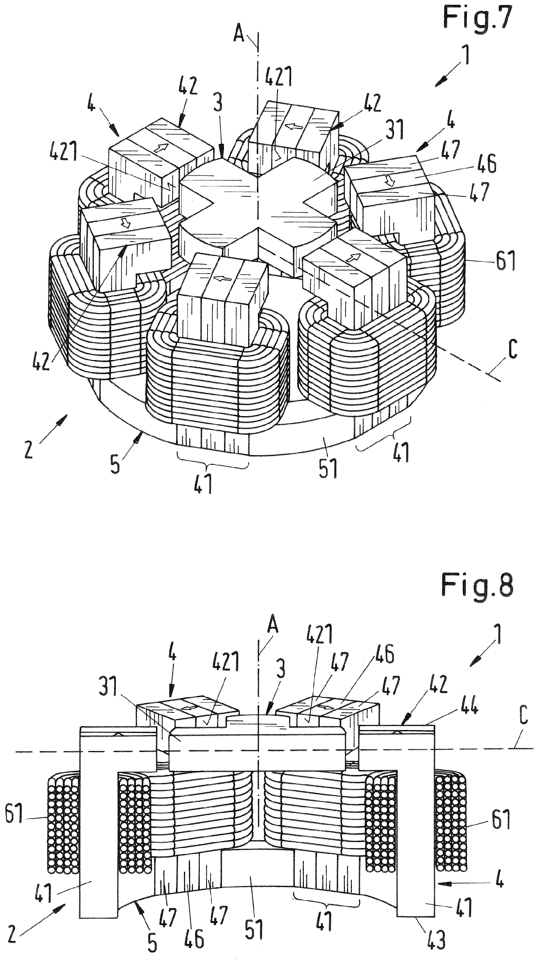

Fig. 7 zeigt in einer perspektivischen Darstellung ein bevorzugtes, drittes Ausführungsbeispiel des erfindungsgemässen Drehantriebs 1. Zum besseren Verständnis zeigt Fig. 8 einen Schnitt in axialer Richtung durch dieses dritte Ausführungsbeispiel. Im Folgenden wird nur auf die Unterschiede zu den vorangehend beschriebenen Ausführungsbeispielen eingegangen. Insbesondere haben die Bezugszeichen die gleiche Bedeutung wie sie bereits im Zusammenhang mit den vorangehend beschriebenen Ausführungsbeispielen erläutert sind. Es versteht sich, dass alle vorangehenden Erläuterungen in gleicher Weise oder in sinngemäss gleicher Weise auch für das dritte Ausführungsbeispiel gelten.

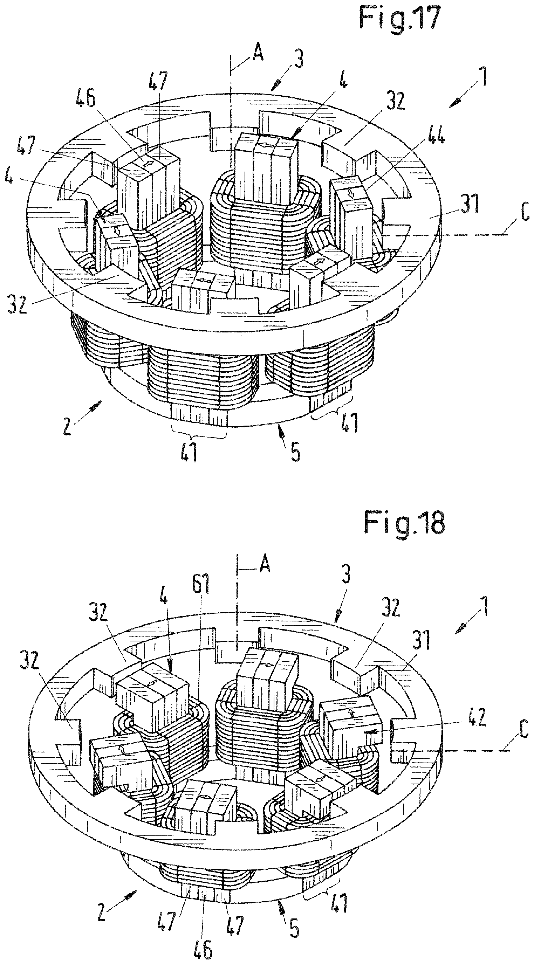

Bei dem dritten Ausführungsbeispiel umfasst jeder Spulenkern 4 einen Querschenkel 42, welcher an dem zweiten Ende 44 des jeweiligen Längsschenkels 41 angeordnet ist und welcher sich in radialer Richtung, also senkrecht zur axialen Richtung A und damit senkrecht zu dem jeweiligen Längsschenkel 41 erstreckt. Bei der in den Fig. 7 und 8 dargestellten Ausgestaltung des elektromagnetischen Drehantriebs 1 als Innenläufer erstrecken sich die Querschenkel 42 in radialer Richtung nach innen, also auf den Rotor 3 zu. Es versteht sich, dass sich bei einer Ausgestaltung des Drehantriebs 1 als Aussenläufer (siehe z.B. Fig. 18) die Querschenkel 42 jeweils in radialer Richtung nach aussen, also wiederum auf den Rotor 3 zu erstrecken.

Jeder Spulenkern 4 hat somit eine L-förmige Ausgestaltung, wobei die Längsschenkel 41 den sich in axialer Richtung A erstreckenden langen Schenkel des L bilden, und die sich senkrecht zu den Längsschenkeln 41 in radialer Richtung auf den Rotor 3 hin gerichtet erstreckenden Querschenkel 42 den kurzen Schenkel des L bilden.

Wie bereits bei dem zweiten Ausführungsbeispiel beschrieben, umfasst auch bei dem dritten Ausführungsbeispiel jeder Spulenkern 4 die zwei permanentmagnetfreien Teile 47, die zwischen sich den permanentmagnetischen Teil 46 einschliessen. Sowohl jeder der permanentmagnetfreien Teile 47 als auch jeder der permanentmagnetischen Teile 46 haben dabei eine L-förmige Ausgestaltung, wobei die beiden Grenzflächen der beiden permanentmagnetfreien Teile 47, die an den permanetmagnetischen Teil 46 angrenzen, jeweils deckungsgleich mit den sie kontaktierenden Grenzflächen des permanentmagnetischen Teils 46 ausgestaltet sind. Somit sind auch hier die beiden permanentmagnetfreien Teile 47 jedes Spulenkerns 4 durch den jeweils zwischen ihnen liegenden permanentmagnetischen Teil 46 vollkommen voneinander getrennt.

Das bedeutet, dass sich die permanentmagnetischen Teile 46 und die beiden permanentmagnetfreien Teile 47 jedes Spulenkerns jeweils auch durch den Querschenkel 42 erstrecken, und auch im Querschenkel 42 der permanentmagnetische Teil 46 zwischen den beiden permanentmagnetfreien Teilen 47 angeordnet ist.

Jeder Querschenkel 42 hat somit eine radial innenliegende (oder bei einer Ausgestaltung als Aussenläufer eine radial aussenliegende) Stirnfläche 421, welche dem Rotor 3 zugewandt ist. Die Mittellinie dieser Stirnfläche 421 die parallel zur radialen Ebene ist, liegt dabei in der magnetischen Rotorebene C, d.h. in derjenigen Ebene, in welcher der Rotor 3 im Betriebszustand gelagert wird.

Wie dies in den Fig. 7 und 8 die Pfeile ohne Bezugszeichen in den permanentmagnetischen Teilen 46 zeigen, sind auch bei dem dritten Ausführungsbeispiel die permanentmagnetischen Teile 46 jeweils in Umfangsrichtung des Stators 2 polarisiert bzw. magnetisiert, d.h. jeder permanentmagnetische Teil 46 weist eine Magnetisierung auf, die senkrecht zur radialen Richtung und senkrecht zur axialen Richtung A ausgerichtet ist. Die permanentmagnetischen Teile 46 benachbarter Spulenkerne 4 sind dabei jeweils in umgekehrter Richtung magnetisiert, das heisst jeder permanentmagnetische Teil 46 eines Spulenkerns 4 ist in Umfangsrichtung gesehen von zwei permanentmagnetischen Teilen 46 benachbarter Spulenkerne 4 umgeben, die jeweils in entgegengesetzter Richtung magnetisiert sind wie er selbst.

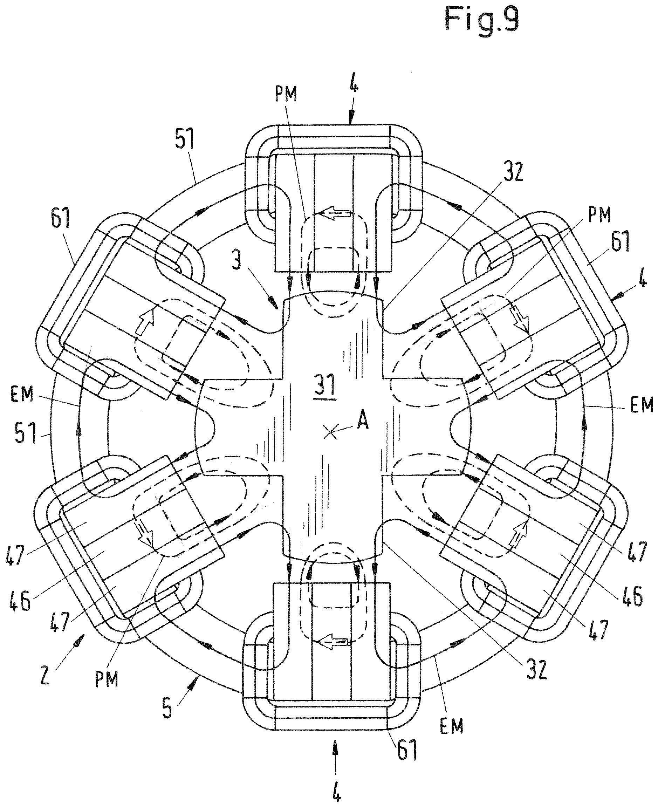

Die Ausgestaltung gemäss dem dritten Ausführungsbeispiel ermöglicht es in besonders guter Weise, den permanentmagnetischen Fluss im Stator 2 in dem Sinne getrennt vom elektromagnetischen Fluss zu führen, dass der elektromagnetische Fluss nicht durch die permanentmagnetischen Teile 46 geführt werden muss und gleichzeitig eine Überlagerung dieser beiden magnetischen Flüsse im Spalt zwischen dem Rotor 3 und dem Stator 2 möglich ist. Dies wird im Folgenden anhand von Fig. 9 erläutert, die eine Aufsicht auf das dritte Ausführungsbeispiel aus der axialen Richtung zeigt.

In Fig. 9 ist der Verlauf des von den permanentmagnetischen Teilen 46 generierten permanentmagnetischen Flusses durch die mit dem Bezugszeichen PM versehenen und gestrichelt dargestellten Feldlinien schematisch dargestellt. Der permanentmagnetische Fluss PM verläuft jeweils von dem permanentmagnetischen Teil 46 in den einen der benachbarten permanentmagnetfreien Teile 47 wird von diesem in radialer Richtung auf den magnetischen Kern 31 des Rotors 3 zu geführt, durchquert den Luftspalt zwischen dem Spulenkern 4 und dem magnetisch wirksamen Kern 31 des Rotors 3, wird von diesem zurückgeführt in den Luftspalt, gelangt dann zu dem anderen der benachbarten permanentmagnetfreien Teile 47 welcher den permanentmagnetischen Fluss PM radial nach aussen und dann zurück in den permanentmagnetischen Teil 46 führt, wodurch sich die Flusslinie schliesst.

Der von den Wicklungen 6 bzw. den Spulen 61 generierte elektromagnetische Fluss ist in Fig. 9 durch die mit dem Bezugszeichen EM versehenen und durchgezogen dargestellten Feldlinien schematisch dargestellt. Der von einer auf dem Längsschenkel 41 eines individuellen Spulenkerns 4 angeordneten Spule 61 durch Bestromung generierte elektromagnetische Fluss EM wird durch die beiden weichmagnetischen, permanentmagnetfreien Teile 47 dieses Spulenkerns 4 in radialer Richtung nach innen auf den magnetischen Kern 31 des Rotors 3 zu geführt, durchquert den Luftspalt zwischen diesen Spulenkern 4 und dem magnetisch wirksamen Kern 31 des Rotors 3, wird von diesem zurückgeführt in den Luftspalt, gelangt dann zu den beiden permanentmagnetfreien Teilen 47 der beiden benachbarten Spulenkerne 4 des individuellen Spulenkerns 4, welche benachbart zu den permanentmagnetfreien Teilen 47 des individuellen Spulenkerns 4 angeordnet sind, wird von diesen beiden permanentmagnetfreien Teilen 47 zunächst radial nach aussen und dann in axialer Richtung zum Rückschluss 5 geführt, wodurch sich die Feldlinie schliesst.

Auf diese Weise lässt es sich gewährleisten, dass der elektromagnetische Fluss EM im Stator 2 nur durch weichmagnetisches Material geführt wird, nämlich die permanentmagnetfreien Teile 47 der Spulenkerne 4 und den Rückschluss 5, nicht aber durch das hartmagnetische Material, aus welchem die permanentmagnetischen Teile 46 der Spulenkerne 4 gefertigt sind. Hieraus resultiert in vorteilhafter Weise eine besonders effiziente Nutzung der Energie des elektromagnetischen Flusses EM für die Lagerung und den Antrieb des Rotors 3.

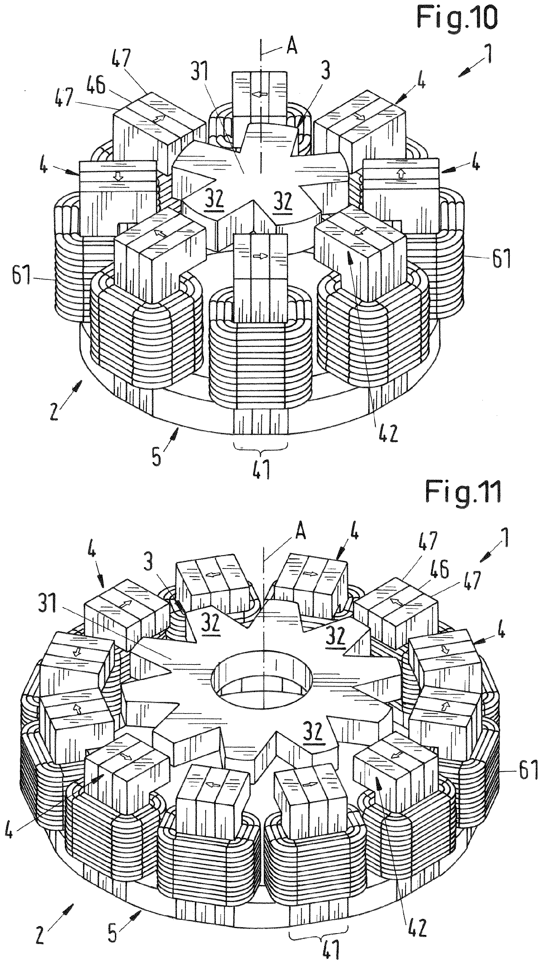

In den Fig. 10 und 11 sind in einer zu Fig. 7 analogen Darstellung zwei Varianten des dritten Ausführungsbeispiels jeweils in einer perspektivischen Darstellung gezeigt. Bei der in Fig. 10 gezeigten Variante hat der Stator 2 insgesamt acht Spulenkerne 4, die alle gleich und wie vorangehend beschrieben ausgestaltet sind. Auf jedem Längsschenkel 41 ist wiederum genau eine Spule 61 angeordnet. Der magnetisch wirksame Kern 31 des Rotors 3 ist scheibenförmig und sternförmig ausgestaltet mit fünf ausgeprägten Rotorzähnen 32. Bei der in Fig. 11 dargestellten Variante hat der Stator 2 insgesamt zwölf Spulenkerne 4, die alle gleich und wie vorangehend beschrieben ausgestaltet sind. Auf jedem Längsschenkel 41 ist wiederum genau eine Spule 61 angeordnet. Der magnetisch wirksame Kern 31 des Rotors 3 ist ringförmig mit einem zentralen Loch ausgestaltet. Er ist wiederum sternförmig und weist zehn ausgeprägte Rotorzähne 32 auf.

Es versteht sich, dass der erfindungsgemässe Drehantrieb 1 auch noch in vielen anderen Varianten bezüglich der Anzahl der Spulenkerne 4 und bezüglich der Anzahl der Rotorzähne 32 ausgestaltet sein kann, wobei aus regelungstechnischen Gründen eine gerade Anzahl von Spulenkernen 4 bevorzugt ist. Für den Fachmann stellt es kein Problem dar, je nach Anwendungsfall eine geeignete Anzahl von Spulenkernen 4 auszuwählen sowie eine daran angepasste Ausgestaltung des magnetisch wirksamen Kerns 31 des Rotors 3, z. B. eine jeweils geeignete Anzahl von Rotorzähnen 32.

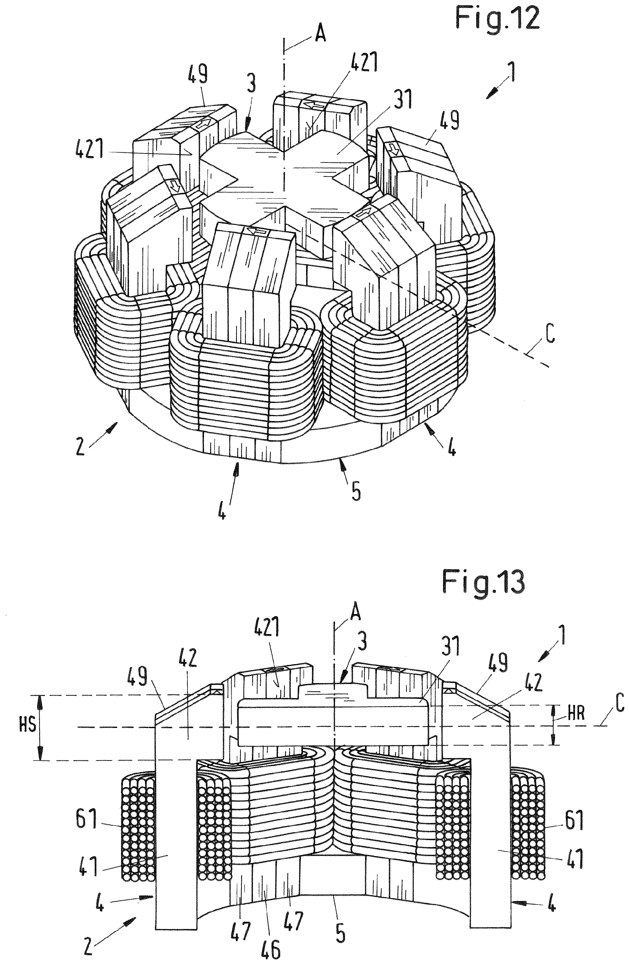

Fig. 12 zeigt in einer perspektivischen Darstellung ein viertes Ausführungsbeispiel eines erfindungsgemässen Drehantriebs 1. Zum besseren Verständnis zeigt Fig. 13 einen Schnitt in axialer Richtung durch dieses vierte Ausführungsbeispiel. Im Folgenden wird nur auf die Unterschiede zu den vorangehend beschriebenen Ausführungsbeispielen eingegangen. Insbesondere haben die Bezugszeichen die gleiche Bedeutung wie sie bereits im Zusammenhang mit den vorangehend beschriebenen Ausführungsbeispielen erläutert sind. Es versteht sich, dass alle vorangehenden Erläuterungen in gleicher Weise oder in sinngemäss gleicher Weise auch für das vierte Ausführungsbeispiel gelten.

Bei dem vierten Ausführungsbeispiel hat der Stator 2 insgesamt sechs Spulenkerne 4, die im Wesentlichen wiederum L-förmig ausgestaltet sind, also mit dem in axialer Richtung A verlaufenden Längsschenkel 41 und den auf den Rotor 3 hin gerichteten Querschenkel 42. Bei dem vierten Ausführungsbeispiel ist jedoch bei jedem Spulenkern 4 der radial aussenliegende Übergang von dem Längsschenkel 41 zu dem Querschenkel 42 mit einer Abschrägung 49 versehen, sodass die axiale Höhe des Querschenkels 42 in radialer Richtung auf den Rotor 3 zu gesehen zunimmt.

Ansonsten ist jeder Spulenkern 4 wiederum mit den beiden im Wesentlichen L-förmigen permanentmagnetfreien Teilen 47 und dem dazwischen liegenden im Wesentlichen L-förmigen permanentmagnetischen Teil 46 ausgestaltet, wobei die aneinander anstossenden Grenzflächen des permanetmagnetischen Teils 46 und der beiden permanentmagnetfreien Teile 47 deckungsgleich sind.

Bei dem vierten Ausführungsbeispiel ist bei jedem Spulenkern 4 die dem Rotor 3 zugewandte Stirnfläche 421 des Querschenkels 42 in axialer Richtung gesehen mit einer Höhe HS ausgestaltet, die grösser ist als die axiale Höhe HR des magnetisch wirksamen Kerns 31 des Rotors 3, sodass jede dieser Stirnflächen 421 die ihr zugewandte Aussenfläche des Rotors 3 bezüglich der axialen Richtung A nach oben und nach unten überragen. Dies ist besonders gut in Fig. 13 zu erkennen.

Diese Ausgestaltung mit HS grösser als HR ist insbesondere im Hinblick auf die passive magnetische Stabilisierung des Rotors 3 gegen Verkippungen und gegen Auslenkungen bezüglich der axialen Richtung A besonders vorteilhaft. Da die Stirnflächen 421 in axialer Richtung A eine grössere Ausdehnung haben als der magnetisch wirksame Teil 31 des Rotors 3, ist der Rotor 3 deutlich besser gegen Verkippungen bezüglich der axialen Richtung A bzw. gegen Verschiebungen in axialer Richtung A passiv magnetisch stabilisiert.

Klassischerweise wird in einem lagerlosen Motor, also auch bei der speziellen Ausgestaltung als Tempelmotor 1, die magnetische Antriebs- und Lagerfunktion durch die Überlagerung zweier magnetischer Drehfelder generiert, die üblicherweise als Antriebs- und Steuerfeld bezeichnet werden. Diese beiden mit den Wicklungen 6 bzw. Spulen 61 des Stators 2 erzeugten Drehfelder haben in der Regel eine Polpaarzahl, die sich um eins unterscheidet. Dabei werden mit dem Antriebsfeld auf den Rotor 3 in der radialen Ebene wirkende Tangentialkräfte erzeugt, die ein Drehmoment bewirken, was die Rotation des Rotors 3 um die axiale Richtung A bewirkt. Durch die Überlagerung des Antriebsfelds und des Steuerfelds lässt sich zusätzlich eine beliebig einstellbare Querkraft auf den Rotor 3 in der radialen Ebene erzeugen, mit welcher die Position des Rotors 3 in der radialen Ebene regelbar ist.

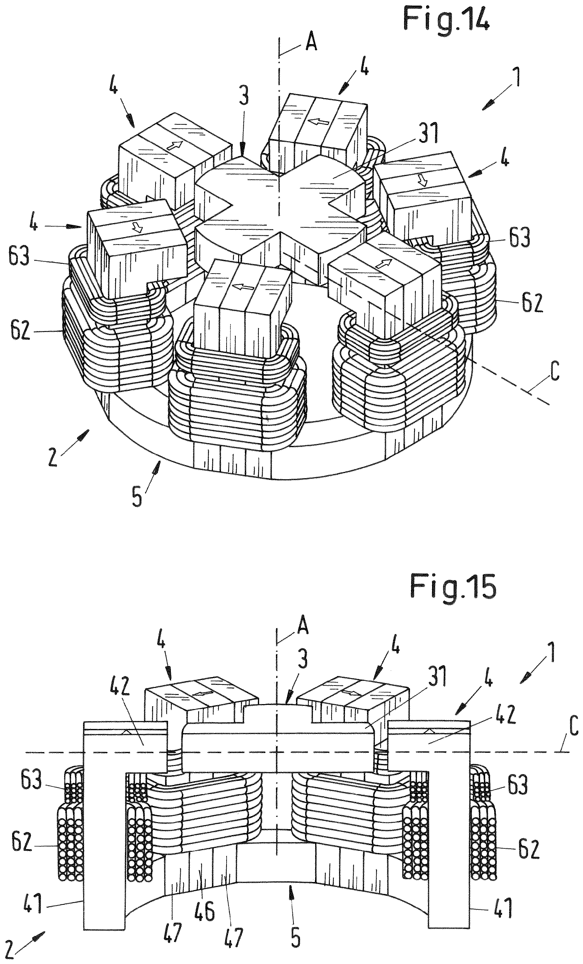

Zur Generierung des Antriebs- und des Steuerfelds ist es einerseits möglich, zwei unterschiedliche Wicklungssysteme zu verwenden, nämlich eines zur Erzeugung des Antriebsfelds und eines zur Erzeugung des Steuerfelds. Die Spulen zur Erzeugung des Antriebsfelds werden dann üblicherweise als Antriebsspulen bezeichnet und die Spulen zur Erzeugung des Steuerfelds als Steuerspulen. Der Strom, der in diese Spulen eingeprägt wird, wird dann als Antriebsstrom bzw. als Steuerstrom bezeichnet. Andererseits ist es aber auch möglich, die Antriebs- und Lagerfunktion mit nur einem einzigen Wicklungssystem zu generieren, sodass es also keine Unterscheidung zwischen Antriebs- und Steuerspulen gibt. Dies kann so realisiert werden, dass die von der Kontrolleinrichtung jeweils ermittelten Werte für den Antriebs- und den Steuerstrom rechnerisch -also z. B. mit Hilfe von Software - addiert bzw. überlagert werden und der sich daraus ergebende Gesamtstrom in die jeweilige Spulen eingeprägt wird. In diesem Fall ist es natürlich nicht mehr möglich, zwischen Steuer- und Antriebsspulen zu unterscheiden. In den bisher beschriebenen vier Ausführungsbeispielen ist die letztgenannte Variante realisiert, das heisst, es gibt keine Unterscheidung zwischen Antriebs- und Steuerspulen, sondern es gibt nur ein Wicklungssystem, in dessen Spulen 61 die rechnerisch ermittelte Summe aus dem Antriebs- und dem Steuerstrom eingeprägt wird. Es ist aber natürlich auch möglich, diese ersten vier Ausführungsbeispiele sowie die weiteren Ausführungsbeispiele und alle beschriebenen Varianten mit zwei getrennten Wicklungssystemen, nämlich jeweils mit separaten Antriebsspulen und separaten Steuerspulen auszugestalten. Diesbezügliche Varianten zur Ausgestaltung des Wicklungssystems werden nun anhand der Fig. 14-16 erläutert.

In Fig. 14 ist in einer der Fig. 7 entsprechenden Darstellung eine Variante gezeigt, bei der auf dem Längsschenkel 41 jedes Spulenkerns 4 jeweils eine Antriebsspule 62 und eine Steuerspule 63 angeordnet sind. Zum besseren Verständnis zeigt Fig. 15 noch einen Schnitt durch diese Variante, wobei der Schnitt in axialer Richtung A erfolgt. Auf jedem Längsschenkel 41 sind die Antriebsspule 62 und die Steuerspule 63 koaxial und bezüglich der axialen Richtung A benachbart zueinander angeordnet. Darstellungsgemäss ist die Steuerspule 63 jeweils oberhalb der Antriebsspule 62 auf dem Längsschenkel 41 des jeweiligen Spulenkerns 4 angeordnet. Dabei ist es auch hier bevorzugt, wenn sowohl die Antriebsspule 62 als auch die Steuerspule 63 bezüglich der axialen Richtung A vollständig unterhalb des magnetisch wirksamen Kerns 31 des Rotors 3 liegen.

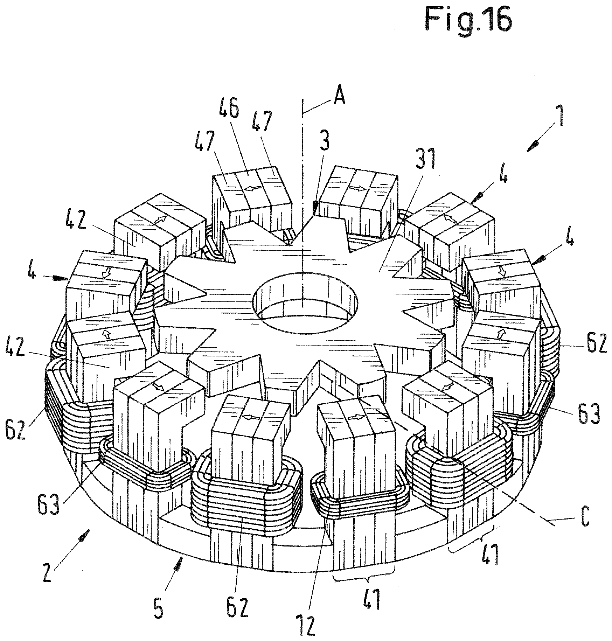

In Fig. 16 ist in einer der Fig. 11 entsprechenden Darstellung eine Variante gezeigt, bei der auf jedem Längsschenkel 41 jeweils nur eine Spule angeordnet ist, also entweder eine Antriebsspule 62 oder eine Steuerspule 63. Dabei ist es bevorzugt, wenn in Umfangsrichtung des Stators 2 gesehen immer abwechselnd eine Steuerspule 63 und eine Antriebsspule 62 auf benachbarten Spulenkernen vorgesehen ist, d. h. jeder Spulenkern 4, der nur eine Antriebsspule 62 aufweist, hat zwei in Umfangsrichtung unmittelbar benachbarte Spulenkerne 4, die jeweils nur eine Steuerspule 63 aufweisen und umgekehrt.

Wie bereits erwähnt, lassen sich diese verschiedenen Wicklungskonzepte, also das Konzept mit separaten Antriebs- 62 und Steuerspulen 63 und das Konzept mit nur einem Typ von Spule 61 auf alle Ausführungsbeispiele des erfindungsgemässen Drehantriebs 1 anwenden.

Für den Betrieb des Tempelmotors 1 werden die Spulen 61 bzw. die Antriebsspulen 62 und die Steuerspulen 63 in an sich bekannter Weise zum Generieren des elektromagnetischen Drehfelds angesteuert. Dazu ist eine nicht dargestellte Stelleinrichtung vorhanden, die eine Verstärkereinheit umfasst und von einer Kontroll- und Regeleinrichtung angesteuert wird. Für die Ausgestaltung der Verstärkereinheit gibt es mehrere Varianten. Verwendet man nur einen Typ von Spule 61, also keine separaten Antriebs- und Steuerspulen, sollte vorzugsweise für jede der Spulen 61 jeweils ein separater Leistungsverstärker vorgesehen sein, mit welchem der Spulenstrom oder die Spulenspannung für diese Spule 61 unabhängig von den Spulenströmen oder den Spulenspannungen der anderen Spulen 61 regelbar ist.

Im Folgenden wird mit beispielhaftem Charakter auf den Fall Bezug genommen, dass jeweils der Spulenstrom als Stellgrösse geregelt wird. Natürlich ist es auch möglich, insbesondere bei einer höheren Anzahl von Spulen, beispielsweise zwölf Spulen oder mehr, verschiedene Spulen 61 jeweils zu einer Gruppe von Spulen zusammenzufassen, die dann zu der gleichen elektrischen Phase gehören und dementsprechend von dem gleichen Leistungsverstärker angesteuert werden. Die Spulen 61 einer Gruppe werden dann beispielsweise in Serie hintereinander geschaltet, sodass in jede Spule der gleichen Gruppe der gleiche Spulenstrom eingeprägt wird.

Es ist also sowohl möglich, dass jede der Spulen 61 jeweils als genau eine diskrete Spule ausgestaltet ist, die für sich genommen eine elektrische Phase bildet, als auch, dass mehrere diskrete Spulen zu einer Gruppe zusammengefasst sind, die dann zu der gleichen elektrischen Phase gehören.

Sind beispielsweise -wie in Fig. 5 gezeigt- sechs Spulen 61 vorgesehen, die jeweils zu einer separaten elektrischen Phase gehören, sind in der Verstärkereinheit insgesamt sechs Leistungsverstärker vorgesehen. Dabei ist gemäss einer bevorzugten Variante jeder Leistungsverstärker als ein bipolarer Leistungsverstärker in an sich bekannter Weise als H-Brückenschaltung ausgestaltet. Mit der Bezeichnung "bipolarer Leistungsverstärker" ist gemeint, dass sowohl die Phasenströme als auch die Phasenspannungen jeweils positives und negatives Vorzeichen annehmen können.

Eine andere Variante für die Leistungsverstärker der Verstärkereinheit zum separaten Regeln der Spulenströme (oder Spulenspannungen) in den Spulen 61 oder in den Gruppen von Spulen 61 ist es, dass jeder Leistungsverstärker jeweils ein Brückenzweig der Verstärkereinheit ist. Für jede der Spulen 61 bzw. für jede der separaten elektrischen Phasen ist jeweils ein Brückenzweig der Verstärkereinheit als separater bipolarer Leistungsverstärker vorgesehen. Jede Spule 61 bzw. jede Gruppe ist einerseits mit dem sie versorgenden bipolaren Leistungsverstärker verbunden. Andererseits ist jede Spule 61 bzw. jede Gruppe von Spulen 61 mit einem gemeinsamen Sternpunkt verbunden, der auf einem Mittelpunktpotential liegt. Vorzugsweise ist der Sternpunkt als belastbarer Sternpunkt ausgestaltet, das heisst er ist mit einem belastbaren Potential verbunden, sodass, abgesehen von den sechs Spulenströmen ein zusätzlicher Strom über den Sternpunkt abfliessen bzw. in diesen hineinfliessen kann. Das bedeutet, die übliche Sternpunktbedingung, dass die Summe der Spulenströme im Sternpunkt immer Null sein muss, ist bei dieser Schaltung nicht mehr notwendig. Dies hat zur Folge, dass auch bei dieser Variante jeder Spulenstrom vollkommen unabhängig von den anderen Spulenströmen geregelt werden kann.

Gemäss einer anderen bevorzugten Variante werden die Spulen 61 bzw. die Gruppen von Spulen 61 mit konventionellen Drehstromstellern versorgt, wobei ein Drehstromsteller üblicherweise drei elektrische Phasen versorgen kann. So ist es beispielsweise bei der in Fig. 7 dargestellten Variante möglich, die sechs separaten Spulen mit zwei unabhängigen Drehstromstellern zu versorgen, von denen jeder drei Spulen 61 versorgt. Dabei sind die beiden Drehstromsteller voneinander getrennt, d.h. insbesondere ihre Sternpunkte sind voneinander getrennt, bzw. unabhängig voneinander. Analoges gilt natürlich auch für Gruppen von Spulen 61.

Insbesondere ist es auch bei der Ausgestaltung mit separaten Antriebsspulen 62 und separaten Steuerspulen 63 vorteilhafterweise möglich, die jeweiligen Phasenströme in den Antriebs- 62 und Steuerspulen 63 mit konventionellen Drehstromstellern bereitzustellen, wobei ein Drehstromsteller üblicherweise drei elektrische Phasen versorgen kann.

Wie bereits erwähnt, kann der erfindungsgemässe elektromagnetische Drehantrieb 1 auch als Aussenläufer, also mit innenliegendem Stator 2 und den Stator radial aussen umgebenden Rotor 3 ausgestaltet sein. Fig. 17 zeigt in einer perspektivischen Darstellung ein fünftes Ausführungsbeispiel eines erfindungsgemässen elektromagnetischen Drehantriebs 1, das als Aussenläufer ausgestaltet ist. Im Folgenden wird nur auf die Unterschiede zu den vorangehend beschriebenen Ausführungsbeispielen eingegangen. Insbesondere haben die Bezugszeichen die gleiche Bedeutung wie sie bereits im Zusammenhang mit den vorangehend beschriebenen Ausführungsbeispielen erläutert sind. Es versteht sich, dass alle vorangehenden Erläuterungen in gleicher Weise oder in sinngemäss gleicher Weise auch für das fünfte Ausführungsbeispiel gelten.

Das fünfte Ausführungsbeispiel entspricht bezüglich der Ausgestaltung des Stators 2 dem in Fig. 5 dargestellten Ausführungsbeispiel. Der Rotor 3 ist hier als Aussenläufer angeordnet, also so, dass er die zweiten Enden 44 der Längsschenkel 41 der Spulenkerne 4 bezüglich der radialen Richtung aussenliegend umgibt. Dazu ist der magnetische Kern 31 ringförmig ausgestaltet mit einer Mehrzahl - hier acht - ausgeprägten Rotorzähnen 32, die sich jeweils bezüglich der radialen Richtung nach innen erstrecken, also den zweiten Enden 44 der Längsschenkel 41 der Spulenkerne 4 zugewandt sind.

Fig. 18 zeigt eine perspektivische Darstellung eines sechsten Ausführungsbeispiels eines erfindungsgemässen Drehantriebs, das ebenfalls als Aussenläufer ausgestaltet ist und ansonsten dem in Fig. 7 dargestellten Ausführungsbeispiel entspricht. Zum besseren Verständnis zeigt Fig. 19 noch einen Schnitt in axialer Richtung durch das sechste Ausführungsbeispiel .

Im Folgenden wird nur auf die Unterschiede zu den vorangehend beschriebenen Ausführungsbeispielen eingegangen. Insbesondere haben die Bezugszeichen die gleiche Bedeutung wie sie bereits im Zusammenhang mit den vorangehend beschriebenen Ausführungsbeispielen erläutert sind. Es versteht sich, dass alle vorangehenden Erläuterungen in gleicher Weise oder in sinngemäss gleicher Weise auch für das sechste Ausführungsbeispiel gelten.

Die Ausgestaltung des Stators 2 des sechsten Ausführungsbeispiels entspricht in weiten Zügen der im Zusammenhang mit Fig. 7 erläuterten Ausgestaltung. Auch hier umfasst jeder Spulenkern 4 zwei jeweils L-förmig ausgestaltete permanentmagnetfreie Teile 47 und einen L-förmigen permanentmagnetischen Teil 46, der zwischen den beiden permanentmagnetfreien Teilen 47 angeordnet ist. Da es sich um einen Aussenläufer handelt, sind hier jedoch die Querschenkel 42 jeweils in radialer Richtung nach aussen ausgerichtet, also auf den Rotor 3 zu. Der Rotor 3 ist hat den magnetische Kern 31, der ringförmig mit einer Mehrzahl - hier acht - ausgeprägten Rotorzähnen 32 ausgestaltet ist, die sich jeweils bezüglich der radialen Richtung nach innen erstrecken, also den Querschenkeln 42 der Spulenkerne 4 zugewandt sind.

Im Folgenden werden anhand der Fig. 20-23 verschiedene Varianten für die bevorzugte Ausgestaltung der Spulenkerne 4 mit dem Längsschenkel 41 erläutert, bei welcher jeder Spulenkern 4 die beiden permanentmagnetfreien Teile 47 und den dazwischen angeordneten permanentmagnetischen Teil 46 aufweist. Diese Erläuterungen gelten in sinngemäss gleicher Weise sowohl für solche Spulenkerne 4, die keinen Querschenkel 42 haben (siehe z. B. Fig. 5) als auch für solche Spulenkerne 4, die sowohl einen Längsschenkel 41 und einem Querschenkel 42 haben, bei denen also die permanentmagnetfreien Teile 47 und der permanentmagnetische Teil 46 jeweils L-förmig ausgestaltet sind.

Wie bereits erwähnt, sind die permanentmagnetfreien Teile 47 der Spulenkern 4 aus einem weichmagnetischen Material gefertigt, welches den magnetischen Fluss gut leitet. Bevorzugte weichmagnetische Materialien umfassen Eisen, Nickel-Eisen oder Silizium-Eisen.

Vorzugsweise sind die permanentmagnetfreien Teile 47 jedes Spulenkerns 4 geblecht ausgestaltet. Dies ist in jeder der Fig. 20-23 zu erkennen, die jeweils eine Variante für die Ausgestaltung des Spulenkerns 4 in einer perspektivischen Darstellung zeigt. Bei der geblechten Ausgestaltung ist jedes permanentmagnetfreie Statorteil 47 aus einer Mehrzahl von dünnen Elementen 48 aufgebaut, die parallel zueinander aufeinander gestapelt sind. Alle Elemente 48 sind identisch ausgestaltet, hier also jeweils L-förmig und auch mit der gleichen Dicke. Wie dies in den Fig. 20-23 zu erkennen ist, sind die Elemente 48, welche den permanentmagnetfreien Teil 47 bilden, in Umfangsrichtung des Rotors 3 bzw. des Stators 2 gestapelt. Eine Mehrzahl deckungsgleicher, paralleler Elemente 48 bildet also den permanentmagnetfreien Teil 47. Damit die einzelnen Elemente 48 zusammenhalten, können sie verklebt oder mit einem Kunststoff vergossen werden. Dieses Vergiessen kann natürlich den gesamten Spulenkern 4 umfassen. Durch diese geblechte Ausgestaltung lassen sich Wirbelströme in den permanentmagnetfreien Teilen 47 wirkungsvoll unterbinden bzw. reduzieren.

Bei der in Fig. 20 dargestellten Variante ist der zwischen den beiden permanentmagnetfreien Teilen 47 angeordnete und L-förmig ausgestaltete permanentmagnetische Teil 46 einstückig. Seine Magnetisierung ist durch den Pfeil ohne Bezugszeichen angezeigt.

Bei der Variante gemäss Fig. 21 ist der permanentmagnetische Teil 46 aus zwei stabförmigen Segmenten 461 zusammengesetzt, von denen das eine seine Längserstreckung in axialer Richtung A hat und das andere seine Längserstreckung in radialer Richtung.

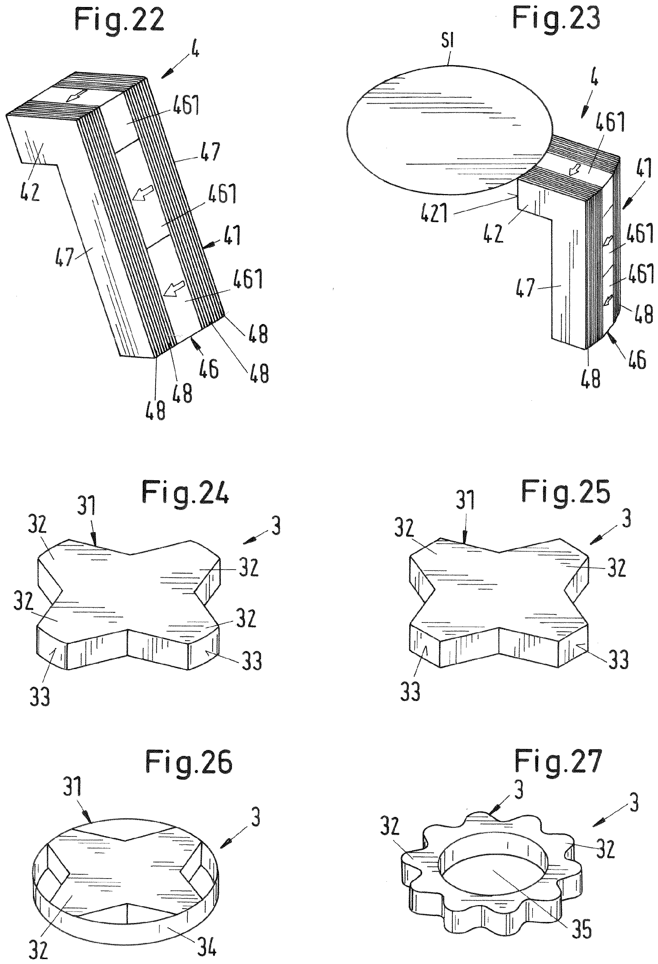

Bei der Variante gemäss Fig. 22 ist der permanentmagnetische Teil 46 aus drei stabförmigen bzw. quaderförmigen Segmenten 461 zusammengesetzt, von denen zwei ihre Längserstreckung in axialer Richtung A haben und das dritte seine Längserstreckung in radialer Richtung.

Fig. 23 veranschaulicht eine Massnahme, welche in vielen Anwendungen bezüglich der Ausgestaltung der L-förmigen Spulenkerne 4 vorteilhaft ist. Bei dieser Variante ist die dem Rotor 3 zugewandte Stirnfläche 421 des Querschenkels 41 gekrümmt ausgestaltet und zwar derart gekrümmt, dass sie der Krümmung der radialen Aussenseite des Rotors 3 folgt. Wenn die Krümmung der Stirnfläche 421 an den Rotor 3 angepasst ist , so folgt z. B. bei einem scheibenförmigen Rotor 3, dass der Luftspalt zwischen dem Spulenkern 4 und dem Rotor 3 in radialer Richtung eine Ausdehnung aufweist, die über die gesamte Stirnfläche 421 gesehen konstant ist. Hiermit lässt sich in diesem Luftspalt ein räumlich sehr gut homogener magnetischer Fluss realisieren. Eine derartige Ausgestaltung lässt sich beispielsweise dadurch realisieren, dass die individuellen Elemente bezüglich der radialen Richtung sukzessive leicht gegeneinander verschoben werden, um die Krümmung der Stirnfläche 421 zu erzielen. In Fig. 23 ist der Innenkreis welcher die Spulenkerne 4 radial innenliegend begrenzt durch den Kreis mit dem Bezugszeichen SI angedeutet. Die individuellen Elemente 48 der permanentmagnetfreien Teile 47 werden dann jeweils so in radialer Richtung gegeneinander verschoben, dass sie mit ihren die jeweilige Stirnfläche 421 bildenden Enden der Kontur des Innenkreises SI folgen.

Es versteht sich, dass bei einer Ausgestaltung als Innenläufer, so wie sie in Fig. 23 angedeutet ist, die Stirnfläche 421 in Umfangsrichtung konkav gewölbt ist während sie bei einer Ausgestaltung als Aussenläufer konvex gewölbt ist.

Im Folgenden werden nun anhand der Fig. 24-41 verschiedene zusätzliche Varianten für die Ausgestaltung des Rotors 3, genauer gesagt für die Ausgestaltung des magnetisch wirksamen Kerns 31 des Rotors 3 erläutert, wobei auf die Ausgestaltung als Innenläufer Bezug genommen wird. Der Rotor 3 ist spulenfrei und frei von Permanentmagneten als Reluktanzläufer ausgestaltet. Vorzugsweise ist der magnetisch wirksame Kern 31 des Rotors 3 bzw. alle seine Teile aus einem weichmagnetischen Material gefertigt, beispielsweise aus Eisen, Nickel-Eisen oder Silizium-Eisen. Dabei kann der magnetisch wirksame Kern 31 z.B. durch Giessen, Stanzen, Pressen von weichmagnetischem Pulver mit anschliessendem Sintern, Schmieden, Umformen oder Zusammensetzen von Teilen wie Blechen hergestellt werden. Insbesondere kann der Rotor 3 also auch, in analoger Weise wie bezüglich der Spulenkerne 4 erläutert wurde, geblecht ausgestaltet sein, also aus mehreren dünnen Elementen, die dann zueinander parallel gestapelt und beispielsweise durch eine Kunststoffummantelung bzw. durch ein Vergiessen in Kunststoff fixiert werden. Im Unterschied zu den Spulenkernen 4 sind bei einer geblechten Ausgestaltung des magnetisch wirksamen Kerns 31 des Rotors 3 die einzelnen Elemente vorzugsweise in axialer Richtung A gestapelt. Natürlich ist es auch möglich, die einzelnen Elemente bei der geblechten Ausgestaltung in radialer Richtung zu stapeln, sodass die Grenzflächen zwischen benachbarten Elementen parallel zur axialen Richtung A verlaufen.

Zunächst werden nun einige Varianten und Massnahmen für die Ausgestaltung des magnetisch wirksamen Kerns 31 des Rotors 3 erläutert, bei denen die für einen Reluktanzläufer benötigte Anisotropie in der Rotormagnetisierung durch die geometrische Ausgestaltung des magnetisch wirksamen Kerns 31 des Rotors 3, also beispielsweise durch ausgeprägte Rotorzähne 32, realisiert wird. Es versteht sich, dass die jeweilige Anzahl der Rotorzähne 32 beispielhaft zu verstehen ist. Eine optimale Anzahl von Rotorzähnen kann der Fachmann je nach Anwendungsfall und insbesondere je nach Ausgestaltung des Stators 2, im Speziellen je nach Anzahl der Spulenkerne 4 ohne Probleme ermitteln. Auch können die im Zusammenhang mit den einzelnen Varianten erläuterten Massnahmen natürlich miteinander kombiniert werden bzw. mit den bereits beschriebenen Ausgestaltungen des Rotors 3 kombiniert werden.

Bei den im Folgenden beschriebenen Varianten für die Ausgestaltung des magnetisch wirksamen Kerns 31 des Rotors 3 ist der magnetisch wirksame Kern 31 bezüglich der axialen Richtung A stets scheiben- oder ringförmig ausgestaltet, wobei die axiale Höhe HR des magnetischen Kerns 31 (vergleiche Fig. 13) vorzugsweise höchstens so gross ist, wie die Höhe HS in axialer Richtung A der ihm zugewandten Stirnflächen 421 der Spulenkerne 4.

Bei der in Fig. 24 in einer perspektivischen Darstellung dargestellten Variante ist der magnetische Kern 31 des Rotors 3 im Wesentlichen kreuzförmig mit vier ausgeprägten Rotorzähnen 32 ausgestaltet. Dabei ist jeder Rotorzahn 32 trapezförmig ausgestaltet, sodass er sich in radialer Richtung auf den Stator 2 zu verjüngt. Die radial aussenliegenden Begrenzungsflächen 33 jedes Rotorzahns 32 sind jeweils gekrümmt -hier konvex - ausgestaltet. Zusammen mit einer wie in Fig. 23 dargestellten gekrümmten Ausgestaltung der Stirnflächen 421 der Spulenkerne 4 hat somit der Luftspalt zwischen den radial aussenliegenden Begrenzungsflächen 33 des Rotorzahns 32 und den Stirnflächen 421 in Umfangsrichtung gesehen eine konstante radiale Erstreckung, was zu einer vorteilhaften sehr homogenen Flussverteilung in diesem Luftspalt führt.

Die in Fig. 25 in einer perspektivischen Darstellung gezeigte Variante des magnetischen Kerns 31 entspricht im Wesentlichen der in Fig. 24 dargestellten, jedoch sind bei der Variante gemäss Fig. 25 die radial aussenliegenden Begrenzungsflächen 33 jedes Rotorzahns 32 planar ausgestaltet, also nicht gekrümmt. Diese Ausgestaltung ist inbesondere dann bevorzugt, wenn auch die Stirnflächen 421 der Spulenkerne 4 planar, also nicht gekrümmt ausgestaltet sind.

Die in Fig. 26 in einer perspektivischen Darstellung gezeigte Variante des magnetischen Kerns 31 entspricht weitgehend der in Fig. 25 gezeigten. Jedoch umfasst der magnetische Kern 31 bei der Variante gemäss Fig. 26 zusätzlich einen geschlossenen, radial aussenliegenden Ring 34, beispielsweise einen Eisensteg, der sich über den gesamten Umfang des magnetisch wirksamen Kerns 31 erstreckt und diesen bezüglich der radialen Richtung begrenzt. Der Ring 34 bildet also die radial äussere Begrenzungsfläche des magnetisch wirksamen Kerns 31. Die Massnahme des radial aussenliegenden Rings 34 ist insbesondere im Hinblick auf die Sensorik vorteilhaft, mit welcher die radiale Position des Rotors 3 und seine Drehstellung ermittelt wird. Durch den radial aussenliegenden Ring 34 ist es nämlich gewährleistet, dass der Sollabstand des magnetisch wirksamen Kerns 31 des Rotors 3 von dem Stator 2 über den gesamten Umfang des magnetischen Kerns 31 gesehen eine konstante Grösse ist. Der Sollabstand ist dabei der radiale Abstand zwischen dem Rotor 3 und dem Stator 2 wenn sich der Rotor 3 in der radialen Ebene in der zentrierten und unverkippten Position befindet. Die Konstanz des Sollabstands über die Umfangsrichtung des Rotors 3 - also entlang des Rings 34 - ermöglicht im Betriebszustand insbesondere eine einfachere messtechnische Erfassung der radialen Position des Rotors 3, weil der Sollabstand des magnetischen Kerns 31 unabhängig von der gerade aktuellen Winkelstellung des Rotors 3 für alle Spulenkerne 4 gleich gross und konstant ist.

Fig. 27 zeigt in einer perspektivischen Darstellung eine Variante, bei welcher der magnetisch wirksame Kern 31 des Rotors 3 im Wesentlichen ringförmig und mit mehreren ausgeprägten Rotorzähnen 32 ausgestaltet ist. Der magnetisch wirksame Kern 31 hat also in seinem Zentrum ein kreisförmiges Loch 35. Den magnetischen Kern 31 ringförmig, also mit dem Loch 35, auszugestalten, erlaubt es, die Masse des magnetisch wirksamen Kerns 31 im Vergleich zu einer scheibenförmigen Ausgestaltung, also ohne Loch 35, zu reduzieren. Somit kann auch der gesamte Rotor 3 mit geringerem Gewicht ausgestaltet werden, was für einige Anwendungen ein Vorteil ist. Ausserdem lässt sich weichmagnetisches Material sparen, wodurch die Materialkosten bei der Herstellung des Rotors 3 reduziert werden. Der magnetisch wirksame Kern 31 hat wiederum eine Mehrzahl - hier zehn - ausgeprägte Rotorzähne 32. Dabei sind die Rotorzähne 32 an ihren radial aussenliegenden Enden abgerundet und auch der Übergangsbereich zwischen zwei benachbarten Rotorzähnen 32 ist abgerundet ausgestaltet. Diese Abrundungen können so ausgestaltet sein, dass sie insgesamt in Umfangsrichtung des magnetisch wirksamen Kerns 31 gesehen, zumindest näherungsweise einer harmonischen Funktion wie beispielsweise einer Sinusfunktion folgen.

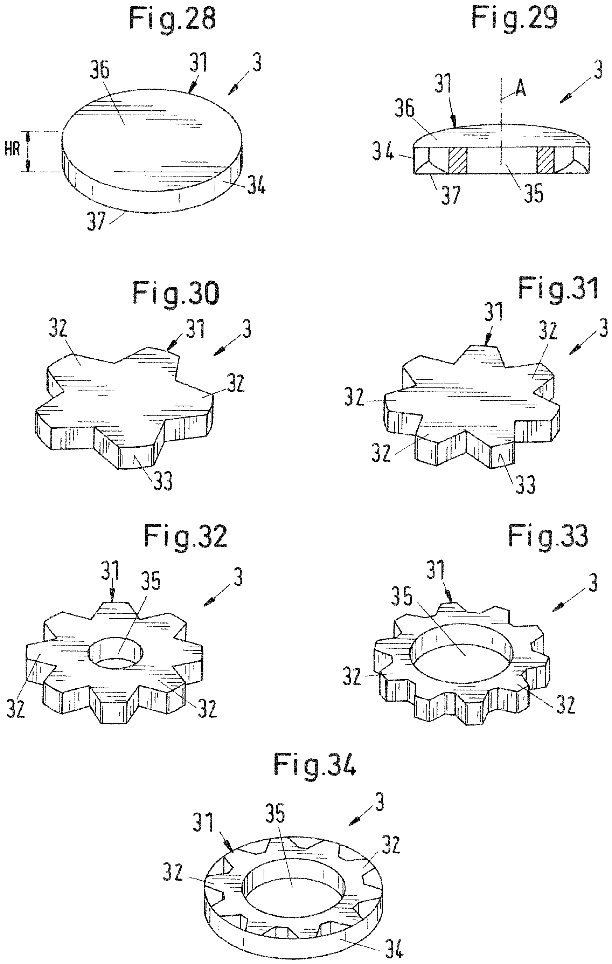

Fig. 28 zeigt in einer perspektivischen Darstellung eine Variante, bei welcher der magnetisch wirksame Kern 31 des Rotors 3 zwei kreisförmige Deckscheiben, nämlich eine untere Deckscheibe 37 und eine obere Deckscheibe 36, umfasst, welche den magnetisch wirksamen Kern 31 in axialer Richtung A nach oben bzw. nach unten begrenzen. Zudem ist der radial aussenliegende Ring 34 vorgesehen, welcher den magnetisch wirksamen Kern 31 bezüglich der radialen Richtung begrenzt.

Ein magnetisch wirksamer Kern 31, der gemäss Fig. 28 ausgestaltet ist, lässt sich beispielsweise herstellen, indem die Variante aus Fig. 26 zusätzlich mit der oberen Deckscheibe 36 und mit der unteren Deckscheibe 37 versehen wird. Die beiden Deckscheiben 36 und 37 sind vorzugsweise auch aus einem weichmagnetischen Material wie beispielsweise Eisen. Diese Umkapselung mittels des radial aussenliegenden Rings 34 und den beiden Deckscheiben 36, 37 gibt dem magnetisch wirksamen Kern 31 insgesamt die äussere Form einer Kreisscheibe, beziehungsweise eines Kreiszylinders der Höhe HR und somit eine besonders symmetrische äussere Gestalt. Dies ist in vielen Fällen im Hinblick auf die Sensorik für die Erfassung der radialen Rotorposition sowie der Winkelstellung vorteilhaft. Bezüglich des Rings 34 ist dies schon im Zusammenhang mit Fig. 26 erläutert. Die Deckplatten 36, 37 geben dem magnetisch wirksamen Kern 31 auch in axialer Richtung A gesehen eine möglichst homogene und symmetrische äussere Form. Dies ist insbesondere dann vorteilhaft, wenn die Sensorik Positionssensoren umfasst, die bezüglich der axialen Richtung A ausserhalb der magnetischen Rotorebene C angeordnet sind, beispielsweise oberhalb oder unterhalb des magnetisch wirksamen Kerns 31. Solche Sensoren können beispielsweise im axialen Streufeld des magnetisch wirksamen Kerns 31 des Rotors 3 messen, womit das magnetische Feld gemeint ist, welches ausserhalb des Luftspalts zwischen dem magnetisch wirksamen Kern 31 des Rotors 3 und den Spulenkernen 4 generiert wird.

Zudem hat die scheibenförmige äussere Gestalt des magnetisch wirksamen Kerns 31 den Vorteil, dass sich der magnetische Kern 31 in besonders einfacher Weise mit anderen Komponenten zum Rotor 3 zusammenfügen lässt, beispielsweise mit Kunststoffummantelungen, Flügeln, Mischelementen usw.

Es versteht sich, dass diese Umkapselung mittels des Rings 34 und der beiden Deckscheiben 36 und 37 als zusätzliche Massnahme auch auf alle anderen Ausgestaltungen des magnetisch wirksamen Kerns 31 des Rotors angewendet werden kann. Als ein Beispiel zeigt Fig. 29 in einem Schnitt in axialer Richtung A den magnetisch wirksamen Kern 31 der Variante aus Fig. 27, bei welcher zusätzlich der radial aussenliegende Ring 34 sowie die beiden Deckscheiben 36 und 37 vorgesehen sind.

Fig. 30 zeigt in einer perspektivischen Darstellung eine Variante, bei welcher der magnetisch wirksame Kern 31 des Rotors 3 ähnlich wie die Variante gemäss Fig. 24 ausgestaltet ist, allerdings hat der magnetisch wirksame Kern 31 bei der Variante gemäss Fig. 30 sechs ausgeprägte Rotorzähne 32.

Fig. 31 zeigt in einer perspektivischen Darstellung eine Variante, bei welcher der magnetisch wirksame Kern 31 des Rotors 3 ähnlich wie die Variante gemäss Fig. 25 ausgestaltet ist, allerdings hat der magnetisch wirksame Kern 31 bei der Variante gemäss Fig. 31 sieben ausgeprägte Rotorzähne 32.

Fig. 32 zeigt in einer perspektivischen Darstellung eine Variante, bei welcher der magnetisch wirksame Kern 31 des Rotors 3 ähnlich wie die Variante gemäss Fig. 31 ausgestaltet ist, allerdings hat der magnetisch wirksame Kern 31 bei der Variante gemäss Fig. 32 acht ausgeprägte Rotorzähne 32 und zusätzlich ist das kreisförmige Loch 35 im Zentrum des magnetisch wirksamen Kerns 31 vorgesehen, so dass dieser im Wesentlichen ringförmig ausgestaltet ist.

Fig. 33 zeigt in einer perspektivischen Darstellung eine Variante, bei welcher der magnetisch wirksame Kern 31 des Rotors 3 ähnlich wie die Variante gemäss Fig. 32 ausgestaltet ist, allerdings hat der magnetisch wirksame Kern 31 bei der Variante gemäss Fig. 33 zehn ausgeprägte Rotorzähne 32 und der Durchmesser des Lochs 35 in radialer Richtung ist grösser ausgestaltet.

Fig. 34 zeigt in einer perspektivischen Darstellung eine Variante, bei welcher der magnetisch wirksame Kern 31 des Rotors 3 ähnlich wie die Variante gemäss Fig. 33 ausgestaltet ist, allerdings hat der magnetisch wirksame Kern 31 bei der Variante gemäss Fig. 34 zusätzlich den radial aussenliegenden Ring 34, der im Zusammenhang mit Fig. 26 erläutert wurde.

Es versteht sich, dass noch viele andere Varianten für die Ausgestaltung des magnetisch wirksamen Kerns 31 des Rotors 3 möglich sind, beispielsweise durch sinngemässes Kombinieren der beschriebenen Varianten oder durch Variieren der Anzahl der Rotorzähne 32 oder deren geometrischer Form. Dem Fachmann ist es beispielsweise bekannt, dass sich mit einer grösseren Anzahl von Rotorzähnen 32 oder durch Variationen ihrer Geometrie das Nutrasten deutlich reduzieren lässt.

Eine andere Möglichkeit für die Ausgestaltung des magnetisch wirksamen Kerns 31 des Rotors 3 besteht darin, den magnetisch wirksamen Kern 31 in an sich bekannter Weise mit Flusssperren zu versehen, die auch als Flussbarrieren bezeichnet werden. Dabei ist der magnetisch wirksame Kern 31 von seiner äusseren geometrischen Form scheibenförmig oder ringförmig ausgestaltet, beispielsweise als kreiszylindrische Scheibe mit der Höhe HR in axialer Richtung (scheibenförmig) oder als eine solche Scheibe mit einem zentralen Loch 35 (ringförmig). Die magnetische Anisotropie des magnetisch wirksamen Kerns 31 des Rotors 3 wird bei dieser Ausgestaltung dadurch erreicht, dass Flussperren in den magnetisch wirksamen Kern 31 eingefügt sind. Die Flussperren sind Ausnehmungen, zum Beispiel Schlitze oder Sperrflächen, in der ferro- oder ferrimagnetischen Struktur des magnetisch wirksamen Kerns 31, welche mit Luft oder anderem nicht weichmagnetischen Material insbesondere mit einem Kunststoff gefüllt sein können. Da der magnetische Fluss durch das nicht weichmagnetische Material, also z.B. Luft oder Kunststoff, einen sehr hohen magnetischen Widerstand erfährt, während das weichmagnetische Material als magnetischer Leiter betrachtet werden kann, kann dem magnetisch wirksamen Kern 31 durch geeignete Anordnung und Ausgestaltung dieser Flussperren eine beliebige magnetische Anisotropie aufgezwungen werden. Insbesondere lässt sich mit solchen Flussperren die Flussführung für die Anforderungen sowohl des Antriebs als auch der magnetischen Lagerung optimieren. Mit solchen Flusssperren lassen sich insbesondere durch eine entsprechende Anordnung auch ausgeprägte Rotorzähne 32 simulieren, d.h. der magnetisch Wirksame Kern 31 hat dann im Wesentlichen die gleiche magnetische Anisotropie wie sie sich durch das geometrische Ausformen von Rotorzähnen 32 realisieren lässt.

So lässt sich beispielsweise das Nutrasten gegenüber dem in Figur 5 dargestellten kreuzförmigen magnetisch wirksamen Kern 31 stark reduzieren. Mit den Flusssperren lassen sich prinzipiell Rotoren 3 beliebiger Polzahl realisieren, welche sowohl vom magnetischen Antrieb her als auch von der passivmagnetischen Stabilisierung der Verkippung und der Axiallage des Rotors als auch von der aktiv magnetischen Stabilisierung der Position des Rotors 3 in der Radialebene optimiert sind.

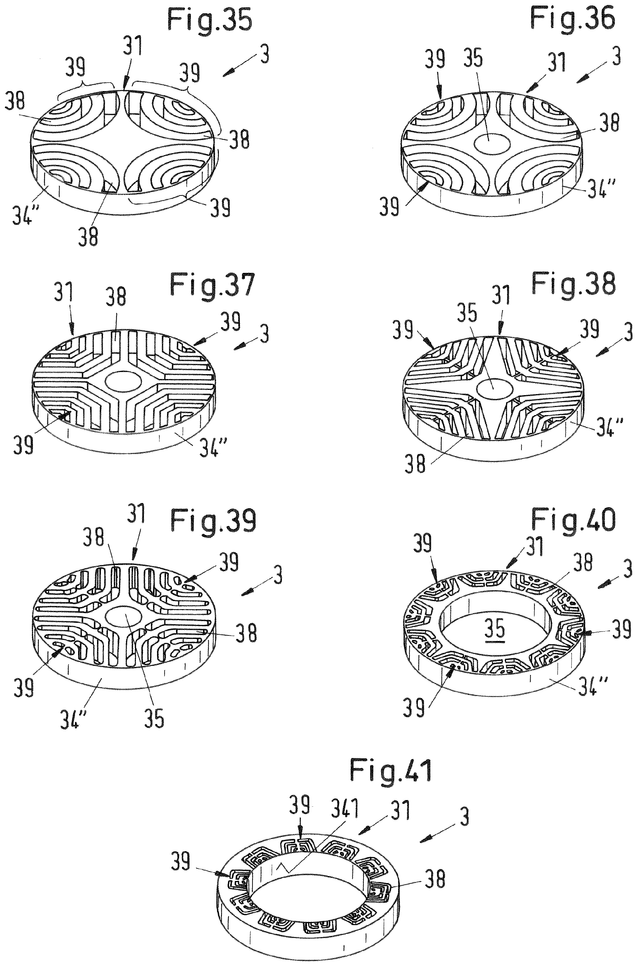

Anhand der Fig. 35 bis 41 werden nun beispielhaft verschiedene Varianten erläutert, wie der magnetisch wirksame Kern 31 des Rotors 3 mit Flussbarrieren bzw. Flusssperen ausgestaltet werden kann.

Fig. 35 zeigt in einer perspektivischen Darstellung den magnetisch wirksamen Kern 31 des Rotors 3 mit einer Mehrzahl von Flussbarrieren 38, mit welchen dem magnetisch wirksamen Kern 31 eine vierpolpaarige magnetische Wirkung aufgeprägt wird. Es sind insgesamt vier Gruppen 39 von Flussbarrieren 38 vorgesehen, wobei benachbarte Gruppen 39 bezüglich der Umfangsrichtung jeweils um 90° versetzt zueinander angeordnet sind, sodass sich die Gruppen paarweise diametral gegenüberliegen. Jede Gruppe 39 umfasst eine Mehrzahl von Flussbarrieren 38, die als viertelkreisförmige, konzentrische Spalte ausgestaltet sind und sich jeweils in axialer Richtung A vollständig durch den magnetisch wirksamen Kern 31 erstrecken. Jeder dieser viertelkreisförmigen Spalte beginnt und endet an der radial aussenliegenden Begrenzungsfläche 34" des magnetisch wirksamen Kerns 31. Dabei ist die radial aussenliegende Begrenzungsfläche 34" eine zusammenhängende Fläche, die nicht von den Spalten durchsetzt wird. Somit resultiert im Hinblick auf die Sensorik der gleiche Vorteil, wie er bereits im Zusammenhang mit Fig. 26 für den Ring 34 erläutert wurde. Die die Flussbarrieren 38 bildenden Spalte können als Luftspalte ausgestaltet sein oder sie können mit einem magnetisch schlecht leitenden Material, insbesondere mit einem Kunststoff ausgefüllt sein.

Fig. 36 zeigt in einer perspektivischen Darstellung eine Variante, bei welcher der magnetisch wirksame Kern 31 des Rotors 3 ähnlich wie die Variante gemäss Fig. 35 ausgestaltet ist, allerdings hat der magnetisch wirksame Kern 31 bei der Variante gemäss Fig. 36 zusätzlich das zentrale Loch 35, ist also ringförmig ausgestaltet.

Fig. 37 zeigt in einer perspektivischen Darstellung eine Variante, bei welcher der magnetisch wirksame Kern 31 des Rotors 3 ähnlich wie die Variante gemäss Fig. 36 ausgestaltet ist, allerdings sind die individuellen Spalte der Flussbarrieren 38 bei der Variante gemäss Fig. 37 nicht viertelkreisförmig ausgestaltet sondern im Wesentlich trapezförmig. Jeder Spalt umfasst zwei Teile, von denen sich jeder von der Begrenzungsfläche 34" radial nach innen erstreckt, wobei die beiden Teile rechtwinklig zueinander verlaufen. Die radial innenliegenden Enden dieser beiden Teile sind dann miteinander verbunden, sodass sich die im Wesentlichen trapezförmige Ausgestaltung ergibt.

Fig. 38 zeigt in einer perspektivischen Darstellung eine Variante, bei welcher der magnetisch wirksame Kern 31 des Rotors 3 ähnlich wie die Variante gemäss Fig. 37 ausgestaltet ist, allerdings sind die individuellen Spalte der Flussbarrieren 38 bei der Variante gemäss Fig. 38 näherungsweise dreieckig ausgestaltet. Jeder Spalt umfasst wiederum die beiden Teile, die sich jeweils von der Begrenzungsfläche 34" nach innen erstrecken. Die beiden Teile verlaufen nun aber nicht mehr in radialer Richtung, sondern aufeinander zu, sodass sich ihre radial innenliegenden Enden treffen, oder fast treffen.

Fig. 39 zeigt in einer perspektivischen Darstellung eine Variante, bei welcher der magnetisch wirksame Kern 31 des Rotors 3 ähnlich wie die Variante gemäss Fig. 37 ausgestaltet ist. Die individuellen Spalte der Flussbarrieren 38 sind wieder im Wesentlich trapezförmig ausgestaltet, wobei jeder Spalt die zwei Teile umfasst, von denen sich jeder von der Begrenzungsfläche 34" in radial nach innen erstreckt, allerdings ist bei der Variante gemäss Fig. 39 die Verbindung zwischen den radial innenliegenden Enden dieser beiden Teile nicht mehr durchgängig, sondern durch einen Steg unterbrochen.

Fig. 40 zeigt in einer perspektivischen Darstellung eine Variante, bei welcher der magnetisch wirksame Kern 31 des Rotors 3 ähnlich wie die Variante gemäss Fig. 39 ausgestaltet ist, allerdings sind bei der Variante gemäss Fig. 40 insgesamt zehn Gruppen 39 von Flussbarrieren 38 vorgesehen, mit welchen dem magnetisch wirksamen Kern 31 eine zehnpolpaarige magnetische Wirkung aufgeprägt wird. Zudem ist der Durchmesser des zentralen Lochs 35 in radialer Richtung grösser ausgestaltet.

Fig. 41 zeigt in einer perspektivischen Darstellung eine Variante, bei welcher der magnetisch wirksame Kern 31 des Rotors 3 für einen Aussenläufer ausgestaltet ist. Die Ausgestaltung der Flussbarrieren 38 ist in sinngemäss gleicher Weise wie bei der Variante gemäss Fig. 40 allerdings ist ihre Anordnung in

Fig. 41 nun für einen Aussenläufer konzipiert. Das heisst bei den jeweils im Wesentlichen trapezförmig ausgestalteten Spalten der Flussbarrieren 38 erstrecken sich nun die beiden Teile von der radial innenliegenden Begrenzungsfläche 341 des magnetisch wirksamen Kerns 31 in radialer Richtung gesehen nach aussen. Auch bei der Aussenläufervariante gemäss Fig. 41 wird dem magnetisch wirksamen Kern 31 eine zehnpolpaarige magnetische Wirkung aufgeprägt.

Für die Herstellung des magnetisch wirksamen Kerns mit den Flussbarrien 38 sind einige Verfahren an sich bekannt. So ist es beispielsweise möglich, in einem scheibenförmigen oder ringförmigen Grundkörper die Flussbarrieren 38 durch Stanzen oder durch Fräsen oder durch sonst eine spanabhebende Methode zu erzeugen. Bei einer geblechten Ausgestaltung des magnetisch wirksamen Kerns 31 können beispielsweise die einzelnen Elemente bevor sie gestapelt werden, durch Fräsen oder Stanzen mit entsprechenden Ausnehmungen bzw. Spalten versehen werden, sodass sich nach dem Stapeln der Elemente die gewünschte Anordnung und Ausgestaltung der Flussbarrieren 38 ergibt.

Im Folgenden werden nun Ausgestaltungen der Positionssensorik und Anordnungen der Positionssensoren erläutert, wobei mit beispielhaftem Charakter auf die Ausgestaltung des Stators 2 gemäss Fig. 7 und die Ausgestaltung des Rotors 3 gemäss Fig. 7 Bezug genommen wird. Es versteht sich, dass die folgenden Erläuterungen nicht auf diese Ausgestaltung des Stators 2 und des Rotors 3 beschränkt sind, sondern in sinngemäss gleicher Weise auch für alle anderen Ausgestaltungen des Stators 2, des Rotors 3 und deren Kombinationen gelten.

Für die Regelung und die Ansteuerung eines lagerlosen Motors und damit auch für die spezielle Ausgestaltung als Tempelmotor 1 müssen die radiale Rotorposition und der Drehwinkel bekannt sein bzw. messtechnisch ermittelt werden. Mit der radialen Rotorposition ist dabei die radiale Lage des Rotors 3 in der radialen Ebene gemeint. Die radiale Ebene ist diejenige, in welcher der Rotor 3 im Betriebszustand magnetisch gelagert wird. Ist der Rotor 3 frei von Verkippungen so stimmt die radiale Ebene mit der magnetischen Rotorebene C überein. Der Drehwinkel des Rotors 3 gibt bei seiner Rotation um die axiale Richtung A die relative Winkellage des Rotors 3 bezüglich des Stators 2 an. Dieser Drehwinkel kann beispielsweise gemessen werden, in dem man in der x-y-Ebene, also in der radialen Ebene, eine beliebig orientierte x-Achse und eine dazu senkrechte y-Achse festlegt, die bezüglich des Stators 2 ortsfest sind. Der momentane Drehwinkel des Rotors 3 lässt sich dann als momentaner Winkel zu dieser x-Achse (oder natürlich auch zu der y-Achse) bestimmen.

Fig. 42 zeigt in einer perspektivischen Darstellung den Stator gemäss Fig. 7 in Kombination mit dem magnetisch wirksamen Kern 31 des Rotors 3 gemäss der Variante in Fig. 27.

Ferner sind in Fig. 42 insgesamt sechs Positionssensoren 7 dargestellt, mit welchen die radiale Rotorposition - also seine Lage in der radialen oder der x-y-Ebene - ermittelbar ist. Die Positionssensoren 7 sind Magnetfeldsensoren und vorzugsweise als Hallsensoren oder GMR-Sensoren ausgestaltet. Die Positionssensoren 7 sind über nicht dargestellte Signalleitungen mit einer nicht dargestellten Kontroll- und Regeleinrichtung signalverbunden.

Es ist eine übliche und bekannte Massnahme, insgesamt vier Positionssensoren 7 vorzusehen, um die Position des Rotors 3 in der radialen Ebene zu bestimmen. Dabei liegen sich die Positionssensoren 7 paarweise diametral gegenüber. Prinzipiell sind zwei Positionssensoren 7 ausreichend, um die Position des Rotors 3 in der x-y-Ebene zur ermitteln, nämlich einer pro Koordinatenrichtung. Es ist jedoch bevorzugt, vier Positionssensoren 7 vorzusehen, um so aus dem Differenzsignal der sich paarweise gegenüberliegenden Positionssensoren 7 eine genauere Bestimmung der Lage des Rotors 3 zu ermöglichen. Da zusätzlich der Drehwinkel des Rotors 3 ermittelt werden muss, sind weitere Positionssensoren 7 notwendig, bei dem in Fig. 42 gezeigten Rotor 3 bzw. magnetisch wirksamen Kern 31 benötigt man im allgemeinen Fall insgesamt mindestens fünf Positionssensoren 7 um sowohl die Rotorposition als auch den Drehwinkel zu bestimmen. Weist der magnetisch wirksame Kern 31 einen radial aussenliegenden Ring 34 auf, wie er im Zusammenhang mit Fig. 26 diskutiert wurde, so reduziert sich die Anzahl der benötigten Positionssensoren 7 auf vier.

Bei der in Fig. 42 dargestellten Anordnung sind insgesamt sechs Positionssensoren 7 vorgesehen, die sich paarweise diametral gegenüberliegen. Die Positionssensoren 7 sind alle in der radialen Ebene angeordnet, in welcher der Rotor 3 gelagert wird, also im unverkippten Zustand des Rotors 3 in seiner magnetischen Mittelebene C. Die Positionssensoren 7 sind äquidistant über die Umfangsrichtung verteilt. Jeweils zwischen den Querschenkeln 42 zweier benachbarter Spulenkerne 4 ist ein Positionssensor 7 angeordnet.

Mit dieser Anordnung der Positionssensoren 7 kann mit Hilfe der Sensorsignale sowohl die radiale Rotorposition als auch der Drehwinkel des Rotors 3 bestimmt werden. Bei den Positionssensoren 7 kann es sich beispielsweise jeweils um Wirbelstromsensoren, optische Sensoren, kapazitive Sensoren oder Magnetfeldsensoren wie Hallsensoren oder GMR Sensoren handeln.

Bei Magnetfeldsensoren kann es eine vorteilhafte Massnahme sein, hinter dem Sensor einen kleinen Permanentmagneten (nicht dargestellt) anzuordnen, falls das magnetische Feld oder das Streufeld am Ort des Positionssensors 7 für eine genügend genaue Messung nicht ausreichen sollte.

In Fig. 43 ist eine weitere Variante für die Anordnung der Positionssensoren 7 in einer perspektivischen Darstellung gezeigt. Zusätzlich zu der in Fig. 42 dargestellten Variante sind bei der Variante gemäss Fig. 43 mehrere - hier vier - Positionssensoren 7 radial innenliegend bezüglich des ringförmigen magnetisch wirksamen Kerns 31 des Rotors 3 in der radialen Ebene angeordnet, die bei unverkipptem Rotor mit der magnetischen Rotorebene C übereinstimmt. Auch die radial innenliegend bezüglich des magnetischen Kerns 31 angeordneten vier Positionssensoren 7 sind vorzugsweise paarweise diametral gegenüberliegend angeordnet und äquidistant über die Umfangsrichtung verteilt. Um mit dieser Anordnung sowohl die radiale Rotorpositions als auch den Drehwinkel des Rotors zuverlässig zu ermitteln, sollten mindestens zwei Positionssensoren 7 radial innenliegend und zwei Positionssensoren 7 radial aussenliegend bezüglich des magnetisch wirksamen Kerns 31 vorgesehen sein.

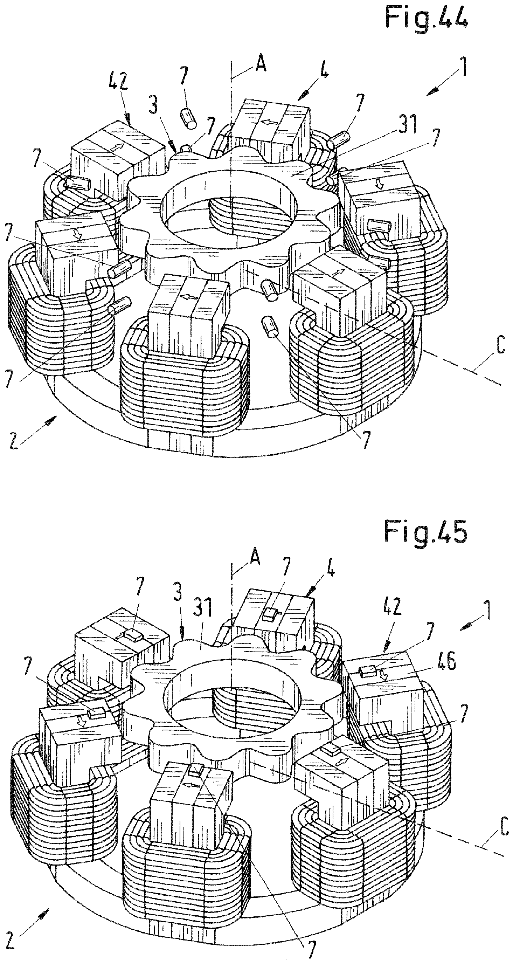

In Fig. 44 ist eine weitere Variante für die Anordnung der Positionssensoren 7 in einer perspektivischen Darstellung gezeigt. Zusätzlich zu der in Fig. 42 dargestellten Variante sind bei der Variante gemäss Fig. 44 eine Mehrzahl -hier sechs- weitere Positionssensoren 7 vorgesehen, welche vorzugsweise paarweise diametral gegenüberliegend zueinander angeordnet und äquidistant über die Umfangsrichtung verteilt sind und welche aller ausserhalb, nämlich darstellungsgemäss unterhalb, der radialen Ebene und damit der magnetischen Rotorebene C angeordnet sind. Jeder dieser weiteren Positionssensoren 7 ist bezüglich seiner radialen Platzierung an der gleichen Stelle angeordnet wie einer der Positionssensoren 7, die in der radialen Ebene angeordnet sind, also an der gleichen radialen Position nur bezüglich der axialen Richtung A unterhalb. Diese Anordnung hat den Vorteil, dass zusätzlich auch die Position des magnetisch wirksamen Kerns bezüglich der axialen Richtung ermittelbar ist. Auch sind Verkippungen des magnetisch wirksamen Kerns 31 bezüglich der axialen Richtung A detektierbar.

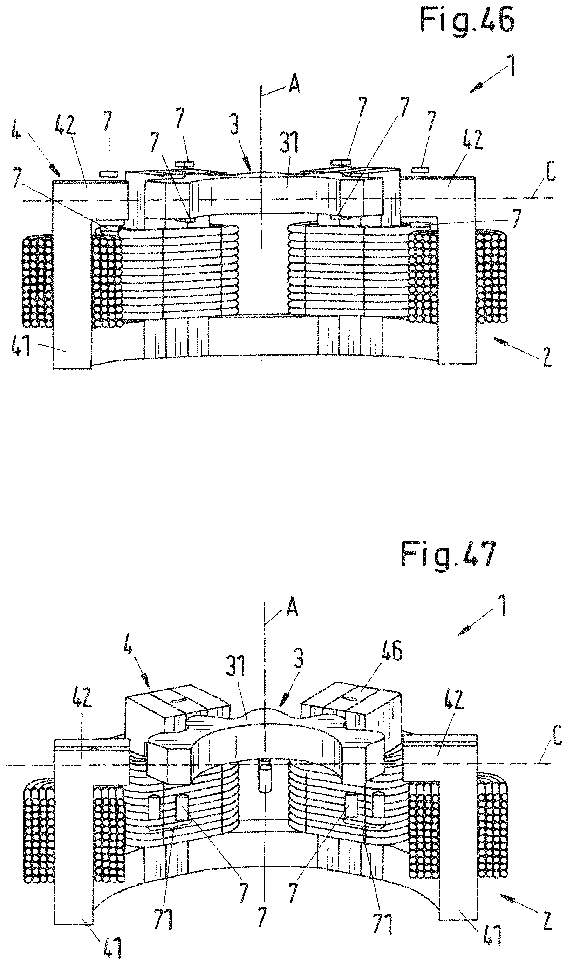

In Fig. 45 ist eine weitere Variante für die Anordnung der Positionssensoren 7 in einer perspektivischen Darstellung gezeigt. Zum besseren Verständnis zeigt Fig. 46 einen Schnitt in axialer Richtung A durch die in Fig. 45 dargestellte Variante. Bei dieser Variante sind insgesamt zwölf Positionssensoren 7 vorgesehen, die alle ausserhalb der radialen Ebene angeordnet sind. An jedem Spulenkern 4 sind genau zwei Positionssensoren 7 vorgesehen, die bezüglich der axialen Richtung A oberhalb und unterhalb des Querschenkels 42 des jeweiligen Spulenkerns 4 angeordnet sind, und zwar derart, dass sich jeder Positionssensor 7 oberhalb oder unterhalb des permanentmagnetischen Teils 46 des Querschenkels 42 befindet. Jeder Positionssensor ist vorzugsweise als Hallsensor oder Magnetfeldsensor ausgestaltet. Je nach Ausgestaltung kann es sein, dass das magnetische Streufeld am Ort der Positionssensoren 7 nicht ausreicht, um den jeweils aktuellen Wert des Drehwinkels oder der radialen Position des Rotors 3 zu ermitteln. Falls dieses Streufeld nicht ausreichend ist, kann jeder der Positionssensoren 7 jeweils mit einem kleinen Permanentmagneten (nicht dargestellt) ausgestattet sein, der beispielsweise auf den jeweiligen Positionssensor 7 aufgeklebt wird.

Diese Anordnung der Positionssensoren 7 oberhalb und unterhalb der Querschenkel 42 ist insbesondere auch unter konstruktiven Aspekten vorteilhaft. Denn es ist möglich, jeweils sechs der Positionssensoren 7 auf einer Leiterplatte, einem PCB (Printed Circuit Board), zu integrieren und dann eine dieser Leiterplatten auf den Querschenkeln 42 anzuordnen und eine Leiterplatte unterhalb der Querschenkel 42.

In Fig. 47 ist in einem Schnitt in axialer Richtung A eine weitere Variante für die Anordnung der Positionssensoren 7 dargestellt. Bei dieser Anordnung sind insgesamt acht Positionssensoren 7 vorhanden, von denen in der Schnittdarstellung der Fig. 47 nur zwei vollständig und vier im Schnitt, also halb- zu erkennen sind. Jeweils zwei Sensoren 7 sind zu einer Gruppe 71 zusammengefasst, sodass vier Gruppen 71 mit je zwei Positionssensoren 7 vorgesehen sind. Alle Positionssensoren 7 sind ausserhalb der radialen Ebene, nämlich bezüglich der axialen Richtung A unterhalb des magnetisch wirksamen Kerns 31 in dem von den Längsschenkeln 41 umgebenen Raum angeordnet. Die vier Gruppen 71 sind paarweise diametral gegenüberliegend angeordnet und bezüglich der Umfangsrichtung äquidistant verteilt. Das heisst bezüglich der Umfangsrichtung sind zwei benachbarte Gruppen 71 jeweils um 90° versetzt. Jede Gruppe 71 umfasst jeweils zwei Positionssensoren 7, die beide bezüglich der axialen Richtung A auf der gleichen Höhe angeordnet sind, wobei einer der Positionssensoren 7 radial weiter innenliegend angeordnet ist als der andere Positionssensor 7 dieser Gruppe 71.

Die vorangehend beschriebenen Ausgestaltungen und Varianten des Stators 2 eignen sich grundsätzlich auch für andere Arten von Rotoren, also auch für solche Rotoren, die Spulen aufweisen, oder solche Rotoren, die mindestens einen Permanentmagneten aufweisen, der zur Generierung des magnetischen Antriebsflusses beiträgt. Durch die Erfindung wird also ferner ein Stator vorgeschlagen für einen elektromagnetischen Drehantrieb, der als Tempelmotor ausgestaltet ist, wobei der Stator 2 vorzugsweise aber nicht unbedingt als Lager- und Antriebsstator ausgestaltet ist, mit welchem ein Rotor im Betriebszustand berührungslos magnetisch um eine Solldrehachse antreibbar und bezüglich des Stators 2 berührungslos magnetisch lagerbar ist, wobei der Stator wie vorangehend beschrieben ausgestaltet ist. Der Stator 2 (siehe z.B. Fig. 7) weist eine Mehrzahl von Spulenkernen 4 auf, von denen jeder einen stabförmigen Längsschenkel 41 umfasst, welcher sich von einem ersten Ende 43 in einer Richtung parallel zur Solldrehachse bis zu einem zweiten Ende 44 erstreckt, wobei alle ersten Enden 43 durch einen Rückschluss 5verbunden sind. Ferner ist eine Mehrzahl von Wicklungen 6, 61 zur Erzeugung eines elektromagnetischen Drehfelds vorgesehen, von denen jede einen der Längsschenkel 41 umgibt. Die Spulenkerne 4 umfassen eine Mehrzahl von Permanentmagneten 46, mit welchen ein permanentmagnetischer Antriebsfluss zum Antreiben des Rotors 3 generierbar ist. Jeder Spulenkern 4 umfasst einen permanentmagnetischen Teil 46, der sich von dem ersten Ende 43 bis zu dem zweiten Ende 44 des Längsschenkels 41 erstreckt, sowie zwei permanentmagnetfreie Teile 47, die sich jeweils von dem ersten Ende 43 bis zu dem zweiten Ende 44 erstrecken. Der permanentmagnetische Teil 46 ist zwischen den beiden permanentmagnetfreien Teilen angeordnet.

Der erfindungsgemässe Stator 2 eignet sich sowohl für Tempelmotoren, bei welchen der Rotor spulenfrei und frei von Permanentmagneten ausgestaltet ist, als auch für Tempelmotoren, bei welchen der Rotor Permanentmagnete und/oder Spulen umfasst.

Der erfindungsgemässe Stator eignet sich auch für solche Tempelmotoren, die nicht nach dem Prinzip des lagerlosen Motors ausgestaltet sind, wo also zusätzlich zu dem den Antrieb erzeugenden Stator 2 separate Lager oder Lagereinheiten, beispielsweise magnetische oder mechanische, für die Lagerung des Rotors 3 vorgesehen sind.

Durch die Erfindung wird ferner eine Rotationsvorrichtung zum Fördern, Pumpen, Mischen oder Rühren von Fluiden vorgeschlagen, die dadurch gekennzeichnet ist, dass die Rotationsvorrichtung einen elektromagnetischen Drehantrieb 1 oder einen Stator 2 umfasst, der erfindungsgemäss ausgestaltet ist. Die vorangehenden Erläuterungen bezüglich des elektromagnetischen Drehantriebs 1, des Stators 2 und des Rotors 3 gelten in gleicher oder in sinngemäss gleicher Weise auch für die erfindungsgemässe Rotationsmaschine. Insbesondere haben die Bezugszeichen die gleiche Bedeutung wie sie bereits im Zusammenhang mit den vorangehend beschriebenen Ausführungsbeispielen erläutert sind.

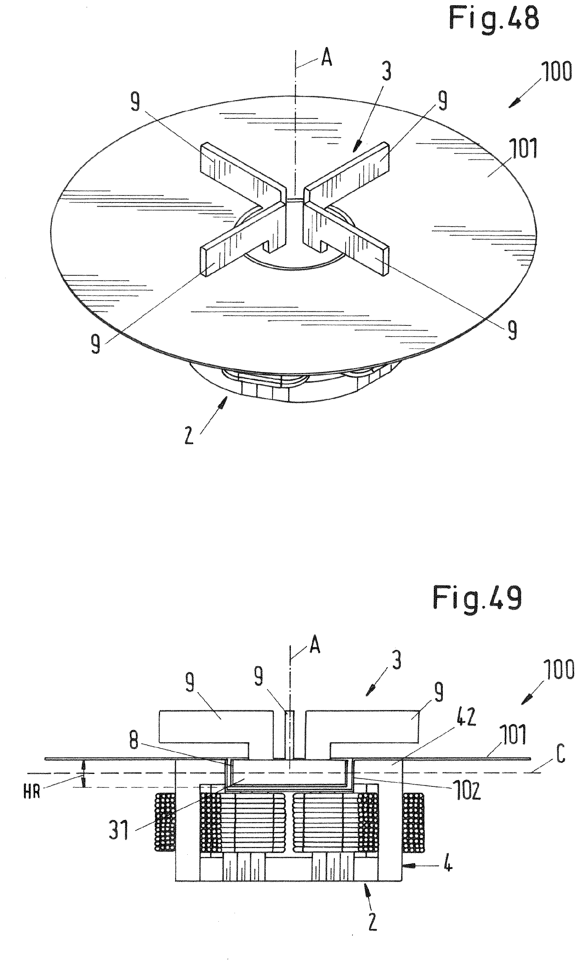

Fig. 48 zeigt in einer perspektivischen Darstellung ein erstes Ausführungsbeispiel einer erfindungsgemässen Rotationsvorrichtung, welches als Mischvorrichtung zum Mischen oder Rühren von Fluiden ausgestaltet ist und gesamthaft mit dem Bezugszeichen 100 bezeichnet ist. Zum besseren Verständnis zeigt Fig. 49 einen Schnitt in axialer Richtung durch dieses erste Ausführungsbeispiel. Die Mischvorrichtung 100 umfasst einen Stator 2, der erfindungsgemäss ausgestaltet ist, also beispielsweise gemäss einer der vorangehend erläuterten Ausgestaltungen oder Varianten. Die Mischvorrichtung 100 umfasst ferner einen Rotor 3, welcher beispielsweise gemäss der vorangehenden Erläuterungen ausgestaltet ist, sowie einen Flansch 101, mit welchem die Mischvorrichtung 100 in einen nicht dargestellten Mischbehälter eingesetzt werden kann. Der Flansch 101 ist hier als im Wesentlichen kreisförmige und formstabile Scheibe ausgestaltet, welche in ihrem Zentrum einen formstabilen Becher 102 zur Aufnahme des Rotors 3 aufweist, und welche vorzugsweise inklusive dem Becher 102 aus einem Kunststoff hergestellt ist. Beispiele für geeignete Kunststoffe werden weiter hinten noch genannt.

Der Rotor 3 umfasst den magnetisch wirksamen Kern 31, der von einer Ummantelung 8 eingeschlossen ist, die vorzugsweise ebenfalls aus einem Kunststoff besteht. Der Rotor 3 umfasst ferner eine Mehrzahl von Flügeln 9 - hier vier - welche an einer der axialen Begrenzungsflächen des magnetisch wirksamen Kerns 31 bzw. seiner Ummantelung 8 angeordnet sind. In der Darstellung gemäss Fig. 48 und 49 sind die Flügel 9 an der oberen axialen Begrenzungsfläche angeordnet und befinden sich vollständig oberhalb der magnetischen Rotorebene C. Auch die Flügel 9 sind vorzugsweise aus einem Kunststoff hergestellt. Die Flügel 9 können einstückig mit der Ummantelung 8 hergestellt sein oder auch als separate Komponenten, die dann an der Ummantelung 8 befestigt werden, beispielsweise durch Verschweissen oder Verkleben. Auch ist es möglich, dass alle Flügel 9 Bestandteil eines separaten Laufrads sind, das dann auf dem magnetisch wirksamen Kern 31 bzw. seiner Ummantelung 8 fixiert wird, z. B. durch Verkleben oder Verschweissen.

Bezüglich Ausgestaltung und Anzahl der Flügel 9 sind natürlich zahlreiche Varianten bekannt, auf die deshalb hier nicht näher eingegangen werden soll.

Der Becher 102 dient der Aufnahme des magnetisch wirksamen Kerns 31 und ist dementsprechend bemessen. Der Becher 102 hat senkrecht zur axialen Richtung A vorzugsweise einen kreisförmigen Querschnitt, wobei der Durchmesser so bemessen ist, dass der Becher 102 möglichst passgenau oder nur mit einem sehr geringen Spiel zwischen die Querschenkel 42 der Spulenkerne 4 des Stators 2 eingesetzt werden kann. Die Tiefe des Bechers 102 in axialer Richtung A ist so bemessen, dass sie etwas grösser ist als die axiale Höhe HR des magnetisch wirksamen Kerns 31 des Rotors 3, sodass der Rotor 3 während des Betriebs durch magnetische Kräfte vom Boden des Bechers 102 abgehoben werden und frei rotieren kann.

Für den Betrieb der Mischvorrichtung 100 wird der Flansch 101 in einen nicht dargestellten Mischbehälter für die zu mischenden Fluide eingesetzt oder mit diesem verbunden. In der Regel bildet der Flansch 101 dann zumindest einen Teil des Bodens des Mischbehälters. Ist der Mischbehälter beispielsweise als flexibler Kunststoffbeutel ausgestaltet, so kann der Flansch 101 mit diesem Beutel zum Mischbehälter verklebt oder verschweisst werden. Ist der Flansch 101 mit dem Mischbehälter verbunden oder in diesen eingesetzt, so bildet der Becher 102 eine Ausstülpung nach aussen bezüglich des Mischbehälters. Der Becher 102 wird in den Stator 2 eingesetzt, der üblicherweise ausserhalb des Mischbehälters angeordnet ist. Der Rotor 3 wird in den Becher 102 eingefügt, sodass der magnetisch wirksame Kern 31 des Rotors 3 vollständig zwischen den Spulenkernen 4 des Stators 2, genauer gesagt zwischen den Querschenkeln 42 zu liegen kommt.

Im Betrieb wird dann der Rotor 3 nach dem Prinzip des lagerlosen Motors von dem Stator 2 berührungslos magnetisch zur Rotation angetrieben und berührungslos magnetisch gelagert, um das Fluid oder die Fluide im Mischbehälter zu mischen. Dabei sind drei Freiheitsgrade des Rotors 3, nämlich seine Rotation und seine Position in der radialen Ebene aktiv magnetisch durch den Stator 2 regelbar bzw. steuerbar, während der Rotor 3 bezüglich der drei anderen Freiheitsgrade, nämlich seiner Position in axialer Richtung sowie Verkippungen bezüglich der radialen Ebene passiv magnetisch, d.h. nicht steuerbar stabilisiert ist. Wenn der Rotor 3 nicht verkippt ist, ist die magnetische Rotorebene C identisch mit der radialen Ebene, in welcher der Rotor 3 bzw. sein magnetisch wirksamer Kern 31 gelagert ist.

Da der Becher 102 bezüglich der axialen Richtung A etwas tiefer ist als die axiale Höhe HR des magnetisch wirksamen Kerns 31 und zudem einen Durchmesser aufweist, der etwas grösser ist als der des magnetisch wirksamen Kerns 31, kann der Rotor 3 im Betriebszustand berührungslos bezüglich des Bechers 102 rotieren. Selbst bei geringfügigen Verkippungen des Rotors 3 oder Verschiebungen seiner radialen und/oder axialen Position kann ein körperlicher Kontakt zwischen dem magnetisch wirksamen Kern 31 bzw. seiner Ummantelung 8 und dem Becher 102 vermieden werden.

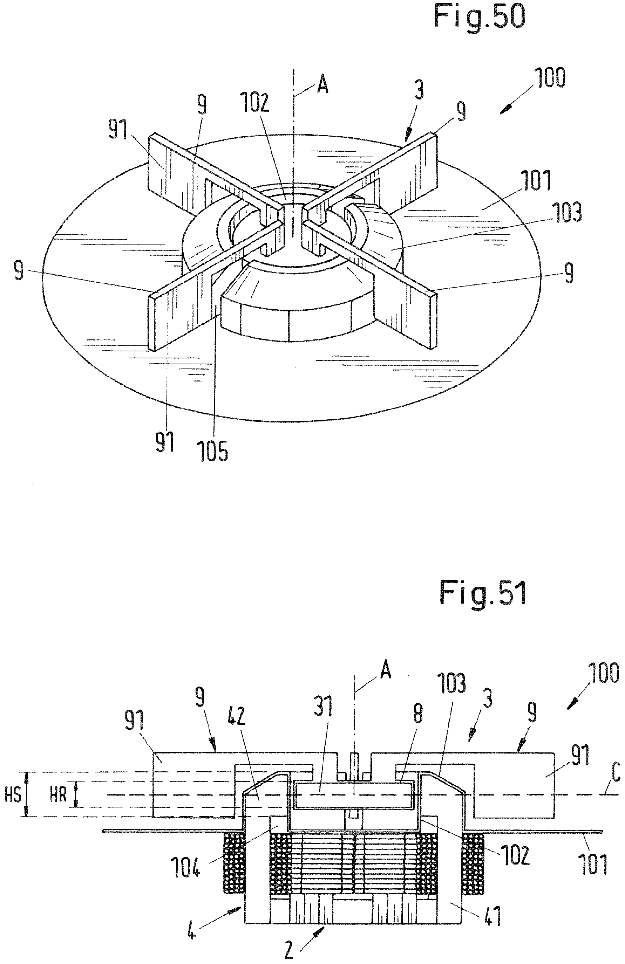

Fig. 50 zeigt in einer perspektivischen Darstellung ein zweites Ausführungsbeispiel einer erfindungsgemässen Rotationsvorrichtung, das ebenfalls als Mischvorrichtung 100 ausgestaltet ist. Zum besseren Verständnis zeigt Fig. 51 einen Schnitt in axialer Richtung durch dieses zweite Ausführungsbeispiel. Im Folgenden wird nur auf die Unterschiede zu dem ersten Ausführungsbeispiel der Rotationsvorrichtung eingegangen. Insbesondere haben die Bezugszeichen die gleiche Bedeutung wie sie bereits im Zusammenhang mit dem vorangehend beschriebenen Ausführungsbeispiel erläutert sind. Es versteht sich, dass alle vorangehenden Erläuterungen in gleicher Weise oder in sinngemäss gleicher Weise auch für das zweite Ausführungsbeispiel gelten.

Bei dem zweiten Ausführungsbeispiel ist der Stator 2 gemäss der in den Fig. 12 und 13 dargestelten Ausführungsform ausgestaltet, bei welcher die Stirnflächen 421 der Querschenkel 42 der Spulenkerne 4 in axialer Richtung A eine Höhe HS aufweisen, die jeweils grösser ist als die axiale Höhe HR des magnetisch wirksamen Kerns 31 des Rotors 3.

Der Becher 102 ist hier so ausgestaltet, dass sein Boden im Wesentlichen in dergleichen Ebene liegt, wie der übrige Teil des scheibenförmigen Flanschs 101, was insbesondere in Fig. 51 gut zu erkennen ist. Der darstellungsgemäss obere Rand der Aussenwand des Bechers 102 ist über eine formstabile Verbindung 103 mit dem Rest des Flanschs 101 verbunden, welche so ausgestaltet ist, dass sie zwischen dem Becher 102 und dem Rest des Flanschs 101 eine Ausnehmung 104 ausbildet, welche die Querschenkel 42 der Spulenkerne 4 aufnehmen kann.

Der Rotor 3 umfasst den magnetischen Kern 31, dessen Ummantelung 8, sowie eine Mehrzahl von Flügeln 9 - hier vier Flügel 9 - die an der darstellungsgemäss oberen axialen Begrenzungsfläche des magnetisch wirksamen Kerns 31 bzw. seiner Ummantelung 8 angeordnet sind. Jeder Flügel 9 erstreckt sich in radialer Richtung über die Ausnehmung 104 hinaus und hat radial aussenliegend zu der Ausnehmung 104 ein Mischblatt 91, welches sich in axialer Richtung A bis kurz vor den Flansch 101 darstellungsgemäss nach unten erstreckt. Bei diesem Ausführungsbeispiel sind die Flügel 9 also derart ausgestaltet, dass sie, genauer gesagt die Mischblätter 91, die magnetische Rotorebene C schneiden. Vorzugsweise liegt dabei die Mittellinie jedes Mischblatts 91, welche die Mittellinie senkrecht zur axialen Richtung A ist, in der magnetischen Rotorebene C.

Da diese Ausgestaltung des Stators 2 eine besonders hohe Stabilität der passiven magnetischen Lagerung mit sich bringt, kann auch dieser Rotor 3 mit den Mischblättern 91 im Betrieb berührungslos magnetisch bezüglich des Stators 2 gelagert werden.

Vorzugsweise ist in der Verbindung 103 eine Öffnung 105 vorgesehen, durch welche das Fluid bzw. Reste des Fluids beim Leeren des Mischbehälters abfliessen kann.

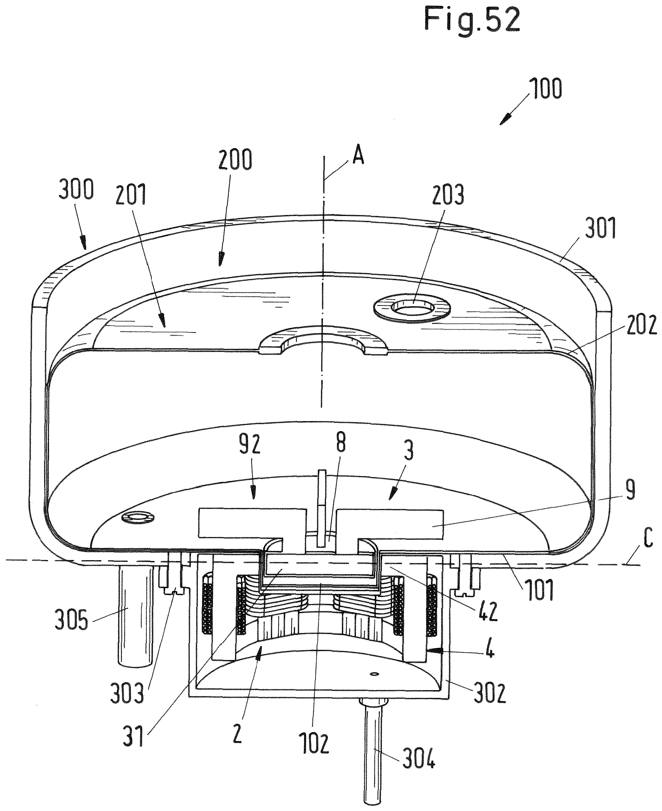

Fig. 52 zeigt einen Schnitt in axialer Richtung A durch ein drittes Ausführungsbeispiel einer erfindungsgemässen Rotationsvorrichtung, das als Pump- oder Mischvorrichtung 100 zum Fördern, Pumpen, Mischen oder Rühren von Fluiden ausgestaltet ist. Im Folgenden wird nur auf die Unterschiede zu vorangehend beschriebenen Ausführungsbeispielen der Rotationsvorrichtung eingegangen. Insbesondere haben die Bezugszeichen die gleiche Bedeutung wie sie bereits im Zusammenhang mit den vorangehend beschriebenen Ausführungsbeispielen erläutert sind. Es versteht sich, dass alle vorangehenden Erläuterungen in gleicher Weise oder in sinngemäss gleicher Weise auch für das dritte Ausführungsbeispiel gelten.

Bei dem dritten Ausführungsbeispiel der Rotationsvorrichtung sind der Stator 2, der Rotor 3 und der Flansch 101 im Wesentlichen so ausgestaltet, wie es im Zusammenhang mit Fig. 48 und Fig. 49 beschrieben ist.

Das dritte Ausführungsbeispiel umfasst eine Einmalvorrichtung 200, die für den Einmalgebrauch ausgestaltet ist, also bestimmungsgemäss nur genau einmal verwendet werden kann, und dann ersetzt werden muss, sowie eine wiederverwendbaren Vorrichtung 300, die für den Mehrfachgebrauch ausgestaltet ist. Die Einmalvorrichtung 200 umfasst den Rotor 3, welcher ein Flügelrad 92 mit den Flügeln 9 zum Fördern, Pumpen, Mischen oder Rühren des Fluids oder der Fluide aufweist. Die wiederverwendbare Vorrichtung 300 umfasst einen formstabilen Stützbehälter 301 zur Aufnahme des Rotors 3, sowie den Stator 2, mit welchem der Rotor 3 im Betriebszustand berührungslos magnetisch antreibbar und lagerbar ist, wobei der Stator 2 erfindungsgemäss ausgestaltet ist.

Die Einmalvorrichtung 200 umfasst ferner einen flexiblen Mischbehälter 201 zur Aufnahme der zu mischenden oder zu fördernden Substanzen, welcher aus einem Kunststoff hergestellt ist. Der Mischbehälter 201 umfasst vorzugsweise einen flexiblen Beutel 202, beispielsweise ein Plastik- oder ein Kunststoffsack, der zusammengefaltet werden kann, sodass er bei der Lagerung möglichst wenig Platz beansprucht. Der Mischbehälter 201 umfasst ferner den Flansch 101 mit dem formstabilen Becher 102 in seinem Zentrum. Der Flansch 101 ist vorzugsweise ebenfalls formstabil und in nicht näher dargestellter Weise fluiddicht mit dem flexiblen Beutel 202 verbunden, beispielsweise verschweisst oder verklebt. Der Rotor 3 ist in dem Mischbehälter 201 angeordnet und befindet sich in dem Becher 102, der dann in den Stator 2 eingesetzt werden kann. Der flexible Mischbehälter 201 der Einmalvorrichtung 200 wird in den Stützbehälter 301 der wiederverwendbaren Vorrichtung 300 eingelegt, welcher den Mischbehälter 201 stützt. Dabei wird der Becher 102 in den Stator 2 eingesetzt, sodass der magnetisch wirksame Kern 31 vollständig zwischen den Querschenkeln 42 der Spulenkerne 4 angeordnet ist.

Es versteht sich, dass der Mischbehälter 201 und/oder der Stützbehälter 301 weitere Öffnungen aufweisen kann, beispielsweise zum Zu- und Abführen von Fluiden oder zum Aufnehmen von Sensoren oder Sonden, mit welchen Eigenschaften der im Mischbehälter 201 befindlichen Substanzen erfasst werden können. Bei der Ausgestaltung gemäss Fig. 52 ist beispielsweise am Mischbehälter 201 ein Einlass 203 vorgesehen, durch welchen Flüssigkeiten, Gase oder andere Substanzen in den Mischbehälter 201 eingebracht werden können. Ferner ist ein Auslass 305 vorgesehen, durch welchen der Mischbehälter 201 entleerbar ist oder durch welche Substanzen aus dem Mischbehälter abgeführt werden können.

Derartige Ausgestaltungen der Rotationsvorrichtung mit der wiederverwendbaren Vorrichtung 300 und der Einmalvorrichtung 200 können vorteilhaft beispielsweise in der pharmazeutischen Industrie und in der biotechnologischen Industrie Verwendung finden. Speziell eignet sich diese Ausgestaltung für solche Anwendungen, bei denen ein sehr hohes Mass an Reinheit oder Sterilität derjenigen Komponenten wesentlich ist, die mit den zu mischenden Substanzen bzw. Fluiden in Kontakt kommen. Diese Ausgestaltung der erfindungsgemässen Rotationsvorrichtung kann auch als Bioreaktor oder als Fermenter ausgebildet sein. Es versteht sich jedoch, dass diese Ausgestaltung auch ganz allgemein eine Pump- oder Mischvorrichtung sein kann, mit der Medien oder Substanzen gemischt werden. Insbesondere können diese Substanzen Fluide oder Feststoffe, vorzugsweise Pulver, sein. Derartige Pump- oder Mischvorrichtungen eignen sich zum Mischen von Flüssigkeiten untereinander und/oder zum Mischen von mindestens einer Flüssigkeit mit einem Pulver oder sonstigen Feststoff und/oder zum Mischen von Gasen mit Flüssigkeiten und/oder Feststoffen.

An seinem Boden weist der Stützbehälter 301 einen zentral angeordneten, im Wesentlichen zylindrisch ausgestalteten Topf 302 zur Aufnahme des Stators 2 auf. Der Topf 302 erstreckt sich in Richtung seiner Zylinderachse, die üblicherweise mit der axialen Richtung A übereinstimmt, und ist am Boden des Stützbehälters 301 befestigt, beispielsweise mittels Schrauben 303. Am Boden des Topfes 302 ist eine Zuführung 304 vorgesehen, welche die elektrischen Leitungen für die Versorgung und die Regelung des Stators 2 umfasst. In dieser Zuführung 304 sind sämtliche elektrischen Verbindungen zusammengefasst, welche für die Energieversorgung und die Ansteuerung des Stators 2 sowie für den Datenaustausch zwischen Sensoren und Messeinrichtungen mit der nicht dargestellten Kontroll- und Regeleinrichtung notwendig sind. Der Topf 302 kann aus einem metallischen Material oder aus einem Kunststoff gefertigt sein.

Das Zusammensetzen des Mischbehälters 201 der Einmalvorrichtung 200 mit dem darin enthaltenen Rotor 3 und des Stützbehälters 301 der wiederverwendbaren Vorrichtung 300 ist äusserst einfach, sowie schnell und insbesondere ohne Werkzeuge durchführbar. Dazu wird der üblicherweise für die Lagerung zusammengefaltete Mischbehälter 201 mit dem darin befindlichen Rotor 3 seiner Verpackung entnommen, in den Stützbehälter 301 eingelegt und der Becher 102 mit dem darin liegenden Rotor 3 in den Topf 302 eingesetzt, sodass der Becher 102 zwischen den Querschenkeln 42 der Spulenkerne 4 zu liegen kommt. Schon dann ist die als Pump- oder Mischvorrichtung 100 ausgestaltete Rotationsvorrichtung bereit für die Anwendung. Nach der Anwendung wird der Mischbehälter 201 mit dem Becher 102 und dem Rotor 3 einfach aus dem Stützbehälter 301 herausgezogen. Der Becher 102 löst sich dabei einfach vom Topf 302 ab. Diese besonders einfache und problemlose Verbindung bzw. Trennung macht dieses dritte Ausführungsbeispiel insbesondere für den Einmalgebrauch verwendbar, wobei der Mischbehälter 201 und der Rotor 3 für den Einmalgebrauch ausgelegt sind, während der Stützbehälter 301 und der Stator 2 mit dem Topf 302 für den Dauergebrauch bzw. für den Mehrfachgebrauch ausgelegt sind.

Der Stator 2 kann mittels einer thermisch leitfähigen Vergussmasse im Topf 302 eingegossen und somit fixiert werden.

Da der Becher 102 des Mischbehälters 201 und der Flansch 101 vorzugsweise formstabil ausgestaltet sind, der Beutel 202 jedoch flexibel, ist es vorteilhaft, jedoch nicht zwingend notwendig, den Becher 102 und den Flansch 101 als separates Teil herzustellen, das anschliessend mit dem Beutel 202 fluiddicht verbunden wird.

Ein weiterer vorteilhafter Aspekt ist es, dass der Rotor 3 als Integralrotor ausgestaltet ist, weil er sowohl der Rotor 3 des elektromagnetischen Antriebs 1 ist, als auch der Rotor 3 der magnetischen Lagerung, als auch der Rotor 3 des Mischers. Dies bietet den Vorteil einer sehr kompakten und platzsparenden Ausgestaltung.

Wenn der Rotor 3 und der Mischbehälter 201 für den Einmalgebrauch ausgelegt sind, sollten die aus Kunststoff gefertigten Teile aus einem möglichst preisgünstigen, handelsüblichen Kunststoff hergestellt werden. Ein weiterer wesentlicher Aspekt bei der Ausgestaltung für den Einmalgebrauch ist es, dass die Einmalteile für gewisse Anwendungsbereiche sterilisierbar sein müssen. Dabei ist es besonders vorteilhaft, wenn die Einmalteile gamma-sterilisierbar sind. Bei dieser Art der Sterilisierung wird das zu sterilisierende Element mit Gamma-Strahlung beaufschlagt. Der Vorteil der Gamma-Sterilisierung, beispielsweise im Vergleich zur Dampfsterilisierung, liegt insbesondere darin, dass die Sterilisierung auch durch die Verpackung hindurch erfolgen kann. Gerade bei Einmalteilen ist es eine gängige Praxis, dass die Teile nach ihrer Herstellung in die für den Versand vorgesehene Verpackung gebracht werden und dann noch eine Zeit lagern, bevor sie an den Kunden ausgeliefert werden. In solchen Fällen erfolgt die Sterilisierung erst kurz vor der Auslieferung an den Kunden durch die Verpackung hindurch, was bei einer Dampfsterilisierung oder anderen Verfahren nicht möglich ist.

Es ist in der Regel nicht notwendig, dass die Einmalteile - wie der Mischbehälter 201 und der Rotor 3 mehr als einmal sterilisierbar sein müssen. Dies ist insbesondere bei der Gamma-Sterilisierung ein grosser Vorteil, weil die Beaufschlagung mit Gamma-Strahlung bei Kunststoffen zu Degradationen führen kann, sodass eine mehrfache Gamma-Sterilisierung den Kunststoff unbrauchbar machen kann.

Da in der Regel bei Einmalteilen auf eine Sterilisierung unter hohen Temperaturen und /oder unter hohem (Dampf-) Druck verzichtet werden kann, können kostengünstigere Kunststoffe eingesetzt werden, beispielsweise solche, die keine hohen Temperaturen aushalten, oder die nicht mehrfach hohen Temperatur- und Druckwerten ausgesetzt werden können.

Unter Berücksichtigung all dieser Aspekte ist es daher bei der Ausgestaltung für den Einmalgebrauch bevorzugt, für die Herstellung der Einmalteile solche Kunststoffe zu verwenden, die zumindest einmal gamma-sterilisierbar sind. Die Materialien sollten dabei gammastabil für eine Dosis von mindestens 40 kGy sein, um eine einmalige Gamma-Sterilisierung zu ermöglichen. Bei der Gamma-Sterilisierung sollten zudem keine giftigen Stoffe entstehen. Zudem ist es bevorzugt, wenn alle Materialien, die mit den zu mischenden bzw. den durchmischten Substanzen in Berührung kommen, USP Class VI Standards erfüllen.

Für die Herstellung des flexiblen Beutels 202 sind beispielsweise folgende Kunststoffe bevorzugt: PolyEthylene (PE), Low Density PolyEthylene (LDPE), Ultra Low Density PolyEthylene (ULDPE), Ethylene Vinyl Acetate (EVA), PolyEthylene Terephthalate (PET), PolyVinylChlorid (PVC), PolyPropylene (PP), PolyUrethan (PU), Silicone

Für die Herstellung des Bechers 102 und die aus Kunststoff bestehenden Teile des Rotors 3, also beispielsweise das Flügelrad 92, die Flügel 9 und die Ummantelung 8, sind beispielsweise folgende Kunststoffe bevorzugt: PolyEthylene (PE), PolyPropylene (PP), Low Density PolyEthylene (LDPE), Ultra Low Density PolyEthylene (ULDPE), Ethylene Vinyl Acetate (EVA), PolyEthylene Terephthalate (PET), PolyVinylChlorid (PVC), PolyVinyliDene Fluoride (PVDF), Acrylonitrile Butadiene Styrene (ABS), PolyAcryl, PolyCarbonate (PC).

Für den Einmalgebrauch weniger geeignete oder sogar ungeeignete Materialien für die Herstellung der Kunststoffteile sind beispielsweise die unter dem Markennamen Teflon bekannten Materialien Polytetrafluoroethylene (PTFE) und und Perfluoralkoxy-Polymere (PFA). Bei diesen Materialien besteht nämlich bei der Gamma-Sterilisierung die Gefahr, dass gefährliche Gase austreten, wie beispielsweise Fluor, das dann giftige oder schädliche Verbindungen wie Flusssäure (HF) bilden kann. Natürlich können solche Materialien bei solchen Anwendungen verwendet werden, bei denen speziell der Rotor 3 nicht für den Einmalgebrauch ausgelegt ist.

Auch bei den dritten Ausführungsbeispiel bilden natürlich der Stator 2 und der Rotor 3 zusammen den als Tempelmotor ausgestalteten elektromagnetischen Drehantrieb 1, der wie bereits erläutert nach dem Prinzip des lagerlosen Motors arbeitet. Beim lagerlosen Motor sind immer mindestens drei Freiheitsgerade des Rotors 3, nämlich seine Rotation um die Solldrehachse A und seine Position in der radialen Ebene, aktiv magnetisch regelbar. Der Freiheitsgrad der axialen Position des Rotors 3 ist passiv magnetisch stabilisiert, das heisst, es ist kein separates Axialmagnetlager oder mechanisches Axiallager notwendig. Dadurch wird einerseits der Rotor 3 besonders einfach und kostengünstig und andererseits lässt sich der Rotor 3 einfach vom Stator 2 und vom Topf 302 trennen. Aufgrund des Fehlens axialer Lagerkomponenten kann nämlich der Rotor 3 zusammen mit dem Becher 102 einfach vom Topf 302 bzw. vom Stator 2 getrennt werden.

Der magnetisch wirksame Kern 31 des Rotors 3 wird im Betrieb bei einer Auslenkung in axialer Richtung A durch die vom Stator 2 ausgehenden Magnetfelder wie durch magnetische Federkräfte zurückgezogen. Diese passiv magnetischen axialen Rückstellkräfte, welche den Rotor 3 bezüglich der axialen Richtung A in seiner Sollposition stabilisieren, nehmen beim Verschieben des Rotors 3 in axialer Richtung A zunächst mit der Auslenkung zu, erreichen bei einer bestimmten Auslenkung, welche von der Geometrie des magnetisch wirksamen Kerns 31 des Rotors 3, der Geometrie der Stirnflächen 421 der Querschenkel 42 der Spulenkerne 4, der Geometrie und den magnetischen Eigenschaften der permanentmagnetischen Teile 46 und dem Luftspalt zwischen dem Stator 2 und dem Rotor 3 abhängen, ein Maximum und nehmen dann wieder ab. Bei der Ausgestaltung der vorliegenden Erfindung wird die Charakteristik des inhärenten axialen Passivmagnetlagers so gewählt, dass die axialen Kräfte, welche auf den Rotor 3 wirken, im ganzen Betriebsbereich unterhalb der Maximalkraft des axialen Passivmagnetlagers liegen, und dass bei solchen Anwendungen, bei welchen der Rotor 3 einfach vom Stator 2 trennbar sein soll, die Maximalkraft des axialen Passivmagnetlagers klein genug bleibt, dass sich der Rotor 3 gegebenenfalls mit dem Mischbehälter 201 einfach und ohne Werkzeug vom Stator 2 trennen lässt. Dabei hat sich für Ausgestaltungen als Pump- oder Mischvorrichtung eine Maximalkraft des axialen Passivmagnetlagers von maximal 200 Newton als noch ohne Werkzeug oder Hilfsvorrichtung handhabbar herausgestellt. Bei kleineren Mischvorrichtungen, wird eine deutlich kleinere Maximalkraft des axialen Passivmagnetlagers gewählt, um das Einsetzen und Entfernen so einfach wie möglich zu gestalten. Typisch sind Werte zwischen 10 Newton und 80 Newton für Mischvorrichtungen für 50 Liter bis 1000 Liter und niederviskose Flüssigkeiten.

Für die beiden verbleibenden Freiheitsgrade, nämlich die Verkippungen des Rotors 3 relativ zur radialen Ebene, ist bei allen Ausgestaltungen ebenfalls eine passiv magnetische Stabilisierung realisierbar. Bei solchen Ausgestaltungen wird die Regelung des als Tempelmotor ausgestalteten lagerlosen Motors besonders einfach und auch die Anzahl der Leistungsverstärkerkanäle kann reduziert werden. Diese alleinige passive Stabilisierung des Rotors 3 gegen Verkippungen funktioniert aber nur dann zuverlässig, wenn gewisse geometrische Bedingungen erfüllt sind. Wenn man mit d den Durchmesser des magnetisch wirksamen Kerns 31 des Rotors 3 und HR die Höhe des magnetisch wirksamen Kerns 31 in axialer Richtung, so muss der Durchmesser mindestens 2,6 mal so gross sein wie die Höhe HR. Es sollte somit die Bedingung d > 2.6 ^{∗} HR erfüllt sein, das heisst, der Durchmesser d sollte grösser sein als das 2,6-fache der Höhe HR.

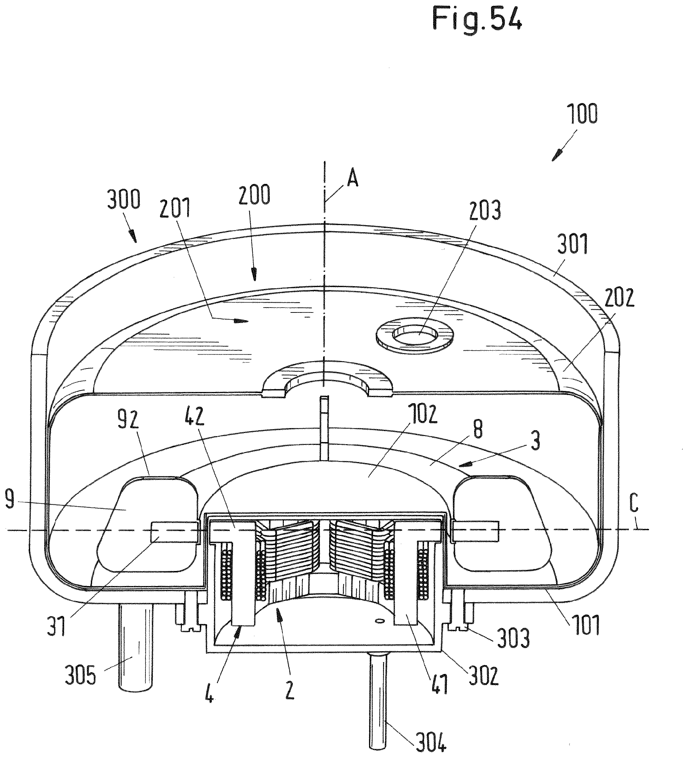

Ist der Rotor 3 als Aussenläufer ausgestaltet (siehe z. B. Fig. 54), so ist in dieser geometrischen Beziehung der Durchmesser des magnetisch wirksamen Kerns 31 durch den Innendurchmesser des magnetisch wirksamen Kerns 31 zu ersetzen, d.h. dann lautet die Bedingung, dass der Innendurchmesser d des magnetisch wirksamen Kerns 31 mindestens 2,6 mal so gross ist wie die Höhe HR. Es sollte somit die Bedingung d > 2.6 ^{∗} HR erfüllt sein, das heisst, der Innendurchmesser d sollte grösser sein als das 2,6-fache der Höhe HR.

Aus diesem Grund ist es auch für die erfindungsgemässe Rotationsvorrichtung bevorzugt, wenn der Rotor 3 bezüglich Verkippungen zur radialen Ebene (zwei Freiheitsgrade) rein passiv magnetisch stabilisiert ist, falls der Durchmesser des Rotors 3 (bzw. der Innendurchmesser bei einer Ausgestaltung als Aussenläufer) mindestens 2.6 mal so gross ist wie die Höhe HR des magnetisch wirksamen Kerns 31 in axialer Richtung A.

Bei Ausgestaltungen der Erfindung, bei denen diese geometrische Bedingung nicht mehr erfüllt ist, kann der Rotor 3 durch andere geeignete Massnahmen bezüglich dieser Verkippungen stabilisiert oder geregelt werden.

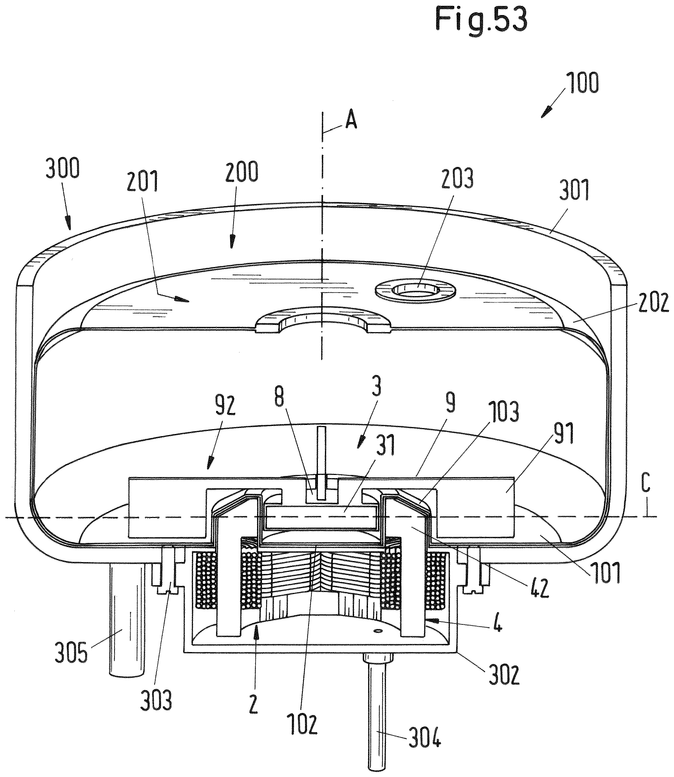

Fig. 53 zeigt einen Schnitt in axialer Richtung A durch ein viertes Ausführungsbeispiels der erfindungsgemässen Rotationsvorrichtung, das als Pump- oder Mischvorrichtung 100 zum Fördern, Pumpen, Mischen oder Rühren von Fluiden ausgestaltet ist. Im Folgenden wird nur auf die Unterschiede zu den vorangehend beschriebenen Ausführungsbeispielen eingegangen. Insbesondere haben die Bezugszeichen die gleiche Bedeutung wie sie bereits im Zusammenhang mit dem ersten, zweiten bzw. dritten Ausführungsbeispiel erläutert sind. Es versteht sich, dass alle vorangehend beschriebenen Varianten, Ausführungsformen und Massnahmen in gleicher Weise oder in sinngemäss gleicher Weise auch bei dem vierten Ausführungsbeispiel realisiert sein können.

Das vierte Ausführungsbeispiel entspricht in wesentlichen Zügen dem dritten Ausführungsbeispiel jedoch ist bei dem vierten Ausführungsbeispiel der Stator 2 und der Rotor 3 so ausgestaltet, wie es im Zusammenhang mit den Fig. 50 und 51 erläutert ist.

Fig. 54 zeigt einen Schnitt in axialer Richtung A durch ein fünftes Ausführungsbeispiels der erfindungsgemässen Rotationsvorrichtung 100. Im Folgenden wird nur auf die Unterschiede zu den vorangehend beschriebenen Ausführungsbeispielen eingegangen. Insbesondere haben die Bezugszeichen die gleiche Bedeutung wie sie bereits im Zusammenhang mit den vorangehenden Ausführungsbeispielen erläutert sind. Es versteht sich, dass alle vorangehenden Erläuterungen in gleicher Weise oder in sinngemäss gleicher Weise auch für das fünfte Ausführungsbeispiel gelten.

Auch das fünfte Ausführungsbeispiel ist als Pump- oder Mischvorrichtung 100 zum Fördern, Pumpen, Mischen oder Rühren von Fluiden ausgestaltet ist. Vorzugsweise, aber nicht notwendigerweise, umfasst auch das fünfte Ausführungsbeispiel Komponenten für den Einmalgebrauch, umfasst also die Einmalvorrichtung 200, die für den Einmalgebrauch ausgestaltet ist, sowie die wiederverwendbare Vorrichtung 300, die für den Mehrfachgebrauch ausgestaltet ist. Die Einmalvorrichtung 200 umfasst den Rotor 3 mit dem Flügelrad 92 und den Flügeln 9 zum Fördern, Pumpen, Mischen oder Rühren des Fluides oder der Fluide sowie den flexiblen Mischbehälter 201 mit dem flexiblen Beutel 202. Die wiederverwendbare Vorrichtung 300 umfasst den formstabilen Stützbehälter 301 zur Aufnahme des Mischbehälters 201, sowie den Stator 2, mit welchem der Rotor 3 im Betriebszustand berührungslos magnetisch antreibbar und lagerbar ist.

Bei dem fünften Ausführungsbeispiel ist der Rotor 3 als Aussenläufer ausgestaltet, das heisst der Rotor 3 ist radial aussenliegend um die Querschenkel 42 der Spulenkerne 4 herum angeordnet, und die Querschenkel 42 erstrecken sich in radialer Richtung nach aussen und somit auf den Rotor 3 zu, sodass die Stirnflächen 421 der Spulenkerne 4 radial aussenliegend angeordnet sind.

Der Stator 2 ist beispielsweise so ausgestaltet, wie es im Zusammenhang mit den Fig. 18 und 19 erläutert ist. Auch der magnetisch wirksame Kern 31 des Rotors 3 ist beispielsweise so ausgestaltet wie in den Fig. 18 und 19 dargestellt. Der magnetische Kern 31 ist von der Ummantelung 8 umschlossen, die wie vorangehend bereits erläutert vorzugsweise aus Kunststoff ist. Zur Bildung der Ummantelung 8 kann der magnetisch wirksame Kern 31 beispielsweise mit dem Kunststoff vergossen werden. Ferner umfasst der Rotor 3 das Flügelrad 92 mit mehreren -hier vier - Flügeln 9, mit welchen das Fluid oder die Substanzen gemischt oder gepumpt oder gerührt werden. Die Flügel 9 bzw. das Flügelrad 91 sind vorzugsweise aus Kunststoff hergestellt und können einstückig mit der Ummantelung 8 hergestellt sein, oder als separate Komponenten, welche anschliessend an der Ummantelung 8 fixiert werden, beispielsweise durch Kleben oder Schweissen. Vorzugsweise sind die Flügel 9 so ausgestaltet und angeordnet, dass der magnetische Kern 31 bezüglich der axialen Richtung A ungefähr mittig durch jeden Flügel 9 verläuft. Hierdurch werden die von den Flügeln 9 während des Betriebs auf den Rotor 3 übertragenen Kräfte sowohl unterhalb als auch oberhalb der magnetischen Rotorebene 3 eingeprägt, was im Hinblick auf die magnetische Stabilisierung der Rotorposition vorteilhaft ist.

Bei der hier beschriebenen Ausgestaltung als Aussenläufer ist der formstabile Becher 102 so auf dem Flansch 101 angeordnet, dass er bezüglich des Mischbehälters 201 nach innen ausgestülpt ist, also in den Mischbehälter 201 hineinragt. Der Rotor 3 ist dann so angeordnet, dass sich der magnetische Kern 31 radial aussenliegend um den Becher 102 herum erstreckt und diesen umgibt. Auf diese Weise ist es möglich, den Stator 2 zu platzieren, dass die Querschenkel 42 der Spulenkerne 4 in dem Becher 102 liegen und somit die Stirnflächen 421 der Querschenkel 42 dem magnetisch wirksamen Kern 31 gegenüberliegend angeordnet sind. Dabei ist der Durchmesser des Bechers 102 bezüglich der radialen Richtung so bemessen, dass der Becher 102 die Querschenkel 42 nur mit einem sehr geringen oder gar keinem Spiel umschliesst, aber problemlos von dem Stator 2 trennbar ist. Der Topf 302, welcher den Stator 2 aufnimmt, überlappt bezüglich der axialen Richtung A mit dem Becher 102, reicht also in den Becher 102 hinein und endet kurz oberhalb der Querschenkel 42.

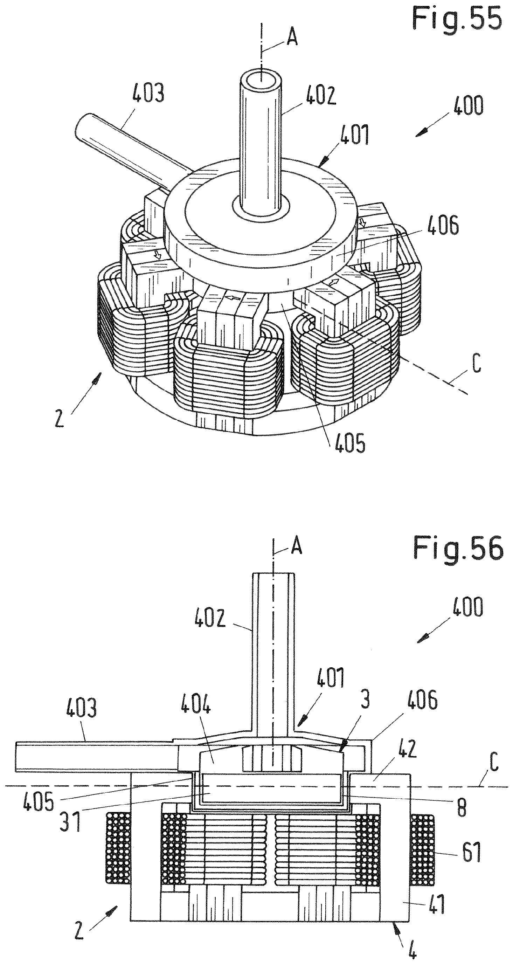

Fig. 55 zeigt eine perspektivische Darstellung eines sechsten Ausführungsbeispiels der erfindungsgemässen Rotationsvorrichtung. Zum besseren Verständnis zeigt Fig. 56 noch einen Schnitt in axialer Richtung durch das sechste Ausführungsbeispiel. Im Folgenden wird nur auf die Unterschiede zu den vorangehend beschriebenen Ausführungsbeispielen eingegangen. Insbesondere haben die Bezugszeichen die gleiche Bedeutung wie sie bereits im Zusammenhang mit den vorangehenden Ausführungsbeispielen erläutert sind. Es versteht sich, dass alle vorangehenden Erläuterungen in gleicher Weise oder in sinngemäss gleicher Weise auch für das sechste Ausführungsbeispiel gelten.

Das sechste Ausführungsbeispiel ist als Pumpvorrichtung 400 zum Pumpen oder Fördern eines Fluids ausgestaltet und umfasst den Stator 2, der beispielsweise so ausgestaltet ist, wie es im Zusammenhang mit Fig. 7 und Fig. 8 erläutert ist.

Die Pumpvorrichtung 400 umfasst ferner ein Pumpengehäuse 401, das vorzugsweise aus einem Kunststoff hergestellt ist. Das Pumpengehäuse 401 hat einen sich in axialer Richtung A erstreckenden Einlass 402 für das zu fördernde Fluid, der zentral in der Mitte des Pumpengehäuses 401 angeordnet und als zylindrische Röhre in axialer Richtung A ausgestaltet ist, sowie einen sich in radialer Richtung erstreckenden Auslass 403 für das zu fördernde Fluid, der als zylindrische Röhre ausgestaltet ist. In dem Pumpengehäuse 401 ist der als Flügelrad ausgestaltete Rotor 3 vorgesehen, der den magnetisch wirksamen Kern 31 umfasst, sowie eine Mehrzahl von Flügeln 404, um das Fluid vom Einlass 402 zum Auslass 403 zu fördern. Die Flügel 404 sind bezüglich der axialen Richtung A darstellungsgemäss oberhalb des magnetisch wirksamen Kerns 31 angeordnet. Die Pumpvorrichtung 400 ist hier also als Zentrifugalpumpe ausgestaltet. Natürlich sind auch andere Ausgestaltungen, z. B. als Axial- oder Helioaxialpumpe möglich.

Der magnetisch wirksame Kern 31 des Rotors 3 ist von der Ummantelung 8 umschlossen, die, genau wie die Flügel 404, vorzugsweise aus Kunststoff gefertigt ist bzw. sind. Das Pumpengehäuse 401 umfasst eine unteren Teil 405, welcher den magnetisch wirksamen Kern 31 des Rotors 3 umgibt, sowie einen dazu axial benachbarten oberen Teil 406, welcher die Flügel 404 des Rotors umgibt. Beide Teile 405 und 406 haben senkrecht zur axialen Richtung eine im Wesentlichen kreisförmige Querschnittsfläche, wobei der untere Teil 405 einen kleineren Durchmesser aufweist als der obere Teil 406. Der Durchmesser des unteren Teils 405 ist dabei so bemessen, dass er mit möglichst geringem Spiel zwischen die Querschenkel 42 der Spulenkerne 4 einsetzbar und in einfacher Weise wieder von dem Stator 2 trennbar ist. Der Durchmesser des oberen Teils 406 ist so bemessen, dass er bezüglich der radiale Richtung mit den Querschenkeln 42 überlappt, so dass er auf den Querschenkeln 42 aufliegen kann.

Wenn das Pumpengehäuse 401 mit dem darin angeordneten Rotor 3 in den Stator 2 eingesetzt ist, umgeben die Querschenkel 42 der Spulenkerne 4 den unteren Teil 405 des Pumpengehäuses 401, in welchem sich der magnetisch wirksame Kern 31 befindet, sodass die magnetische Rotorebene C in der radialen Ebene liegt und der magnetisch wirksame Kern 31 bezüglich der axialen Richtung A vollständig zwischen den Stirnflächen 421 der Querschenkel 42 liegt. Somit ist der Rotor 3 im Betriebszustand berührungslos magnetisch antreibbar und berührungslos magnetisch bezüglich des Stators lagerbar.

Der obere Teil 406 des Pumpengehäuses 401 befindet sich bezügliche der axialen Richtung A unmittelbar oberhalb der Querschenkel 42 der Spulenkerne 4. Der Auslass 403, der in diesen oberen Teil 406 mündet, liegt bezüglich der axialen Richtung A auf der gleichen Höhe wie die Flügel 404, was insbesondere im Hinblick auf die hydrodynamischen Kräfte vorteilhaft ist, die im Betrieb auf den Rotor 3 einwirken. Denn diese hydrodynamischen Kräfte verteilen sich möglichst gleichmässig über den Rotor 3. Eine solche relative Anordnung zwischen dem Auslass 403 und den Flügeln 404 ist insbesondere durch die Ausgestaltung des Drehantriebs 1 als Tempelmotor möglich, weil hier die Querschenkel 42 frei von Wicklungen sind, welche eine solche Anordnung des Auslasses 403 zumindest deutlich erschweren würden.

Auch die als Pumpvorrichtung 400 ausgestaltete Rotationsvorrichtung kann in vorteilhafter Weise mit Komponenten für den Einmalgebrauch ausgestaltet sein und die Einmalvorrichtung 200 und die wiederverwendbare Vorrichtung 300 aufweisen. Für solche Anwendungen umfasst die Einmalvorrichtung 200 vorzugsweise das Pumpengehäuse 401 und der darin angeordnete Rotor 3 als Einmalteile für den Einmalgebrauch, die also bestimmungsgemäss nur einmal verwendet werden können, und dann für die nächste Anwendung durch ein neues ungebrauchtes Teil ersetzt werden müssen. Die wiederverwendbare Vorrichtung 300 umfasst bei dieser Ausgestaltung den Stator 2.

Aufgrund der Abwesenheit von mechanischen Lagern eignet sich die Pumpvorrichtung 400 insbesondere für solche Anwendungen, bei denen sehr empfindliche Substanzen gefördert werden, beispielsweise Blutpumpen, oder bei denen sehr hohe Anforderungen an die Reinheit gestellt werden, beispielsweise in der pharmazeutischen Industrie oder in der biotechnologischen Industrie, oder mit denen abrasive Substanzen gefördert werden, welche mechanische Lager sehr schnell zerstören würden, beispielsweise Pumpen für Slurry in der Halbleiterindustrie.

Auch bei der Pumpvorrichtung 400 ist es ein vorteilhafter Aspekt, dass der Rotor 3 als Integralrotor ausgestaltet ist, weil er sowohl der Rotor 3 des elektromagnetischen Antriebs 1 ist, als auch der Rotor 3 der magnetischen Lagerung, als auch der Rotor 3 der Pumpvorrichtung 400, mit welchem das zu fördernde Fluid gepumpt wird. Dies bietet den Vorteil einer sehr kompakten und platzsparenden Ausgestaltung.

## Patentansprüche

1. Stator für einen elektromagnetischen Drehantrieb, der als Tempelmotor ausgestaltet ist, wobei mit dem Stator (2) ein Rotor (3) im Betriebszustand berührungslos magnetisch um eine Solldrehachse antreibbar ist, wobei der Stator (2) eine Mehrzahl von Spulenkernen (4) aufweist, von denen jeder einen stabförmigen Längsschenkel (41) umfasst, welcher sich von einem ersten Ende (43) in einer Richtung parallel zur Solldrehachse bis zu einem zweiten Ende (44) erstreckt, wobei alle ersten Enden (43) durch einen Rückschluss (5) verbunden sind, und wobei eine Mehrzahl von Wicklungen (6; 61) zur Erzeugung eines elektromagnetischen Drehfelds vorgesehen ist, von denen jede einen der Längsschenkel (41) umgibt, wobei die Spulenkerne (4) eine Mehrzahl von Permanentmagneten (46) umfassen, mit welchen ein permanentmagnetischer Vormagnetisierungsfluss generierbar ist, **dadurch gekennzeichnet, dass** jeder Spulenkern (4) einen permanentmagnetischen Teil (46) umfasst, der sich von dem ersten Ende (43) bis zu dem zweiten Ende (44) des Längsschenkels (1) erstreckt, sowie zwei permanentmagnetfreie Teile (47), die sich jeweils von dem ersten Ende (43) bis zu dem zweiten Ende (44) erstrecken, wobei der permanentmagnetische Teil (46) zwischen den beiden permanentmagnetfreien Teilen (47) angeordnet ist.

2. Stator nach Anspruch 1, wobei der Stator (2) als Lager- und Antriebsstator ausgestaltet ist, mit welchem der Rotor (3) im Betriebszustand berührungslos magnetisch bezüglich des Stators (2) lagerbar ist.

3. Elektromagnetischer Drehantrieb, der als Tempelmotor ausgestaltet ist, mit einem Rotor (3), welcher berührungslos magnetisch antreibbar ist, welcher spulenfrei und frei von Permanentmagneten ausgestaltet ist, und welcher einen magnetisch wirksamen Kern (31) umfasst, sowie mit einem Stator (2), mit welchem der Rotor (3) im Betriebszustand berührungslos magnetisch um eine Solldrehachse antreibbar ist, **dadurch gekennzeichnet, dass** der Stator (2) gemäss einem der vorangehenden Ansprüche ausgestaltet ist.

4. Drehantrieb nach Anspruch 3, wobei der Stator (2) als Lager- und Antriebsstator ausgestaltet ist, mit welchem der Rotor (3) im Betriebszustand berührungslos magnetisch bezüglich des Stators (2) lagerbar ist.

5. Drehantrieb nach einem der Ansprüche 3-4, wobei jeder Spulenkern (4) einen Querschenkel (42) umfasst, welcher an dem zweiten Ende (44) des Längsschenkels (41) angeordnet ist, und welcher sich in einer radialen Richtung erstreckt, die senkrecht zu einer durch die Solldrehachse definierten axialen Richtung (A) ist.

6. Drehantrieb nach einem der Ansprüche 3-5, wobei sich der permanentmagnetische Teil (46) und die beiden permanentmagnetfreien Teile (47) des Spulenkerns (4) jeweils durch den Querschenkel (42) erstrecken, und wobei im Querschenkel (42) der permanentmagnetische Teil (46) zwischen den beiden permanentmagnetfreien Teilen (47) angeordnet ist.

7. Drehantrieb nach einem der Ansprüche 3-6, bei welchem die permanentmagnetischen Teile (46) jeweils senkrecht zur radialen Richtung und senkrecht zur axialen Richtung (A) polarisiert sind, wobei die Permanentmagnete (46) benachbarter Spulenkerne (4) jeweils in entgegengesetzter Richtung polarisiert sind.

8. Drehantrieb nach einem der Ansprüche 3-7, bei welchem die permanentmagnetfreien Teile (47) der Spulenkerne jeweils geblecht aus Elementen (48) hergestellt sind, wobei die Elemente (48) in Umfangsrichtung des Rotors (3) gestapelt sind.

9. Drehantrieb nach einem der Ansprüche 3-8, wobei der Stator (2) eine gerade Anzahl von Spulenkernen (4) aufweist, vorzugsweise sechs oder acht oder zwölf Spulenkerne (4).

10. Drehantrieb nach einem der Ansprüche 5 bis 9, wobei die dem Rotor (3) zugewandten Stirnflächen (421) der Querschenkel (42) der Spulenkerne (4) in axialer Richtung (A) eine Höhe (HS) aufweisen, die jeweils grösser ist als die axiale Höhe (HR) des magnetisch wirksamen Kerns (31) des Rotors (3).

11. Drehantrieb nach einem der Ansprüche 3-10, wobei die Wicklungen (6) Antriebsspulen (62) zum Erzeugen eines elektromagnetischen Antriebsfelds für den Rotor (3) umfassen, sowie davon separate Steuerspulen (63) zum Einstellen einer auf den Rotor (3) wirkenden Querkraft in radialer Richtung.

12. Drehantrieb nach einem der Ansprüche 3-11, bei welchem der magnetisch wirksame Kern (31) des Rotors (3) scheiben- oder ringförmig ausgestaltet ist und eine radial äussere Begrenzungsfläche (34, 34") aufweist, die in einem zentrierten Zustand des Rotors (3) von allen Spulenkernen (4) den gleichen Abstand in radialer Richtung hat.

13. Rotationsvorrichtung zum Fördern, Pumpen, Mischen oder Rühren von Fluiden, **dadurch gekennzeichnet, dass** die Rotationsvorrichtung einen elektromagnetischen Drehantrieb umfasst, der gemäss einem der Ansprüche 3-12 ausgestaltet ist oder einen Stator der gemäss Anspruch 2 ausgestaltet ist.

14. Rotationsvorrichtung nach Anspruch 13, mit einer Einmalvorrichtung (200), die für den Einmalgebrauch ausgestaltet ist, sowie mit einer wiederverwendbaren Vorrichtung (300), die für den Mehrfachgebrauch ausgestaltet ist, wobei die Einmalvorrichtung (200) zumindest den Rotor (3) umfasst, welcher eine Mehrzahl von Flügeln (9) zum Fördern, Pumpen, Mischen oder Rühren des Fluides oder der Fluide aufweist, und wobei die wiederverwendbare Vorrichtung (300) einen Stützbehälter (301) zur Aufnahme des Rotors (3) umfasst, sowie den Stator (2), mit welchem der Rotor (3) im Betriebszustand berührungslos magnetisch antreibbar und lagerbar ist, wobei der Stator (2) mindestens einen Permanentmagneten (45;46) zum Erzeugen eines permanentmagnetischen Vormagnetisierungsflusses umfasst, sowie mindestens eine Wicklung (6;61) zum Erzeugen eines elektromagnetischen Flusses, und wobei der permanentmagnetische Vormagnetisierungsfluss und der elektromagnetische Fluss zusammen den Rotor antreiben und lagern.

## Claims

1. A stator for an electromagnetic rotary drive that is configured as a temple motor, wherein a rotor (3) can be contactlessly magnetically driven about a desired axis of rotation by the stator (2) in the operating state; wherein the stator (2) has a plurality of coil cores (4) of which each comprises a bar-shaped longitudinal limb (41) that extends from a first end (43) in a direction in parallel with the desired axis of rotation up to a second end (44); wherein all the first ends (43) are connected by a reflux (5); and wherein a plurality of windings (6, 61) for generating an electromagnetic rotational field are provided of which windings each surrounds one of the longitudinal limbs (41), wherein the coil cores (4) comprise a plurality of permanent magnets (46) by which a permanent magnetic pre-magnetization flux can be generated, **characterized in that** each coil core (4) comprises a permanent magnetic portion (46) that extends from the first end (43) up to the second end (44) of the longitudinal limb (41) and comprises two permanent magnet-free portions (47) that each extend from the first end (43) up to the second end (44), with the permanent magnetic portion (46) being arranged between the two permanent magnetic portions (47).

2. A stator in accordance with claim 1, wherein the stator (2) is configured as a bearing and drive stator by which the rotor (3) can be contactlessly magnetically supported with respect to the stator (2) in the operating state.

3. An electromagnetic rotary drive that is configured as a temple motor, having a rotor (3) that is contactlessly magnetically drivable, that is configured as coil-free and free of permanent magnets and that comprises a magnetically effective core (31), and having a stator (2) by which the rotor (3) is contactlessly magnetically drivable about a desired axis of rotation in the operating state, **characterized in that** the stator (2) is a stator (2) in accordance with one of the proceeding claims.

4. A rotary drive in accordance with claim 3, wherein the stator (2) is configured as a bearing and drive stator by which the rotor (3) can be contactlessly magnetically supported with respect to the stator (2) in the operating state.

5. A rotary drive in accordance with one of the claims 3-4, wherein each coil core (4) comprises a transverse limb (42) that is arranged at the second end (44) of the longitudinal limb (41) and that extends in a radial direction that is perpendicular to an axial direction (A) defined by the desired axis of rotation.

6. A rotary drive in accordance with any one of the claim 3-5, wherein the permanent magnetic portion (46) and the two permanent magnet-free portions (47) of the coil core (4) each extend through the transverse limb (42); and wherein the permanent magnetic portion (46) is arranged between the two permanent magnet-free portions (47) in the transverse limb (42).

7. A rotary drive in accordance with any one of the claims 3-6, wherein the permanent magnetic portions (46) are each polarized perpendicular to the radial direction and perpendicular to the axial direction (A), with the permanent magnets (46) of adjacent coil cores (4) each being polarized in opposite directions.

8. A rotary drive in accordance with any one of the claims 3-7, wherein the permanent magnet-free portions (47) of the coil cores are each manufactured as in bundled laminate form from elements (48), with the elements (48) being stacked in the peripheral direction of the rotor (3).

9. A rotary drive in accordance with any one of the claims 3-8, wherein the stator (2) has an even number of coil cores (4), preferably six or eight or twelve coil cores (4).

10. A rotary drive in accordance with any one of the claims 5-9, wherein the end faces (421) of the transverse limbs (42) of the coil cores (4) facing the rotor (3) have a height (HS) in the axial direction (A) that is respectively larger than the axial height (HR) of the magnetically effective core (31) of the rotor (3).

11. A rotary drive in accordance with any one of the claims 3-10, wherein the windings (6) comprise drive coils (62) for generating an electromagnetic drive field for the rotor (3) and comprise control coils (63) separate therefrom for setting a transverse force acting on the rotor (3) in the radial direction.

12. A rotary drive in accordance with any one of the claims 3-11, in which the magnetically effective core (31) of the rotor (3) is configured in disk form or ring form and has a radially outer boundary surface (34, 34") that has the same spacing from all coil cores (4) in the radial direction in a centered state of the rotor (3).

13. A rotational apparatus for conveying, pumping, mixing or stirring fluids, **characterized in that** the rotational apparatus comprises an electromagnetic rotary drive that is configured in accordance with any one of the claims 3-12 or comprises a stator that is configured in accordance with claim 2.

14. A rotational apparatus in accordance with claim 13, having a single-use apparatus (200) that is configured for single use; and having a reusable apparatus (300) that is configured for multiple use, wherein the single-use apparatus (200) at least comprises the rotor (3) that has a plurality of vanes (9) for conveying, pumping, mixing or stirring the fluid or fluids; and wherein the reusable apparatus (300) comprises a support tank (301) for receiving the rotor (3) and comprises the stator (2) by which the rotor (3) can be contactlessly magnetically driven and supported in the operating state; wherein the stator (2) comprises at least one permanent magnet (45, 46) for generating a permanent magnetic pre-magnetization flux and comprises at least one winding (6, 61) for generating an electromagnetic flux; and wherein the permanent magnetic pre-magnetization flux and the electromagnetic flux together drive and support the rotor.

## Revendications

1. Un stator pour un entraînement rotatif électromagnétique conçu comme un moteur de temple, dans lequel un rotor (3) peut être entraîné magnétiquement sans contact autour d'un axe de rotation de consigne avec le stator (2) en état de fonctionnement, dans lequel le stator (2) présente une pluralité de noyaux de bobines (4) comprenant chacun une branche longitudinale (41) en forme de tige s'étendant d'une première extrémité (43) dans une direction parallèle à l'axe de rotation de consigne à une deuxième extrémité (44), dans lequel toutes les premières extrémités (43) sont reliées par une voie de retour (5), et dans lequel une pluralité d'enroulements (6; 61) pour la génération d'un champ tournant électromagnétique est prévue, chacun d'entre eux entourant l'une des branches longitudinales (41), dans lequel les noyaux de bobine (4) comprennent une pluralité d'aimants permanents (46) avec lesquels un flux de prémagnétisation magnétique permanent peut être généré, **caractérisé en ce que** chaque noyau de bobine (4) comprend une partie magnétique permanente (46) s'étendant de la première extrémité (43) à la deuxième extrémité (44) de la branche longitudinale (1), et deux parties sans aimant permanent (47) s'étendant chacune de la première extrémité (43) à la deuxième extrémité (44), dans lequel la partie magnétique permanente (46) est disposée entre les deux parties sans aimant permanent (47).

2. Un stator selon la revendication 1, dans lequel le stator (2) est conçu comme un stator de palier et un stator d'entraînement, avec lequel le rotor (3) peut être soutenu magnétiquement sans contact par rapport au stator (2) en état de fonctionnement.

3. Un entraînement rotatif électromagnétique, conçu comme un moteur de temple, avec un rotor (3), qui peut être entraîné magnétiquement sans contact, qui est conçu sans bobines et sans aimants permanents, et qui comprend un noyau magnétique efficace (31), et avec un stator (2) avec lequel le rotor (3) peut être entraîné magnétiquement sans contact autour d'un axe de rotation de consigne en état de fonctionnement, **caractérisé en ce que** le stator (2) est conçu selon l'une des revendications précédentes.

4. Un entraînement rotatif selon la revendication 3, dans lequel le stator (2) est conçu comme un stator de palier et un stator d'entraînement, avec lequel le rotor (3) peut être soutenu magnétiquement sans contact par rapport au stator (2) en état de fonctionnement.

5. Un entraînement rotatif selon l'une des revendications 3 à 4, dans lequel chaque noyau de bobine (4) comprend une branche transversale (42) qui est disposée à la deuxième extrémité (44) de la branche longitudinale (41) et qui s'étend dans une direction radiale perpendiculaire à une direction axiale (A) définie par l'axe de rotation de consigne.

6. Un entraînement rotatif selon l'une des revendications 3 à 5, dans lequel la partie magnétique permanente (46) et les deux parties sans aimant permanent (47) du noyau de bobine (4) s'étendent chacune à travers la branche transversale (42), et dans lequel la partie magnétique permanente (46) est disposée entre les deux parties sans aimant permanent (47) dans la branche transversale (42).

7. Un entraînement rotatif selon l'une des revendications 3 à 6, dans lequel les parties magnétiques permanentes (46) sont chacune polarisées perpendiculairement à la direction radiale et perpendiculairement à la direction axiale (A), dans lequel les aimants permanents (46) de noyaux de bobines (4) adjacents sont chacun polarisés dans la direction opposée.

8. Un entraînement rotatif selon l'une des revendications 3 à 7, dans lequel les parties sans aimant permanent (47) des noyaux de bobine sont chacune constituées d'éléments (48) de manière tôlée, dans lequel les éléments (48) sont empilés dans la direction circonférentielle du rotor (3).

9. Un entraînement rotatif selon l'une des revendications 3 à 8, dans lequel le stator (2) présente un nombre pair de noyaux de bobine (4), de préférence six ou huit ou douze noyaux de bobine (4).

10. Un entraînement rotatif selon l'une des revendications 5 à 9, dans lequel les faces frontales (421) des branches transversales (42) des noyaux de bobine (4) tournées vers le rotor (3) présentent une hauteur (HS) dans la direction axiale (A) qui est à chaque fois supérieure à la hauteur axiale (HR) du noyau magnétique efficace (31) du rotor (3).

11. Un entraînement rotatif selon l'une des revendications 3 à 10, dans lequel les enroulements (6) comprennent des bobines d'entraînement (62) pour générer un champ d'entraînement électromagnétique pour le rotor (3), et des bobines de commande (63) séparées de celles-ci pour régler une force transversale agissant sur le rotor (3) dans la direction radiale.

12. Un entraînement rotatif selon l'une des revendications 3 à 11, dans lequel le noyau magnétique efficace (31) du rotor (3) est en forme de disque ou d'anneau et présente une surface limite radialement extérieure (34, 34") qui, dans un état centré du rotor (3), présente la même distance dans la direction radiale par rapport à tous les noyaux de bobine (4).

13. Un dispositif rotatif pour convoyer, pomper, mélanger ou agiter des fluides, **caractérisé en ce que** le dispositif rotatif comprend un entraînement rotatif électromagnétique qui est conçu selon l'une des revendications 3 à 12 ou un stator qui est conçu selon la revendication 2.

14. Un dispositif rotatif selon la revendication 13, avec un dispositif jetable (200) conçu pour l'usage unique et un dispositif réutilisable (300) conçu pour l'usage multiple, dans lequel le dispositif jetable (200) comprend au moins le rotor (3) ayant une pluralité de pales (9) pour convoyer, pomper, mélanger ou agiter le fluide ou les fluides, et dans lequel le dispositif réutilisable (300) comprend un récipient de support (301) pour recevoir le rotor (3), et le stator (2) avec lequel le rotor (3) peut être entraîné et soutenu magnétiquement sans contact en état de fonctionnement, dans lequel le stator (2) comprend au moins un aimant permanent (45; 46) pour générer un flux de prémagnétisation magnétique permanent, et au moins un enroulement (6; 61) pour générer un flux électromagnétique, et dans lequel le flux de prémagnétisation magnétique permanent et le flux électromagnétique entraînent et soutiennent ensemble le rotor.
